# EUROPEAN PATENT SPECIFICATION

(11) **EP 1 786 925 B1**
(45) Date of publication and mention of the grant of the patent: **08.12.2010**
(21) Application number: 05768832.7
(22) Date of filing: 29.07.2005
(51) Int. Cl.: C12Q 1/68, C12N 15/12, C07K 14/47, C12N 15/11, A61P 35/00

(54) **GENES AND POLYPEPTIDES RELATING TO BREAST CANCERS**
GENE UND POLYPEPTIDE IN VERBINDUNG MIT BRUSTKREBSERKRANKUNGEN
GENES ET POLYPEPTIDES LIES AUX CANCERS DU SEIN

(30) Priority: 10.08.2004 US 600146 P
(43) Date of publication of application: 23.05.2007
(73) Proprietor: Oncotherapy Science, Inc., Kawasaki-shi Kanagawa 213-0012 (JP)
(72) Inventor: NAKAMURA, Yusuke, c/o THE UNIVERSITY OF TOKYO, Tokyo 1138654 (JP); KATAGIRI, Toyomasa, c/o THE UNIVERSITY OF TOKYO, Tokyo 1138654 (JP); NAKATSURU, Shuichi, c/o ONCOTHERAPY SCIENCE, INC., Kawasaki-shi, Kanagawa 2130012 (JP)
(74) Representative: Vossius & Partner
(86) International application number: PCT/JP2005/014369
(87) International publication number: WO 2006/016525

(56) References cited:
- WO-A-2005/039382
- DATABASE Geneseq [Online] 29 January 2004 (2004-01-29), "Human protein 2089 amino acid sequence." XP002350062 retrieved from EBI accession no. GSP:ADE38347 Database accession no. ADE38347 & WO 03/065006 A (MILLENNIUM PHARMACEUTICALS, INC; HUNTER, JOHN, JOSEPH; MACBETH, KYLE,) 7 August 2003 (2003-08-07)
- DATABASE Geneseq [Online] 9 April 2003 (2003-04-09), "Differentially expressed breast cancer associated protein #22." XP002350063 retrieved from EBI accession no. GSP:ABU57635 Database accession no. ABU57635 & US 2002/156263 A1 (CHEN HUEI-MEI) 24 October 2002 (2002-10-24)
- DATABASE Geneseq [Online] 13 March 2002 (2002-03-13), "Human dominant negative protein kinase (HPK 38) SEQ ID NO:4." XP002350064 retrieved from EBI accession no. GSP:ABB04768 Database accession no. ABB04768
- BERTUCCI FRANCOIS ET AL: "Breast cancer revisited using DNA array-based gene expression profiling" INTERNATIONAL JOURNAL OF CANCER, NEW YORK, NY, US, vol. 103, no. 5, 20 February 2003 (2003-02-20), pages 565-571, XP002316056 ISSN: 0020-7136
- NAGAHATA T ET AL: "Expression profiling to predict postoperative prognosis for estrogen receptor-negative breast cancers by analysis of 25,344 genes on a cDNA microarray" CANCER SCIENCE, JAPANESE CANCER ASSOCIATION, TOKYO, JP, vol. 95, no. 3, March 2004 (2004-03), pages 218-225, XP002982989 ISSN: 1347-9032
- JIANG Y ET AL: "DISCOVERY OF DIFFERENTIALLY EXPRESSED GENES IN HUMAN BREAST CANCER USING SUBTRACTED CDNA LIBRARIES AND CDNA MICROARRAYS" ONCOGENE, BASINGSTOKE, HANTS, GB, vol. 21, no. 14, 2002, pages 2270-2282, XP001075060 ISSN: 0950-9232
- BASIK M ET AL: "Amplified genes as therapeutic targets in cancer" TARGETS, ELSEVIER, vol. 2, no. 4, 1 August 2003 (2003-08-01), pages 147-153, XP004886667 ISSN: 1477-3627
- CHEN Y ET AL: "Down-regulation of CXCR4 by inducible small interfering RNA inhibits breast cancer cell invasion in vitro" CANCER RESEARCH, AMERICAN ASSOCIATION FOR CANCER RESEARCH, BALTIMORE, MD, US, vol. 63, no. 16, 15 August 2003 (2003-08-15), pages 4801-4804, XP002322095 ISSN: 0008-5472
- AGATA NAOKI ET AL: "Silencing MUC1 in malignant cells by siRNA: Potential effects on breast cancer cell proliferation." PROCEEDINGS OF THE AMERICAN ASSOCIATION FOR CANCER RESEARCH ANNUAL MEETING, vol. 44, July 2003 (2003-07), page 1348, XP008054436 & 94TH ANNUAL MEETING OF THE AMERICAN ASSOCIATION FOR CANCER RESEARCH; WASHINGTON, DC, USA; JULY 11-14, 2003 ISSN: 0197-016X
- HARVEY AMANDA J ET AL: "Use of RNA interference to validate Brk as a novel therapeutic target in breast cancer: Brk promotes breast carcinoma cell proliferation." ONCOGENE. 7 AUG 2003, vol. 22, no. 32, 7 August 2003 (2003-08-07), pages 5006-5010, XP002350948 ISSN: 0950-9232
- ISHIKAWA N ET AL: "Characterization of SEZ6L2 cell-surface protein as a novel prognostic marker for lung cancer" CANCER SCIENCE, JAPANESE CANCER ASSOCIATION, TOKYO, JP, vol. 97, no. 8, 1 August 2006 (2006-08-01) , pages 737-745, XP002460091 ISSN: 1347-9032
- OETZEL C ET AL: "The tyrosine kinase inhibitor CGP 57148 (ST1 571) induces apoptosis in BCR-ABL-positive cells by down-regulating BCL-X." CLINICAL CANCER RESEARCH : AN OFFICIAL JOURNAL OF THE AMERICAN ASSOCIATION FOR CANCER RESEARCH MAY 2000, vol. 6, no. 5, May 2000 (2000-05), pages 1958-1968, ISSN: 1078-0432
- "Chapter 25" In: "Molecular biology of the cell" Garland Science , pages 1572-1573
- ISHIKAWA N ET AL: "Characterization of SEZ6L2 cell-surface protein as a novel prognostic marker for lung cancer" CANCER SCIENCE, JAPANESE CANCER ASSOCIATION, TOKYO, JP, vol. 97, no. 8, 1 August 2006 (2006-08-01) , pages 737-745, XP002460091 ISSN: 1347-9032
- OETZEL C ET AL: "The tyrosine kinase inhibitor CGP 57148 (ST1 571) induces apoptosis in BCR-ABL-positive cells by down-regulating BCL-X." CLINICAL CANCER RESEARCH : AN OFFICIAL JOURNAL OF THE AMERICAN ASSOCIATION FOR CANCER RESEARCH MAY 2000, vol. 6, no. 5, May 2000 (2000-05), pages 1958-1968, ISSN: 1078-0432
- "Chapter 25" In: "Molecular biology of the cell" Garland Science , pages 1572-1573

## Description

### FIELD OF THE INVENTION

The present invention relates to the field of biological science, more specifically to the field of cancer research. In particular, novel genes are disclosed *B1194* and *A2282*, involved in the proliferation mechanism of breast cancer, as well as polypeptides encoded by the genes. The disclosed genes and polypeptides can be used, for example, in the diagnosis of breast cancer, and as target molecules for developing drugs against breast cancer. Thus, the present invention relates to the embodiments characterized in the claims.

### BACKGROUND OF THE INTENTION

Breast cancer, a genetically heterogeneous disease, is the most common malignancy in women. An estimation of approximately 800,000 new cases worldwide were reported each year (Parkin DM, et. al., (1999). CA Cancer J Clin 49:33-64). Mastectomy is still the concurrent first option for the medical treatment. Despite surgical removal of the primary tumors, relapse at local or distant sites may occur due to micrometastasis undetectable at the time of diagnosis (Saphner T, et al., (1996). J Clin Oncol, 14, 2738-2746). Cytotoxic agents are usually administered as adjuvant therapy after surgery, aiming to kill those residual or pre-malignant cells. Treatment with conventional chemotherapeutic agents is often empirical and is mostly based on histological tumor parameters, and, in the absence of specific mechanistic understanding, target-directed drugs, are therefore becoming the bedrock treatment for breast cancer. Tamoxifen and aromatase inhibitors, two representatives of its kind, have proved to have great responses when used as adjuvant or chemoprevention in patients with metastasized breast cancer (Fisher B, et al., (1998). J Natl Cancer Inst, 90, 1371-1388; Cuzick J (2002). Lancet 360, 817-824). However, the drawback is that only patients' expressed estrogen receptors are sensitive to these drugs. Recently, concerns were even raised regarding their side effects, for example endometrial cancer resulting from long term tamoxifen treatment and bone fractures resulting from aromatase therapy in the postmenopausal women (Coleman RE (2004). Oncology. 18 (5 Suppl 3), 16-20).

In spite of recent progress in diagnostic and therapeutic strategies, prognosis of patients with advanced cancers remains very poor. Although molecular studies have revealed the involvement of alterations in tumor suppressor genes and/or oncogenes in carcinogenesis, the precise mechanisms still remain to be elucidated.

cDNA microarray technologies have enabled the construction of comprehensive profiles of gene expression in normal and malignant cells, and the comparison of gene expression in malignant and corresponding normal cells (Okabe et al., Cancer Res 61:2129-37 (2001); Kitahara et al., Cancer Res 61: 3544-9 (2001); Lin et al., Oncogene 21:4120-8 (2002); Hasegawa et al., Cancer Res 62:7012-7 (2002)). This approach facilitates the understanding of the complex nature of cancer cells, and helps to elucidate the mechanism of carcinogenesis. Identification of genes that are deregulated in tumors can lead to more precise and accurate diagnosis of individual cancers, and to the development of novel therapeutic targets (Bienz and Clevers, Cell 103:311-20 (2000)). To disclose mechanisms underlying tumors from a genome-wide point of view, and discover target molecules for diagnosis and development of novel therapeutic drugs, the present inventors have analyzed the expression profiles of tumor cells using a cDNA microarray of 23,040 genes (Okabe et al., Cancer Res 61:2129-37 (2001); Kitahara et al., Cancer Res 61:3544-9 (2001); Lin et al., Oncogene 21:4120-8 (2002); Hasegawa et al., Cancer Res 62:7012-7 (2002)).

Studies designed to reveal mechanisms of carcinogenesis have already facilitated the identification of molecular targets for anti-tumor agents. For example, inhibitors of farnesyltransferase (FTIs), which were originally developed to inhibit the growth-signaling pathway related to Ras and whose activation depends on post-translational farnesylation, have been shown to be effective in treating Ras-dependent tumors in animal models (Sun J, et al., Oncogene. 1998;16:1467-73.). Clinical trials on humans, using a combination of anti-cancer drugs and the anti-HER2 monoclonal antibody, trastuzumab, to antagonize the proto-oncogene receptor HER2/neu, have been achieving improved clinical response and overall survival of breast cancer patients (Molina MA, et al., Cancer Res. 2001;61:4744-9.). A tyrosine kinase inhibitor, STI-571, which selectively inactivates bcr-abl fusion proteins, has been developed to treat chronic myelogenous leukemias wherein constitutive activation of bcr-abl tyrosine kinase plays a crucial role in the transformation of leukocytes. Agents of these kinds are designed to suppress oncogenic activity of specific gene products (O'Dwyer ME & Druker BJ, Curr Opin Oncol. 2000;12:594-7.). Therefore, gene products commonly up-regulated in cancerous cells may serve as potential targets for developing novel anti-cancer agents.

It has been demonstrated that CD8+ cytotoxic T lymphocytes (CTLs) recognize epitope peptides derived from tumor-associated antigens (TAAs) presented on the MHC Class I molecule, and lyse tumor cells. Since the discovery of the MAGE family as the first example of TAAs, many other TAAs have been discovered using immunological approaches (Boon, Int J Cancer 54: 177-80 (1993); Boon and van der Bruggen, J Exp Med 183: 725-9 (1996); van der Bruggen et al., Science 254: 1643-7 (1991); Richard et al., J Exp Med 178: 489-95 (1993); Kawakami et al., J Exp Med 180: 347-52 (1994)). Some of the discovered TAAs are now in the stage of clinical development as targets of immunotherapy. TAAs discovered to date include MAGE (van der Bruggen et al., Science 254: 1643-7 (1991)), gp100 (Kawakami et al., J Exp Med 180: 347-52 (1994)), SART (Shichijo et al., J Exp Med 187: 277-88 (1998)), and NY-ESO-1 (Chen et al., Proc Natl Acad Sci USA 94: 1914-8 (1997)). On the other hand, gene products which had been demonstrated to be specifically over-expressed in tumor cells, have been shown to be recognized as targets inducing cellular immune responses. Such gene products include p53 (Umano et al., Brit J Cancer 84: 1052-7 (2001)), HER2/neu (Tanaka et al., Brit J Cancer 84: 94-9 (2001)), CEA (Nukaya et al., Int J Cancer 80: 92-7 (1999)), and the like.

In spite of significant progress in basic and clinical research concerning TAAs (Rosenberg et al., Nature Med 4: 321-7 (1998); Mukherji et al., Proc Natl Acad Sci USA 92: 8078-82 (1995); Hu et al., Cancer Res 56: 2479-83 (1996)), only a limited number of candidate TAAs for the treatment of adenocarcinomas, including breast cancer, are currently available. TAAs abundantly expressed in cancer cells, and at the same time whose expression is restricted to cancer cells, would be promising candidates as immunotherapeutic targets. Further, identification of new TAAs inducing potent and specific anti-tumor immune responses is expected to encourage clinical use of peptide vaccination strategies in various types of cancer (Boon and van der Bruggen, J Exp Med 183: 725-9 (1996); van der Bruggen et al., Science 254: 1643-7 (1991); Brichard et al., J Exp Med 178: 489-95 (1993); Kawakami et al., J Exp Med 180: 347-52 (1994); Shichijo et al., J Exp Med 187: 277-88 (1998); Chen et al., Proc Natl Acad Sci USA 94: 1914-8 (1997); Harris, J Natl Cancer Inst 88: 1442-55 (1996); Butterfield et al., Cancer Res 59: 3134-42 (1999); Vissers et al., Cancer Res 59: 5554-9 (1999); van der Burg et al., J Immunol 156: 3308-14 (1996); Tanaka et al., Cancer Res 57: 4465-8 (1997); Fujie et al., Int J Cancer 80: 169-72 (1999); Kikuchi et al., Int J Cancer 81: 459-66 (1999); Oiso et al., Int J Cancer 81: 387-94 (1999).

It has been repeatedly reported that peptide-stimulated peripheral blood mononuclear cells (PBMCs) from certain healthy donors produce significant levels of IFN-γ in response to the peptide, but rarely exert cytotoxicity against tumor cells in an HLA-A24 or -A0201 restricted manner in ⁵¹Cr-release assays (Kawano et al., Cancer Res 60: 3550-8 (2000); Nishizaka et al., Cancer Res 60: 4830-7 (2000); Tamura et al., Jpn J Cancer Res 92: 762-7 (2001)). However, both of HLA-A24 and HLA-A020 are popular HLA alleles in Japanese, as well as Caucasian populations (Date et al., Tissue Antigens 47: 93-101 (1996); Kondo et al., J Immunol 155: 4307-12 (1995); Kubo et al., J Immunol 152: 3913-24 (1994); Imanishi et al., Proceeding of the eleventh International Histocompatibility Workshop and Conference Oxford University Press, Oxford, 1065 (1992); Williams et al., Tissue Antigen 49: 129 (1997)). Thus, antigenic peptides of cancers presented by these HLAs may be especially useful for the treatment of cancers among Japanese and Caucasian populations. Further, it is known that the induction of low-affinity CTL *in vitro* usually results from the use of a peptide at a high concentration, generating a high level of specific peptide/MHC complexes on antigen presenting cells (APCs), which will effectively activate these CTL (Alexander-Miller et al., Proc Natl Acad Sci USA 93: 4102-7 (1996)).

Previously, through gene expression profile analysis using a genome-wide DNA microarray, it has been shown that a polynucleotide (Gene 2089) encoding a polypeptide of sequence SEQ ID NO:8 (that corresponds to SEQ ID NO: 4 of the present application) is upregulated in breast cancer cell lines among numerous other genes (WO03/065006). Furthermore, it has been shown by DNA microarray that the expression of the sequences of SEQ ID NO: 111-112 (corresponding to the sequences SEQ ID NOs: 3 to 4 of the present application) is enhanced in breast cancer cells (US2002/156263).

To disclose the mechanism of breast carcinogenesis and identify novel diagnostic markers and/or drug targets for the treatment of these tumors, the present inventors analyzed the expression profiles of genes in breast carcinogenesis using a genome-wide cDNA microarray containing 27,648 genes. From the pharmacological point of view, suppressing oncogenic signals is easier in practice than activating tumor-suppressive effects. Thus, the present inventors searched for genes that were up-regulated during breast carcinogenesis.

### SUMMARY OF THE INVENTION

Accordingly, in an effort to understand the carcinogenic mechanisms associated with cancer and identify potential targets for developing novel anti-cancer agents, large scale analyses of gene expression patterns in purified populations of breast cancer cells were performed using a cDNA microarray representing 27,648 genes. More particularly, to isolate novel molecular targets for treatments of breast cancer, using a combination of cDNA microarray and laser beam micro-dissection, precise genome-wide expression profiles of 77 breast tumors were examined, including 8 ductal carcinomas in situ (DCIS) and 69 invasive ductal carcinomas (IDC).

Among the up-regulated genes, B1194 was identified designed hypothetical protein FLJ 10252, that was more than two-fold over-expressed in 24 of 41 (59%) breast cancer cases for which expression data was available, especially in 20 of 36 (56%) cases with well-differentiated typed breast cancer specimens. Also identified was A2282, designed maternal embryonic leucine zipper kinase (MELK), that was more than three-fold over-expressed in 25 of 33 (76%) breast cancer cases for which expression data was available, especially in 10 of 14 (71%) cases with moderately-differentiated typed breast cancer specimens. Subsequent semi-quantitative RT-PCR confirmed that B1194 and A2282 were up-regulated in clinical breast cancer specimens and breast cancer cell lines as compared to normal human tissues, including breast ductal cells or normal breast. Northern blot analysis also revealed that the approximately 2.4 kb transcript of B1194 and A2282 were expressed exclusively in testis (B1194) and breast cancer cell lines (B 1194 and A2282). Immunocytochemical staining indicated that exogenous B1194 was localized to the nucleus apparatus in COS7 cells. Treatment of breast cancer cells with small interfering RNAs (siRNAs) effectively inhibited the expression of B 1194 and A2282, and suppressed cell/tumor growth of breast cancer cell line T47D and/or MCF7, suggesting that these genes play a key role in cell growth proliferation. These findings suggest that over-expression of B 1194 and A2282 may be involved in breast tumorigenesis and may provide promising strategies for specific treatment for breast cancer patients.

Thus, novel genes B 1194 and A2282 that were significantly over-expressed in breast cancer cells were isolated and it was confirmed by semi-quantitative RT-PCR and Northern blot analysis that the expression pattern of B 1194 and that among of variants of A2282, V1, V2, and V3, were specifically over-expressed in breast cancer cells. It was reported previously that ESTs of both B 1194 and A2282 were up-regulated in a non-small cell lung cancer (WO 2004/031413). However, the relationship of these genes to breast cancer was previously unknown. Furthermore, the full length nucleotide sequence of these genes is provided.

Accordingly, disclosed are novel proteins involved in the proliferation mechanism of breast cancer cells and the genes encoding such proteins, as well as methods for producing and using the same in the diagnosis and treatment of breast cancer.

Among the transcripts that were commonly up-regulated in breast cancers, novel human genes *B1194, A2282V1, A2282V2* and *A2282V3* were identified on chromosome band 1q41 and 9p13.1, respectively. Gene transfer of *B1194, A2282V1, A2282V2* and *A2282V3* promoted proliferation of cells. Furthermore, reduction of *B1194, A2282V1, A2282V2* and *A2282V3* expression by transfection of their specific antisense S-oligonucleotides or small interfering RNAs inhibited the growth of breast cancer cells. Many anticancer drugs, such as inhibitors of DNA and/or RNA synthesis, metabolic suppressors, and DNA intercalators, are not only toxic to cancer cells but also for normally growing cells. However, agents suppressing the expression of B 1194 may not adversely affect other organs due to the fact that normal expression of the gene is restricted to the testis, and thus may be of great importance for treating cancer.

Thus, provided are isolated genes, *B1194, A2282V1, A2282 V2* and *A2282V3,* which are candidates as diagnostic markers for cancer as well as promising potential targets for developing new strategies for diagnosis and effective anti-cancer agents. Further, the present invention provides polypeptides encoded by these genes, as well as the production and the use of the same. More specifically, the present invention provides the following:

The present disclosure also provides novel human polypeptides, B 1194, A2282V1, A2282V2, and A2282V3, or functional equivalents thereof, that promote cell proliferation and are up-regulated in cell proliferative diseases, such as breast cancer.

In a preferred embodiment, the B1194 polypeptide includes a putative 528 amino acid protein. B 1194 is encoded by the open reading frame of SEQ ID NO: 1. The B1194 polypeptide preferably includes the amino acid sequence set forth in SEQ ID NO: 2. The present disclosure also provides an isolated protein encoded from at least a portion of the *B1194* polynucleotide sequence, or polynucleotide sequences at least 30%, and more preferably at least 40% complementary to the sequence set forth in SEQ ID NO: 1.

In a preferred embodiment, the A2282V1, A2282V2, and A2282V3 polypeptides include a putative 651, 619, and 580 amino acid protein encoded by the open reading frame of SEQ ID NO: 3, 5, and 7, respectively. The A2282V1, A2282V2, and A2282V3 polypeptides preferably include the amino acid sequences set forth in SEQ ID NO: 4, 6, and 8, respectively. The present disclosure also provides an isolated protein encoded from at least a portion of the *A2282V1, A2282V2,* and *A2282V3* polynucleotide sequences, or polynucleotide sequences at least 15%, and more preferably at least 25% complementary to the sequence set forth in SEQ ID NO: 3, 5, and 7, respectively.

The present disclosure further provides novel human genes, *B1194, A2282V1, A2282V2* and *A2282V3,* whose expressions are markedly elevated in a great majority of breast cancers as compared to corresponding non-cancerous tissues. The isolated *B1194* gene includes a polynucleotide sequence as described in (SEQ ID NO: 1. In particular, the *B1194* cDNA includes 2338 nucleotides that contain an open reading frame of 1587 nucleotides (SEQ ID NO: 1). The present disclosure further encompasses polynucleotides which hybridize to and which are at least 30%, and more preferably at least 40% complementary to the polynucleotide sequence set forth in SEQ ID NO: 1, to the extent that they encode a B1194 protein or a functional equivalent thereof. Examples of such polynucleotides are degenerates and allelic mutants of SEQ ID NO: I. On the other hand, the isolated *A2282V1, A2282V2,* and *A2282V3* genes include a polynucleotide sequence as described in SEQ ID NO: 3,5, and 7, respectively. In particular, the *A2282V1, A2282V2,* and *A2282V3* cDNAs include 2501, 2368, and 2251 nucleotides that contain an open reading frame of 1956, 1860, and0 1743 nucleotides, respectively (SEQ ID NO: 3, 5, and 7, respectively). The present disclosure further encompasses polynucleotides which hybridize to and which are at least 15%, and more preferably at least 25% complementary to the polynucleotide sequences set forth in SEQ ID NO: 3, 5, and 7, respectively, to the extent that they encode an A2282V1, A2282V2, or A2282V3 protein or a functional equivalent thereof. Examples of such polynucleotides are degenerates and allelic mutants of SEQ ID NOs: 3, 5, and 7.

As used herein, an isolated gene is a polynucleotide the structure of which is not identical to that of any naturally occurring polynucleotide or to that of any fragment of a naturally occurring genomic polynucleotide spanning more than three separate genes. The term therefore includes, for example, (a) a DNA which has the sequence of part of a naturally occurring genomic DNA molecule in the genome of the organism in which it naturally occurs; (b) a polynucleotide incorporated into a vector or into the genomic DNA of a prokaryote or eukaryote in a manner such that the resulting molecule is not identical to any naturally occurring vector or genomic DNA; (c) a separate molecule, such as a cDNA, a genomic fragment, a fragment produced by polymerase chain reaction (PCR), or a restriction fragment; and (d) a recombinant nucleotide sequence that is part of a hybrid gene, i.e., a gene encoding a fusion polypeptide.

Accordingly, in one aspect, the disclosure provides an isolated polynucleotide that encodes a polypeptide described herein or a fragment thereof. Preferably, the isolated polypeptide includes a nucleotide sequence that is at least 60% identical to the nucleotide sequence shown in SEQ ID NO: 1, 3, 5, or 7. More preferably, the isolated nucleic acid molecule is at least 65%, 70%, 75%, 80%, 85%, 90%, 91%, 92%, 93%, 94%, 95%, 96%, 97%, 98%, 99%, or more, identical to the nucleotide sequence shown in SEQ ID NO: 1, 3, 5, or 7. In the case of an isolated polynucleotide which is longer than or equivalent in length to the reference sequence, *e.g*., SEQ ID NO: 1, 3, 5, or 7, the comparison is made with the full length of the reference sequence. Where the isolated polynucleotide is shorter than the reference sequence, *e.g*., shorter than SEQ ID NO: 1,3,5, or 7, the comparison is made to segment of the reference sequence of the same length (excluding any loop required by the homology calculation).

The present disclosure also provides a method of producing a protein by transfecting or transforming a host cell with a polynucleotide sequence encoding a B 1194, A2282V1, A2282V2, or A2282V2 protein, and expressing the polynucleotide sequence. In addition, the present disclosure provides vectors comprising a nucleotide sequence encoding a B1194, A2282V1, A2282V2, or A2282V3 protein, and host cells harboring a polynucleotide encoding a B1194, A2282V1, A2282V2, or A2282V3 protein. Such vectors and host cells may be used for producing the B 1194, A2282V 1, A2282V2, and A2282V3 proteins.

An antibody that recognizes a B 1194, A2282V1, A2282V2, or A2282V3 protein is also provided. In part, an antisense polynucleotide (e.g., antisense DNA), ribozyme, and siRNA (small interfering RNA) of the *B1194, A2282V1, A2282V2,* or *A2282V3* gene is also provided.

The present disclosure further provides a method for diagnosis of breast cancer that includes the step of determining an expression level of a *B1194, A2282V1, A2282V2,* or *A2282V3* gene in a biological sample of specimen and comparing the expression level of the *B1194, A2282V1, A2282V2,* or *A2282V3* gene with that in normal sample, wherein a high expression level of the *B1194, A2282V1, A2282V2,* or *A2282V3* gene in the sample is indicative of breast cancer.

Further, a method of screening for a compound useful in the treatment of breast cancer is provided. The method includes the step of contacting a B 1194, A2282V1, A2282V2, or A2282V3 polypeptide with test compounds, and selecting test compounds that bind to the B1194, A2282V1, A2282V2, or A2282V3 polypeptide.

The present invention further provides a method of screening for a useful in the treatment of breast cancer, wherein the method includes the step of contacting a B 1194, A2282V1, A2282V2, or A2282V3 polypeptide with a test compound, and selecting the test compound that suppresses the biological activity of the B1194, A2282V1, A2282V2, or A2282V3 polypeptide.

The present disclosure also provides a pharmaceutical composition useful in the treatment of breast cancer. The pharmaceutical composition may be, for example, an anti-cancer agent. The pharmaceutical composition can be described as at least a portion of the antisense S-oligonucleotides or siRNA of the *B1194, A2282V1, A2282V2,* or *A2282V3* polynucleotide sequence shown and described in SEQ ID NO: 1, 3, 5, or 7 respectively. Examples of suitable target sequences of siRNA include the nucleotide sequences set forth in SEQ ID NOs: 38, 39, 40, and 41. The target sequence of siRNA for *B1194,* including those having the nucleotide sequence of SEQ ID NO: 38 or 39, may be suitably used to treat breast cancer; the target sequence of siRNA for *A2282V1, A2282V2,* or *A2282V3,* including those having the nucleotide sequence of SEQ ID NO: 40 or 41, may also be suitably used to treat breast cancer. The pharmaceutical compositions of the present invention also include those compounds selected by the present methods of screening for compounds useful in the treatment of breast cancer.

The course of action of the pharmaceutical composition is desirably to inhibit growth of the cancerous cells. The pharmaceutical composition may be applied to mammals including humans and domesticated mammals.

The present invention further provides methods for treating breast cancer using the pharmaceutical composition provided by the present invention.

In addition, the present disclosure provides a method for treating or preventing breast cancer comprising the step of administering a B1194, A2282V1, A2282V2, or A2282V3 polypeptide. It is expected that anti-tumor immunity would be induced by the administration of such a B1194, A2282V1, A2282V2, or A2282V3 polypeptide. Thus, the present disclosure also provides a method for inducing anti-tumor immunity comprising the step of administering the B 1194, A2282V1, A2282V2, or A2282V3 polypeptide, as well as pharmaceutical compositions for treating or presenting breast cancer comprising the B1194, A2282V1, A2282V2, or A228V3 polypeptide.

These and other objects and features of the disclosure of the invention will become more fully apparent when the following detailed description is read in conjunction with the accompanying figures and examples.

### BRIEF DESCRIPTION OF THE DRAWINGS

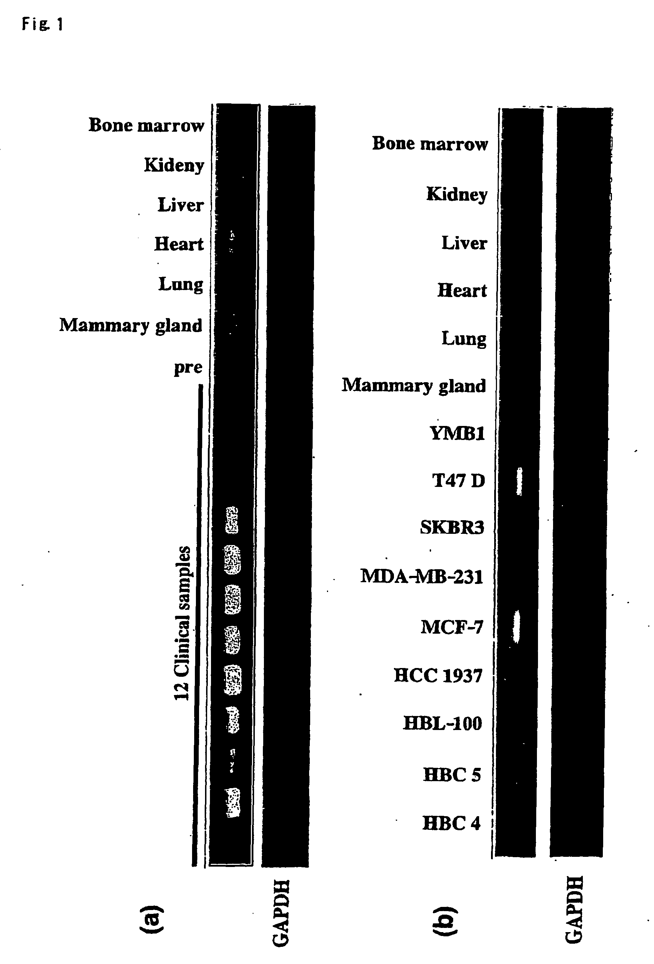

Figure 1 is comprised of a series of photographs showing the results of RT-PCR to validate the over expression of genes B 1194. Lines 1 to 12 of Figure 1(a) are clinical samples subjected to one round of T7 based amplification prior to reverse-transcription, and lines 1 to 9 of Figure 1(b) are breast cancer cell lines. The abbreviation "pre" represents a mixture of normal breast ductal cells and is used herein as a universal control on microarray experiment.

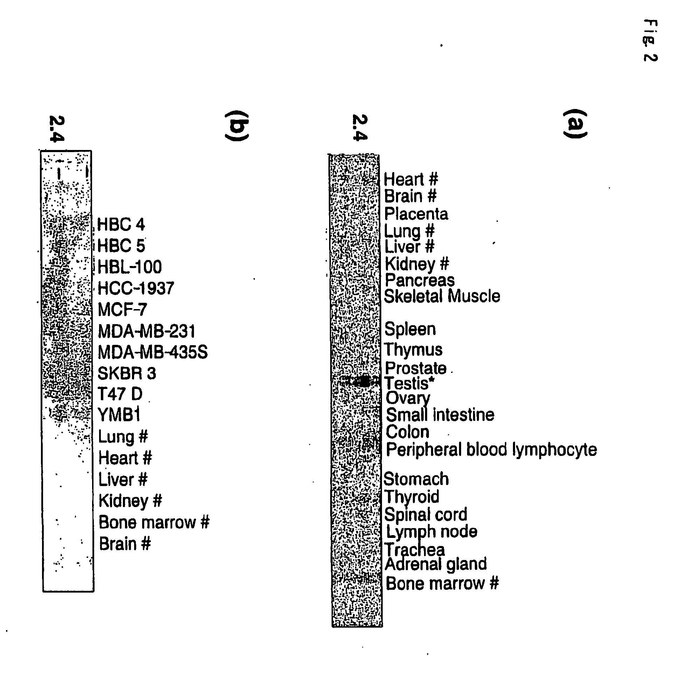

Figure 2 is comprised of a series of photographs showing the results of Northern blot analysis, particularly the expression pattern of B1194 in (a) multiple normal tissues, (b) breast cancer cell lines, respectively. "#" indicates vital organs.

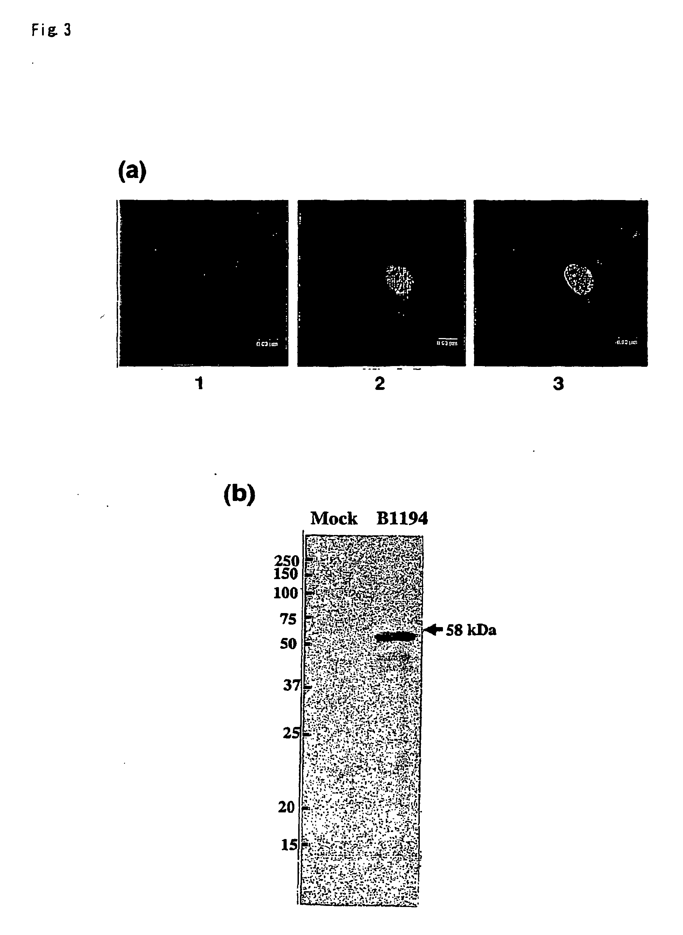

Figure 3(a) is comprised of a series of photographs depicting the subcellular localization of B1194 protein in COS7 cells. DAPI (nucleus); B1194 (FITC) and merge of them are demonstrated in photograph No. 1, 2 and 3 respectively. Figure 3(b) is a photograph of Western blot analysis of B1194 protein.

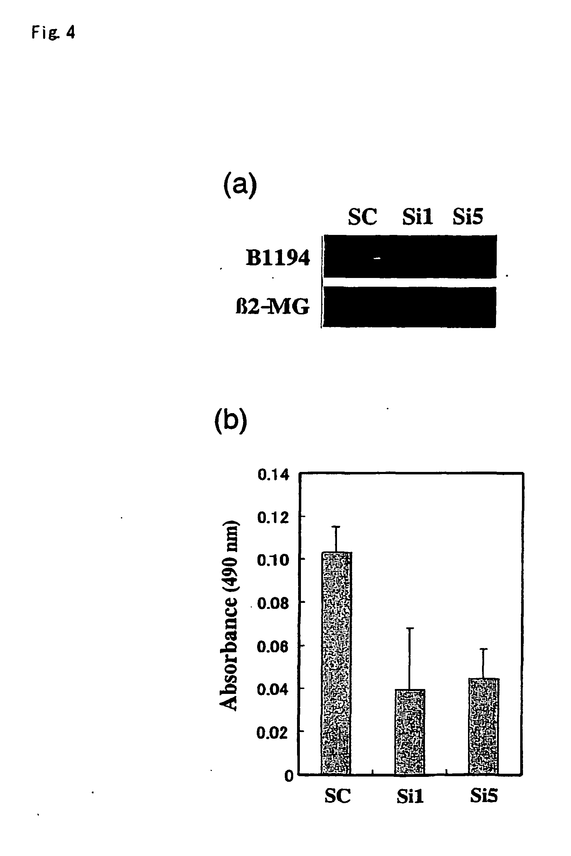

Figure 4(a) is comprised of a series of photographs depicting the results of semi-quantitative RT-PCR, particularly showing the knock down effect of endogenous B1194 in T47D cell lines. Figure 4(b) is a bar chart depicting the results of an MTT assay that shows low proliferation in si 1 and si5 culture.

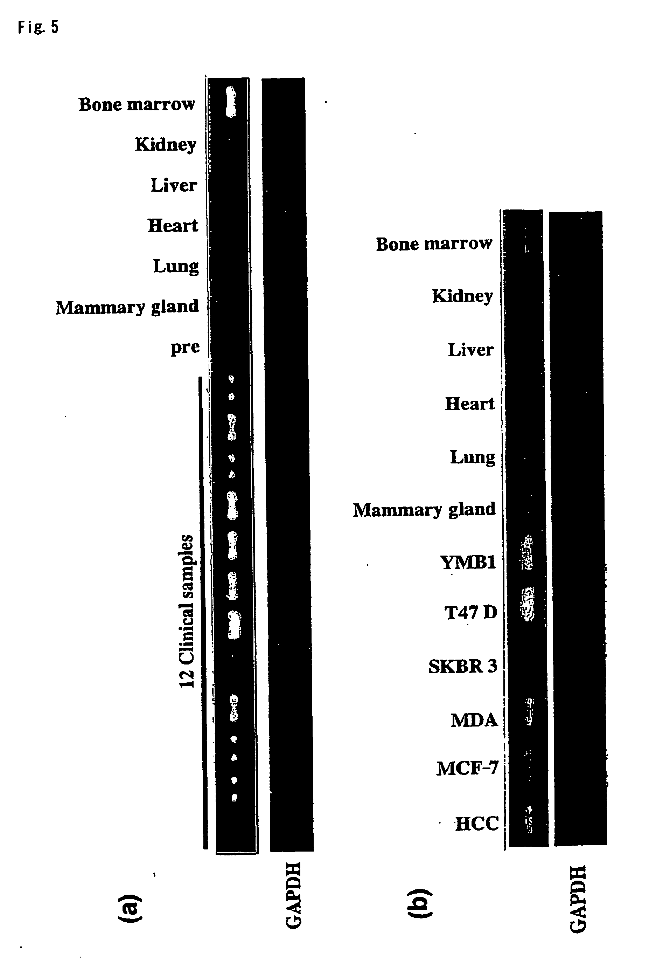

Figure 5 is comprised of a series of photographs depicting the results of semi quantitative RT-PCR, particularly the expression of A2282. Lines 1 to 12 of Figure 5(a) are clinical samples subjected to one round of T7 based amplification prior to reverse-transcription. Lines 1 to 6 of Figure 5 (b) are cancer cell lines. Again, the abbreviation "pre" represents a mixture of normal breast ductal cells and is used herein as a universal control for the cDNA microarray analysis.

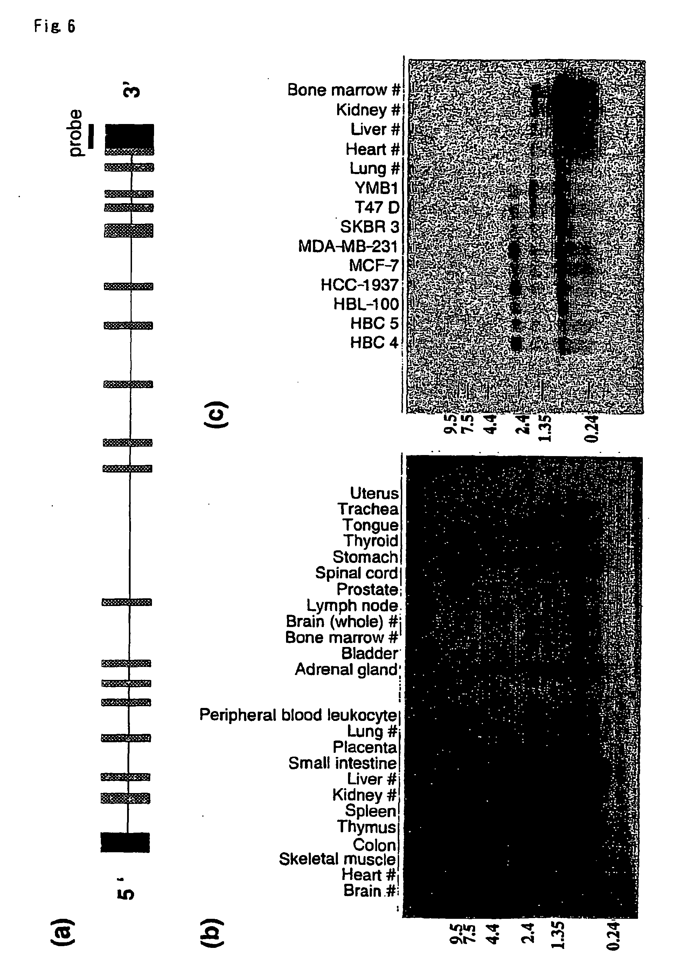

Figure 6(a) depicts the genomic structure of A2282. Figures 6(b) and 6(c) are photographs depicting the results of Northern blot analysis, particularly showing the expression pattern of (b) multiple normal tissues, (c) breast cancer cell lines and normal tissues, respectively. "#" indicates vital organs.

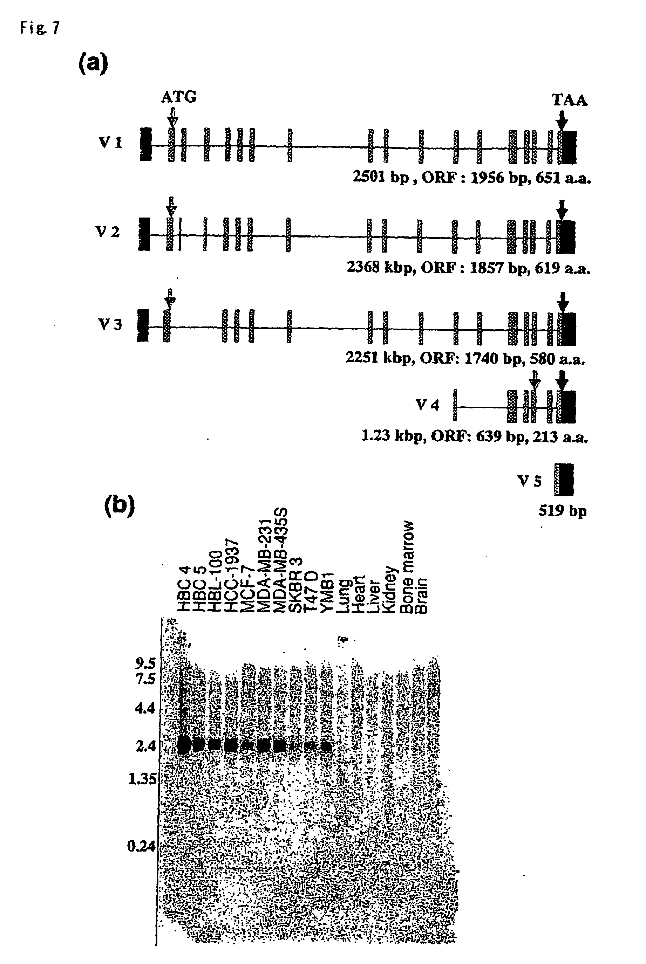

Figure 7(a) depicts the structure of the A2282 variants. Five transcripts of A2282 were isolated from cDNA library screening. ATG and TAA represent the translation initiation and termination codon, respectively. The black and shaded blocks indicate untranslated regions and coding sequences. Figure 7(b) is a photograph depicting the results of Northern blot analysis in breast cancer cell lines and normal tissues.

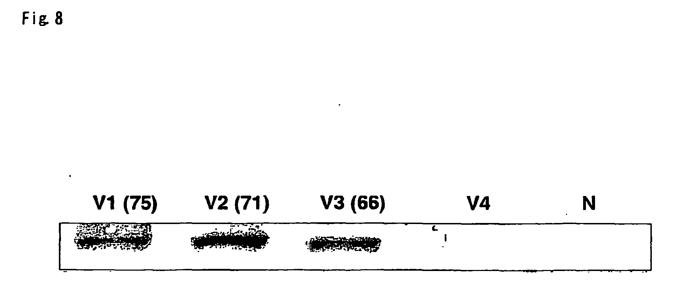

Figure 8 is a photograph showing the translational capability of four A2282 transcripts *in vitro.* The predicted protein molecular weight is indicated in the bracket next to each variant. N represents negative control.

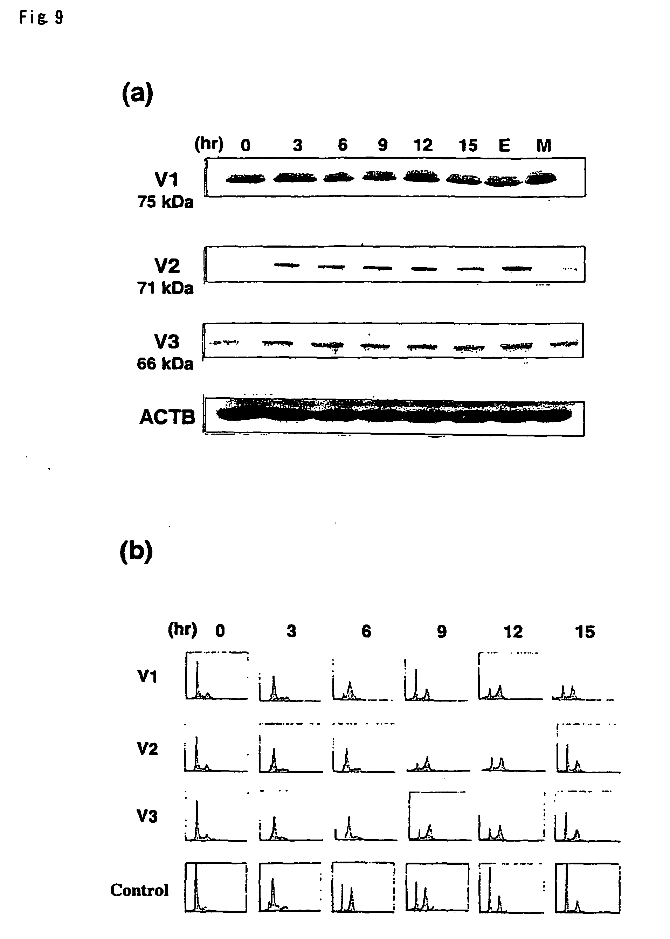

Figure 9(a) is a photograph showing the results of Western blot analysis, particularly showing the expression of the A2282 protein. The abbreviations "E" and "M" indicate no drug treatment (exponential growth) and mitotic phase, respectively. βactin (ACTB) was used as an internal control. Equal amounts of total protein (10µg) were loaded in each line. Figure 9 (b) is comprised of a series of graphs showing the results of cell cycle analysis. The effect of three A2282 transcripts on cell cycle transition is examined in synchronized G1 phase of HeLa cells by flow cytometry. Non transfected cells are used as a control.

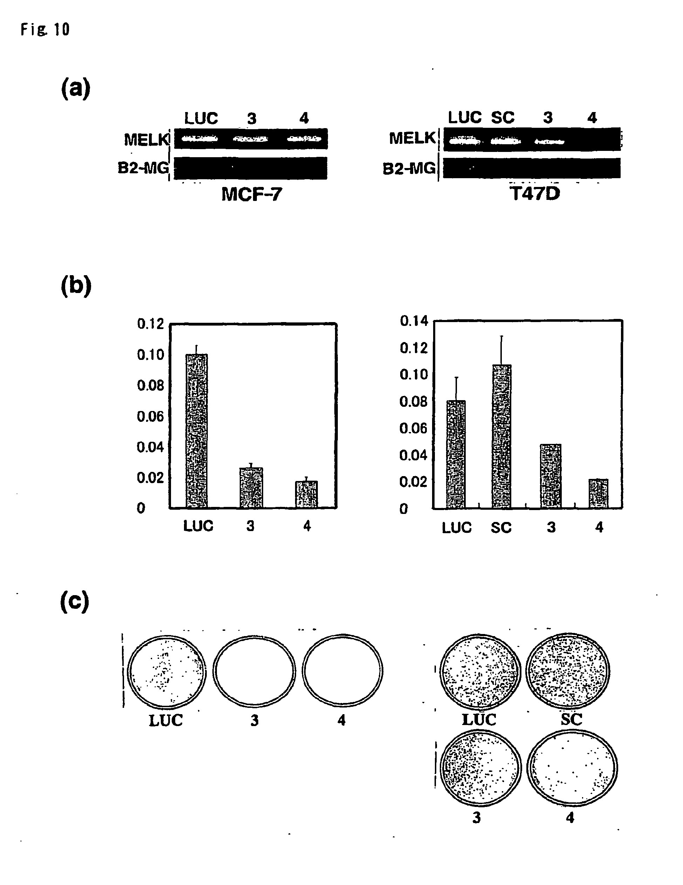

Figure 10(a) are photographs of semi-quantitative RT-PCR showing knock down effect of endogenous A2282 in MCF-7 and T47D cell lines. Figure 10(b) is comprised of a series of bar charts depicting the results of an MTT assay, particularly showing low proliferation in No. 3 and No. 4 culture. Figure 10(c) is comprised of a series of photographs depicting the results of a colony-formation assay that demonstrates a decrease in colony density in A2282 gene knock-down cultures.

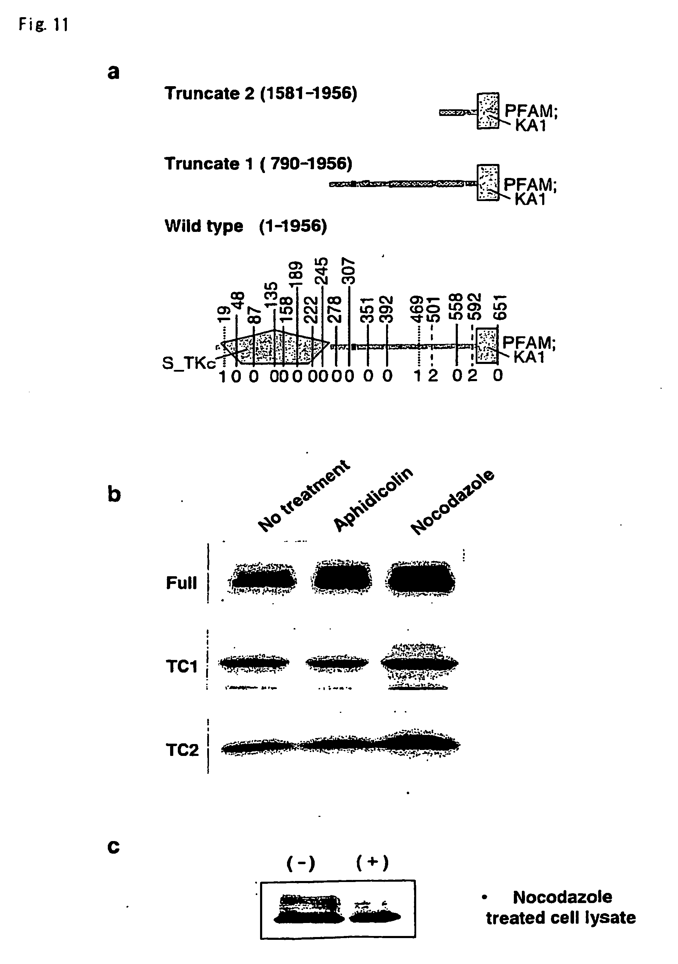

Figure 11 demonstrates that the A2282 protein is phosphorylated at the kinase domain. Specifically, Figure 11 (a) is a systematic representation of wild type and two truncated A2282 proteins. Figure 11 (b) depicts the results of western blot analysis for all three transcripts using anti-HA antibody. Figure 11(c) depicts the results of the λ-PPase assay which confirmed phosphorylation of wild type A2282 protein.

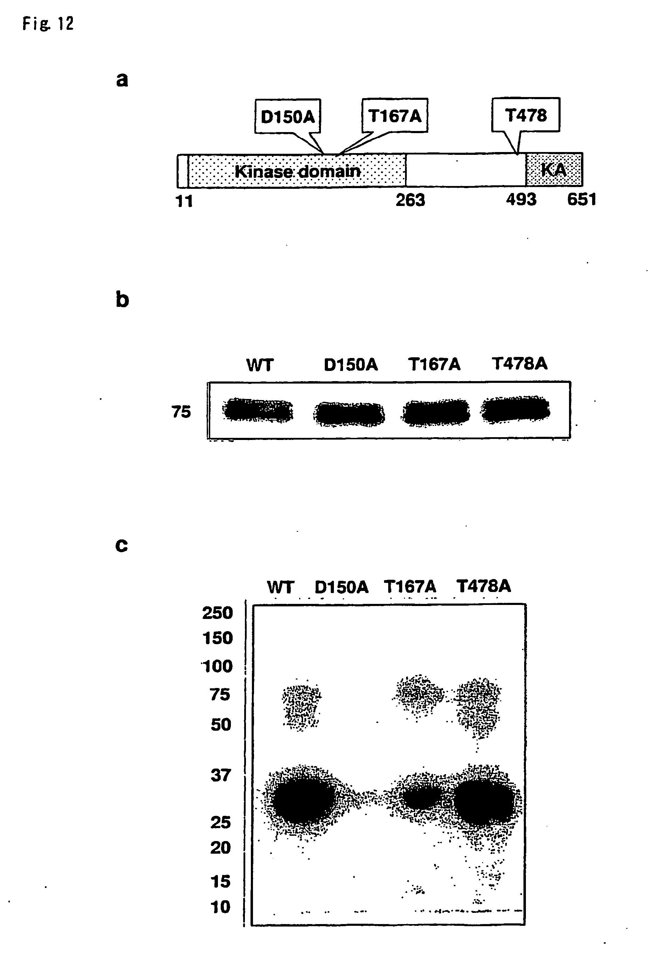

Figure 12 depicts the results of immunoprecipitation and immune complex kinase assays. Specifically, Figure 12(a) is a systematic representation of wild type and mutant transcripts. Figure 12(b) examines the phosphorylation status of three mutants in HEK 293 cell lines. Figure 12(c) assesses the kinase activity of three mutants by immune complex kinase assay. Histone H1 was used as *in vitro* substrate.

### DETAILED DESCRIPTION OF THE PREFERRED EMBODIMENTS

### 1. Overview

The present application identifies novel human genes *B1194, A2282V1, A2282V2,* and *A2282V3* whose expression is markedly elevated in breast cancer as compared to corresponding non-cancerous tissues. The *B1194* cDNA consists of 2338 nucleotides that contain an open reading frame of 1587 nucleotides as set forth in SEQ ID NO: 1. The open reading frame encodes a putative 528-amino acid protein. Similarly, the *A2282V1, A2282V2,* and *A2282V3* cDNA include 2501, 2368, and 2251 nucleotides that contain an open reading frame of 1956, 1860, and 1743 nucleotides, respectively (SEQ ID NO: 3, 5, and 7, respectively). The nucleotide sequences of SEQ ID NO: 5 and 7 (*A2282V2* and *A2282V3,* respectively) were submitted with DNA Databank of Japan (DDBJ), and accession numbers AB183427 and AB183428 were respectively assigned. Since the expression of the protein was up-regulated in breast cancer, the proteins were dubbed A2282V1, A2282V2, and A2282V3 (up-regulated in breast cancer).

Consistently, exogenous expression of *B1194, A2282V1, A2282V2,* or *A2282V3* in cells conferred increased cell growth, while suppression of expression with antisense S-oligonucleotides or small interfering RNA (siRNA) resulted in significant growth-inhibition of cancerous cells. These findings suggest that B1194, A2282V1, A2282V2, and A2282V3 render oncogenic activities to cancer cells, and that inhibition of the activity of these proteins could be a promising strategy for the treatment of cancer.

More particularly, herein it was discovered that B1194, designed to FLJ-10252 hypothetical protein gene, is significantly up-regulated through the expression profiles of breast cancer. This finding was confirmed by semi-quantitative RT-PCR using clinical samples and Northern blot analysis. In addition, the expression of this gene was found to be a cancer specific rather than an ubiquitous event. Treatment of breast cancer cells with small interfering RNAs (siRNAs) effectively inhibited expression of B1194 and suppressed cell/tumor growth of breast cancer cell line T47D. These findings taken together suggest that the FLJ-10252 hypothetical protein is a prominent novel molecular candidate for breast cancer drug development.

In addition, through the precise expression profiles of breast cancer by means of genome wide cDNA microarray, novel gene A2282 that were significantly over-expressed in breast cancer cells as compared to normal human tissues was identified. Treatment of breast cancer cells with siRNA effectively inhibited expression of A2282 and significantly suppressed cell/tumor growth of breast cancer.

A2282, designed to MELK, a new member of the KIN1/PAR-I/MARK family identified during development of the embryos of xenopus and mouse (Blot J, et al., (2002). Dev Biol, 241, 327-338; Heyer BS, et al., (1999). Dev Dyn, 215, 344-351), was selected for study due to its significant elevated-expression in breast cancer. Five variants of human MELK gene were identified, and, from among them, the approximately 2.4 kb transcripts showed cancer specific expression, whereas two other transcripts were not able to be translated in almost all organs. Sequence analysis revealed internal deletions in the catalytic domain at N-terminus in two transcripts, which were subsequently designated as V2 and V3. These deletions caused a premature translational termination leading to translation of a shorter protein with an alternative start codon in the same reading frame, generating novel initiation translational codons of V2 or V3, and producing the deleted N-terminal portion. By alignment of the amino acid sequences of the three variants, it was clearly demonstrated that V2 and V3 still retained the partial kinase domain but not the putative transmembrane region. Nevertheless, it is unclear whether this deletion affects the kinetic activity of this protein or not.

In order to characterize these transcripts, the phosphorylation status of these variants along cell cycle was examined. In agreement with previous studies (Davezac N, et al., (2002). Oncogene, 21, 7630-7641), V1 was shown to be strongly phosphorylated during mitosis. However, no phosphorylated V2 and V3 were observed in any cell cycle phases. Interestingly, transient expression of these transcripts in synchronized HeLa cells had slightly different effects. Expression of V1 resulted in shortening of the first cell cycle, follows by a G2/M phase arrest. By contrast, induction of V2 and V3 led to a prolonged first cell cycle comparing with untransfected control cells. Despite the discrepancy of initial outcome, all the variants were eventually able to arrest the cells at G2/M phase. Similar results have also been reported (Davezac N, et al., (2002). Oncogene, 21, 7630-7641; Vulsteke V, et al., (2004). J Biol Chem, 279, 8642-7). In addition, recent evidence suggests that the C-terminal domain of MELK is likely to be the strong inhibitor of the kinase activity of MELK (Vulsteke V, et al., (2004). J Biol Chem, 279, 8642-7). This finding speculates that the kinase domain of MELK might contribute to a shortening of the cell cycle in breast cancer, and its activity might be strictly controlled under negative regulation of its C-terminal domain.

In conclusion, these findings show that A2282 is an indispensable cancer specific gene essential for cancer cell growth via unidentified signaling pathway. Based on these results, MELK appears to be a promising molecular target for breast cancer treatment.

### II. Definitions

The words "a", "an", and "the" as used herein mean "at least one" unless otherwise specifically indicated.

In the context of the present invention, "inhibition of binding" between two proteins refers to at least reducing binding between the proteins. Thus, in some cases, the percentage of binding pairs in a sample will be decreased compared to an appropriate (*e.g*., not treated with test compound or from a non-cancer sample, or from a cancer sample) control. The reduction in the amount of proteins bound may be, *e.g*., less than 90%, 80%, 70%, 60%, 50%, 40%, 25%, 10%, 5%, 1% or less (*e.g*., 0%), than the pairs bound in a control sample.

A "pharmaceutically effective amount" of a compound is a quantity that is sufficient to treat and/or ameliorate cancer in an individual. An example of a pharmaceutically effective amount may an amount needed to decrease the expression of B1194, A2282V1, A2282V2, or A2282V3 when administered to an animal. The decrease may be, e.g., at least a 5%, 10%, 20%, 30%, 40%, 50%, 75%, 80%, 90%, 95%, 99%, or 100% change in expression.

The phrase "pharmaceutically acceptable carrier" refers to an inert substance used as a diluent or vehicle for a drug.

In the present disclosure, the term "functionally equivalent" means that the subject polypeptide has the activity to promote cell proliferation like the B1194, A2282V1, A2282V2, or A2282V3 proteins and to confer oncogenic activity to cancer cells. In addition, a functionally equivalent polypeptide may have the protein kinase activity associated with the A2282V1, A2282V2, and A2282V3 proteins. Assays for determining such activities are well known in the art. For example, whether the subject polypeptide has a cell proliferation activity or not can be judged by introducing the DNA encoding the subject polypeptide into a cell expressing the respective polypeptide, and detecting promotion of proliferation of the cells or increase in colony forming activity. Such cells include, for example, COS7 and NIH3T3 cells for B 1194 and A2282V 1, A2282V2, A2282V3.

The terms "isolated" and "biologically pure" refer to material that is substantially or essentially free from components which normally accompany it as found in its native state. However, the term "isolated" is not intended to refer to the components present in an electrophoretic gel or other separation medium. An isolated component is free from such separation media and in a form ready for use in another application or already in use in the new application/milieu.

In the context of the present invention, a "percentage of sequence identity" is determined by comparing two optimally aligned sequences over a comparison window, wherein the portion of the polynucleotide sequence in the comparison window may comprise additions or deletions (*i.e*., gaps) as compared to the reference sequence (*e.g*., a polypeptide of the invention), which does not comprise additions or deletions, for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity.

The terms "identical" or percent "identity," in the context of two or more nucleic acids or polypeptide sequences, refer to two or more sequences or subsequences that are the same sequences. Two sequences are "substantially identical" if two sequences have a specified percentage of amino acid residues or nucleotides that are the same (*i.e*., 60% identity, optionally 65%, 70%, 75%, 80%, 85%, 90%, or 95% identity over a specified region, or, when not specified, over the entire sequence), when compared and aligned for maximum correspondence over a comparison window, or designated region as measured using one of the following sequence comparison algorithms or by manual alignment and visual inspection. Optionally, the identity exists over a region that is at least about 50 nucleotides in length, or more preferably over a region that is 100 to 500 or 1000 or more nucleotides in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted, *e.g*., by the local homology algorithm of Smith and Waterman (1981) Adv. Appl. Math. 2:482-489, by the homology alignment algorithm of Needleman and Wunsch (1970) J. Mol. Biol. 48:443, by the search for similarity method of Pearson and Lipman (1988) Proc. Nat'l. Acad Sci. USA 85:2444, by computerized implementations of these algorithms (GAP, BESTFIT, FASTA, and TFASTA in the Wisconsin Genetics Software Package, Genetics Computer Group, 575 Science Dr., Madison, WI), or by manual alignment and visual inspection (*see, e.g.,* Ausubel et al., Current Protocols in Molecular Biology (1995 supplement)).

Two examples of algorithms that are suitable for determining percent sequence identity and sequence similarity are the BLAST and BLAST 2.0 algorithms, which are described in Altschul et al. (1977) Nuc. Acids Res. 25:3389-3402, and Altschul et al. (1990) J. Mol. Biol. 215:403-410, respectively. Software for performing BLAST analyses is publicly available through the National Center for Biotechnology Information. This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra).* These initial neighborhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a word length (W) of 11, an expectation (E) of 10, M=5, N=-4 and a comparison of both strands. For amino acid sequences, the BLASTP program uses as defaults a word length of 3, and expectation (E) of 10, and the BLOSUM62 scoring matrix (*see* Henikoff and Henikoff (1989) Proc. Natal Acad. Sci. USA 89:10915) alignments (B) of 50, expectation (E) of 10, M=5, N=-4, and a comparison of both strands.

The BLAST algorithm also performs a statistical analysis of the similarity between two sequences (*see, e.g.,* Karlin and Altschul (1993) Proc. Natl. Acad. Sci. USA 90:5873-7). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The term "antisense oligonucleotides" as used herein means, not only those in which the nucleotides corresponding to those constituting a specified region of a DNA or mRNA are entirely complementary, but also those having a mismatch of one or more nucleotides, as long as the DNA or mRNA and the antisense oligonucleotide can specifically hybridize with the nucleotide sequence of SEQ ID NO: 1, 3, 5, or 7.

Such polynucleotides are contained as those having, in the "at least 15 continuous nucleotide sequence region", a homology of at least 70% or higher, preferably at 80% or higher, more preferably 90% or higher, even more preferably 95% or higher. The algorithm stated herein can be used to determine the homology. Such polynucleotides are useful as probes for the isolation or detection of DNA encoding the polypeptide of the invention as stated in a later example or as a primer used for amplifications.

The terms "label" and "detectable label" are used herein to refer to any composition detectable by spectroscopic, photochemical, biochemical, immunochemical, electrical, optical or chemical means. Such labels include biotin for staining with labeled streptavidin conjugate, magnetic beads (*e.g*., DYNABEADS™), fluorescent dyes (*e.g*., fluorescein, Texas red, rhodamine, green fluorescent protein, and the like), radiolabels (*e.g.,* ³H, ¹²⁵I, ³⁵S, ¹⁴C, or ³²P), enzymes (*e.g*., horse radish peroxidase, alkaline phosphatase and others commonly used in an ELISA), and calorimetric labels such as colloidal gold or colored glass or plastic (*e.g*., polystyrene, polypropylene, latex, etc.) beads. Patents teaching the use of such labels include U.S. Pat. Nos. 3,817,837; 3,850,752; 3,939,350; 3,996,345; 4,277,437; 4,275,149; and 4,366,241. Means of detecting such labels are well known to those of skill in the art. Thus, for example, radiolabels may be detected using photographic film or scintillation counters, fluorescent markers may be detected using a photodetector to detect emitted light. Enzymatic labels are typically detected by providing the enzyme with a substrate and detecting, the reaction product produced by the action of the enzyme on the substrate, and calorimetric labels are detected by simply visualizing the colored label.

The term "antibody" as used herein encompasses naturally occurring antibodies as well as non-naturally occurring antibodies, including, for example, single chain antibodies, chimeric, bifunctional and humanized antibodies, as well as antigen-binding fragments thereof, (*e.g*., Fab', F(ab')₂, Fab, Fv and rIgG). *See also,* Pierce Catalog and Handbook, 1994-1995 (Pierce Chemical Co., Rockford, IL). *See also, e.g.,* Kuby, J., *Immunology,* 3^{rd} Ed., W.H. Freeman & Co., New York (1998). Such non-naturally occurring antibodies can be constructed using solid phase peptide synthesis, can be produced recombinantly or can be obtained, for example, by screening combinatorial libraries consisting of variable heavy chains and variable light chains as described by Huse et al., Science 246:1275-1281 (1989). These and other methods of making, for example, chimeric, humanized, CDR-grafted, single chain, and bifunctional antibodies are well known to those skilled in the art (Winter and Harris, Immunol. Today 14:243-246 (1993); Ward et al., Nature 341:544-546 (1989); Harlow and Lane, Antibodies, 511-52, Cold Spring Harbor Laboratory publications, New York, 1988; Hilyard et al., Protein Engineering: A practical approach (IRL Press 1992); Borrebaeck, Antibody Engineering, 2d ed. (Oxford University Press 1995).

The term "antibody" includes both polyclonal and monoclonal antibodies. The term also includes genetically engineered forms such as chimeric antibodies (*e.g*., humanized murine antibodies) and heteroconjugate antibodies (*e.g*., bispecific antibodies). The term also refers to recombinant single chain Fv fragments (scFv). The term antibody also includes bivalent or bispecific molecules, diabodies, triabodies, and tetrabodies. Bivalent and bispecific molecules are described in, *e.g*., Kostelny et al. (1992) J Immunol 148:1547, Pack and Pluckthun (1992) Biochemistry 31:1579, Holliger et al. (1993) Proc Natl Acad Sci U S A. 90:6444, Gruber et al. (1994) J Immunol :5368, Zhu et al. (1997) Protein Sci 6:781, Hu et al. (1997) Cancer Res. 56:3055, Adams et al. (1993) Cancer Res. 53:4026, and McCartney, et al. (1995) Protein Eng. 8:301.

Typically, an antibody has a heavy and light chain. Each heavy and light chain contain a constant region and a variable region, (the regions are also known as "domain'). Light and heavy chain variable regions contain four "framework" regions interrupted by three hyper-variable regions, also called "complementarity-determining regions" or."CDRs". The extent of the framework regions and CDRs have been defined. The sequences of the framework regions of different light or heavy chains are relatively conserved within a species. The framework region of an antibody, that is the combined framework regions of the constituent lights and heavy chains, serves to position and align the CDRs in three dimensional spaces.

The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, a V_{H} CDR3 is located in the variable domain of the heavy chain of the antibody in which it is found, whereas a V_{L} CDR1 is the CDR1 from the variable domain of the light chain of the antibody in which it is found. References to "V_{H}" refer to the variable region of an immunoglobulin heavy chain of an antibody, including the heavy chain of an Fv, scFv , or Fab. References to "V_{L}" refer to the variable region of an immunoglobulin light chain, including the light chain of an Fv, scFv, dsFv or Fab.

The phrase "single chain Fv" or "scFv" refers to an antibody in which the variable domains of the heavy chain and of the light chain of a traditional two chain antibody have been joined to form one chain. Typically, a linker peptide is inserted between the two chains to allow for proper folding and creation of an active binding site.

A "chimeric antibody" is an immunoglobulin molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity.

A "humanized antibody" is an immunoglobulin molecule that contains minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, a humanized antibody will comprise substantially all of at least one, and typically two, variable domains, in which all or substantially all of the CDR regions correspond to those of a non-human immunoglobulin and all or substantially all of the framework (FR) regions are those of a human immunoglobulin consensus sequence. The humanized antibody optimally also will comprise at least a portion of an immunoglobulin constant region (Fc), typically that of a human immunoglobulin (Jones et al.. Nature 321:522-525 (1986); Riechmann et al. Nature 332:323-327 (1988); and Presta, Curr. Op. Struct. Biol. 2:593-596 (1992)). Humanization can be essentially performed following the method of Winter and coworkers (Jones et al., Nature 321:522-525 (1986); Riechmann et al., Nature 332:323-327 (1988); Verhoeyen et al., Science 239:1534-1536 (1988)), by substituting rodent CDRs or CDR sequences for the corresponding sequences of a human antibody. Accordingly, such humanized antibodies are chimeric antibodies (U.S. Patent No. 4,816,567), wherein substantially less than an intact human variable domain has been substituted by the corresponding sequence from a non-human species.

The terms "epitope" and "antigenic determinant" refer to a site on an antigen to which an antibody binds. Epitopes can be formed both from contiguous amino acids or noncontiguous amino acids juxtaposed by tertiary folding of a protein. Epitopes formed from contiguous amino acids are typically retained on exposure to denaturing solvents whereas epitopes formed by tertiary folding are typically lost on treatment with denaturing solvents. An epitope typically includes at least 3, and more usually, at least 5 or 8-10 amino acids in a unique spatial conformation. Methods of determining spatial conformation of epitopes include, for example, x-ray crystallography and 2-dimensional nuclear magnetic resonance. *See, e.g.,* Epitope Mapping Protocols in Methods in Molecular Biology, Vol. 66, Glenn E. Morris, Ed (1996).

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers, those containing modified residues, and non-naturally occurring amino acid polymer.

The term "amino acid" refers to naturally occurring and synthetic amino acids, as well as amino acid analogs and amino acid mimetics that function similarly to the naturally occurring amino acids. Naturally occurring amino acids are those encoded by the genetic code, as well as those amino acids that are later modified, *e.g*., hydroxyproline, γ-carboxyglutamate, and O-phosphoserine. Amino acid analogs refers to compounds that have the same basic chemical structure as a naturally occurring amino acid, *e.g*., an a carbon that is bound to a hydrogen, a carboxyl group, an amino group, and an R group, e.g., homoserine, norleucine, methionine sulfoxide, methionine methyl sulfonium. Such analogs may have modified R groups (*e.g*., norleucine) or modified peptide backbones, but retain the same basic chemical structure as a naturally occurring amino acid. Amino acid mimetics refers to chemical compounds that have a structure that is different from the general chemical structure of an amino acid, but that functions similarly to a naturally occurring amino acid.

Amino acids may be referred to herein by their commonly known three letter symbols or by the one-letter symbols recommended by the IUPAC-IUB Biochemical Nomenclature Commission. Nucleotides, likewise, may be referred to by their commonly accepted single-letter codes.

The term "recombinant" when used with reference, *e.g*., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, *e.g*., recombinant cells express genes that are not found within the native (non-recombinant) form of the cell or express native genes that are otherwise abnormally expressed, under expressed or not expressed at all. By the term "recombinant nucleic acid" herein is meant nucleic acid, originally formed *in vitro,* in general, by the manipulation of nucleic acid, e.g., using polymerases and endonucleases, in a form not normally found in nature. In this manner, operable linkage of different sequences is achieved. Thus an isolated nucleic acid, in a linear form, or an expression vector formed *in vitro* by ligating DNA molecules that are not normally joined, are both considered recombinant for the purposes of this invention. It is understood that once a recombinant nucleic acid is made and reintroduced into a host cell or organism, it will replicate non-recombinantly, *i.e*., using the *in vivo* cellular machinery of the host cell rather than *in vitro* manipulations; however, such nucleic acids, once produced recombinantly, although subsequently replicated non-recombinantly, are still considered recombinant for the purposes of the invention. Similarly, a "recombinant protein" is a protein made using recombinant techniques, *i.e*., through the expression of a recombinant nucleic acid as depicted above.

Unless otherwise defined, all technical and scientific terms used herein have the same meaning as commonly understood by one of ordinary skill in the art to which this invention belongs. In case of conflict, the present specification, including definitions, will control.

### III. Novel nucleotides, polypeptides, vectors and host cells

The present disclosure encompasses the novel human gene *B1194,* including a polynucleotide sequence as described in SEQ ID NO: 1, as well as degenerates and mutants thereof, to the extent that they encode a B 1194 protein, including the amino acid sequence set forth in SEQ ID NO: 2 or its functional equivalent. Examples of polypeptides functionally equivalent to B 1194 include, for example, homologous proteins of other organisms corresponding to the human B 1194 protein, as well as mutants of human B1194 proteins.

The present disclosure also encompasses novel human genes *A2282V1, A2282V2,* and *A2282V3,* including polynucleotide sequences described in SEQ ID NO: 3, 5, 7 respectively, as well as degenerates and mutants thereof, to the extent that they encode a A2282V1, A2282V2, or A2282V3 protein, including the amino acid sequence set forth in (SEQ ID NO: 4, 6,8 or its functional equivalent. Examples of polypeptides functionally equivalent to A2282V1, A2282V2, or A2282V3 include, for example, homologous proteins of other organisms corresponding to the human A2282V1, A2282V2, or A2282V3 protein, as well as mutants of human A2282V1, A2282V2, or A2282V3 proteins.

Methods for preparing polypeptides functionally equivalent to a given protein are well known by a person skilled in the art and include known methods of introducing mutations into the protein. For example, one skilled in the art can prepare polypeptides functionally equivalent to the human B1194, A2282V1, A2282V2, or A2282V3 protein by introducing an appropriate mutation in the amino acid sequence of either of these proteins by site-directed mutagenesis (Hashimoto-Gotoh et al., Gene 152:271-5 (1995); Zoller and Smith, Methods Enzymol 100: 468-500 (1983); Kramer et al., Nucleic Acids Res. 12:9441-9456 (1984); Kramer and Fritz, Methods Enzymol 154: 350-67 (1987); Kunkel, Proc Natl Acad Sci USA 82: 488-92 (1985); Kunkel, Methods Enzymol 204: 125-139 (1991)). Amino acid mutations can occur in nature, too. The polypeptide of the present disclosure includes those proteins having the amino acid sequences of the human B 1194, A2282V1 , A2282V2," or A2282V3 protein in which one or more amino acids are mutated, provided resulting mutated polypeptides that are functionally equivalent to the human B1194, A2282V 1, A2282V2, or A2282V3 proteins. The number of amino acids to be mutated in such a mutant is generally 10 amino acids or less, preferably 6 amino acids or less, and more preferably 3 amino acids or less.

Mutated or modified proteins, proteins having amino acid sequences modified by substituting, deleting, inserting, and/or adding one or more amino acid residues of a certain amino acid sequence, have been known to retain the original biological activity (Mark et al., Proc Natl Acad Sci USA 81: 5662-6 (1984); Zoller and Smith, Nucleic Acids Res 10:6487-500 (1982); Dalbadie-McFarland et al., Proc Natl Acad Sci USA 79: 6409-13 (1982)).

The amino acid residue to be mutated is preferably mutated into a different amino acid in which the properties of the amino acid side-chain are conserved (a process known as conservative amino acid substitution Examples of properties of amino acid side chains are hydrophobic amino acids (A, I, L, K. F, P, W, Y, V), hydrophilic amino acids (R, D, N, C, E, Q, G, H, K, S, T), and side chains having the following functional groups or characteristics in common: an aliphatic side-chain (G, A, V, L, I, P); a hydroxyl group containing side-chain (S, T, Y); a sulfur atom containing side-chain (C, M); a carboxylic acid and amide containing side-chain (D, N, E, Q); a base containing side-chain (R, K, H); and an aromatic containing side-chain (H, F, Y, W). Note, the parenthetic letters indicate the one-letter codes of amino acids.

Conservative substitution tables providing functionally similar amino acids are well known in the art. Such conservatively modified variants are in addition to and do not exclude polymorphic variants, interspecies homologs, and alleles of the invention. For example, the following eight groups each contain amino acids that are conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M) (*see, e.g.,* Creighton, Proteins (1984)).

An example of a polypeptide to which one or more amino acids residues are added to the amino acid sequence of human B 1194, A2282V1, A2282V2, or A2282V3 protein is a fusion protein containing the human B1194, A2282V1, A2282V2, or A2282V3 protein. Fusion proteins, fusions of the human B1194, A2282V 1, A2282V2, or A2282V3 protein and other peptides or proteins, are included in the present invention. Fusion proteins can be made by techniques well known to a person skilled in the art, such as by linking a DNA encoding a human B1194, A2282V 1, A2282V2, or A2282V3 protein of the present invention with DNA encoding another peptide or protein, so that the frames match, inserting the fusion DNA into an expression vector, and expressing it in a host. There is no restriction as to the peptides or proteins fused to the protein of the present invention.

Known peptides that can be used as peptides that are fused to the protein of the present invention include, for example, FLAG (Hopp et al., Biotechnology 6: 1204-10 (1988)), 6xHis containing six His (histidine) residues, 10xHis, Influenza agglutinin (HA), human c-myc fragment, VSP-GP fragment, p18HIV fragment, T7-tag, HSV-tag, E-tag, SV40T antigen fragment, lck tag, β-tubulin fragment, B-tag, Protein C fragment, and the like. Examples of proteins that may be fused to a protein of the invention include GST (glutathione-S-transferase), Influenza agglutinin (HA), immunoglobulin constant region, β-galactosidase, MBP (maltose-binding protein), and such.

Fusion proteins can be prepared by fusing commercially available DNA, encoding the fusion peptides or proteins discussed above, with the DNA encoding the polypeptide of the present invention and expressing the fused DNA prepared.

An alternative method known in the art to isolate functionally equivalent polypeptides is, for example, the method using a hybridization technique (Sambrook et al., Molecular Cloning 2nd ed. 9.47-9.58, Cold Spring Harbor Lab. Press (1989)). One skilled in the art can readily isolate a DNA having high homology with a whole or part of the DNA sequence encoding the human B1194, A2282V1, A2282V2, A2282V3 protein (*i.e.,* SEQ ID NO: 1, 3, 5, or 7), and isolate functionally equivalent polypeptides to the human B1194, A2282V1, A2282V2, or A2282V3 protein from the isolated DNA. The disclosed polypeptides include those that are encoded by DNA that hybridize with a whole or part of the DNA sequence encoding the human B 1194, A2282V1, A2282V2, or A2282V3 protein and are functionally equivalent to the human B1194, A2282V1, A2282V2, or A2282V3 protein. These polypeptides include mammal homologues corresponding to the protein derived from human (for example, a polypeptide encoded by a monkey, rat, rabbit and bovine gene). For example, in isolating a cDNA highly homologous to a DNA encoding the human B 1194 protein from animals, it is particularly preferable to use tissues from testis or breast cancer cell line. alternatively, in isolating a cDNA highly homologous to a DNA encoding the human A2282V1, A2282V2, or A2282V3 protein from animals, it is particularly preferable to use tissues from breast cancer cell line.

The condition of hybridization for isolating a DNA encoding a polypeptide functionally equivalent to the human B1194, A2282V1, A2282V2, or A2282V3 protein can be routinely selected by a person skilled in the art. For example, hybridization may be performed by conducting pre-hybridization at 68°C for 30 min or longer using "Rapid-hyb buffer" (Amersham LIFE SCIENCE), adding a labeled probe, and warming at 68°C for 1 hour or longer. The following washing step can be conducted, for example, in a low stringent condition. A low stringency condition is, for example, 42°C; 2X SSC, 0.1% SDS, or preferably 50°C, 2X SSC, 0.1% SDS. More preferably, high stringency conditions are used. An example of a high stringency condition includes washing 3 times in 2X SSC, 0.0.1% SDS at room temperature for 20 min, then washing 3 times in 1x SSC, 0.1 % SDS at 37°C for 20 min, and washing twice in 1x SSC, 0.1 % SDS at 50°C for 20 min. However, several factors, such as temperature and salt concentration, can influence the stringency of hybridization and one skilled in the art can suitably select the factors to achieve the requisite stringency.

In place of hybridization, a gene amplification method, for example, the polymerase chain reaction (PCR) method, can be utilized to isolate a DNA encoding a polypeptide functionally equivalent to the human B1194, A2282V1, A2282V2, or A2282V3 protein, using a primer synthesized based on the sequence information of the protein encoding DNA (SEQ ID NO: 1, 3, 5, or 7).

Polypeptides that are functionally equivalent to the human B 1194, A2282V1, A2282V2, or A2282V3 protein encoded by the DNA isolated through the above hybridization techniques or gene amplification techniques, normally have a high homology to the amino acid sequence of the human B1194, A2282V1, A2282V2, or A2282V3 protein. "High homology" typically refers to a homology of 40% or higher, preferably 60% or higher, more preferably 80% or higher, even more preferably 95% or higher. The homology of a polypeptide can be determined by following the algorithm in "Wilbur and Lipman, Proc Natl Acad Sci USA 80: 726-30 (1983)".

A polypeptide as disclosed may have variations in amino acid sequence, molecular weight, isoelectric point, the presence or absence of sugar chains, or form, depending, on the cell or host used to produce it or the purification method utilized. Nevertheless, so long as it has a function equivalent to that of the disclosed human B1194, A2282V1, A2282V2, A2282V3 protein, it is within the scope of the present disclosure.

The disclosed polypeptides can be prepared as recombinant proteins or natural proteins, by methods well known to those skilled in the art. A recombinant protein can be prepared by inserting a DNA, which encodes a polypeptide of the present invention (for example, a DNA comprising the nucleotide sequence of SEQ ID NO: 1, 3, 5, or 7), into an appropriate expression vector, introducing the vector into an appropriate host cell, obtaining the extract, and purifying the polypeptide by subjecting the extract to chromatography, for example, ion exchange chromatography, reverse phase chromatography, gel filtration, or affinity chromatography utilizing a column to which antibodies against the disclosed is fixed, or by combining more than one of aforementioned columns.

Also when the disclosed polypeptide is expressed within host cells (for example, animal cells and *E. coli)* as a fusion protein with glutathione-S-transferase protein or as a recombinant protein supplemented with multiple histidines, the expressed recombinant protein can be purified using a glutathione column or nickel column. Alternatively, when the disclosed polypeptide is expressed as a protein tagged with c-myc, multiple histidines, or FLAG, it can be detected and purified using antibodies to c-myc, His, or FLAG, respectively.

After purifying the fusion protein, it is also possible to exclude regions other than the objective polypeptide by cutting with thrombin or factor-Xa as required. A natural protein can be isolated by methods known to a person skilled in the art, for example, by contacting the affinity column, in which antibodies binding to the B 1194, A2282V1, A2282V2, A2282V3 protein described below are bound, with the extract of tissues or cells expressing the disclosed polypeptide. The antibodies can be polyclonal antibodies or monoclonal antibodies.

The present disclosure also encompasses partial peptides of the polypeptide of the present invention. The partial peptide has an amino acid sequence specific to the polypeptide of the present invention and consists of at least 7 amino acids, preferably 8 amino acids or more, and more preferably 9 amino acids or more. The partial peptide can be used, for example, for preparing antibodies against the polypeptide of the present invention, screening for a compound that binds to the disclosed polypeptide , and screening for accelerators or inhibitors of the disclosed polypeptide.

A partial peptide of the disclosure can be produced by genetic engineering, by known methods of peptide synthesis, or by digesting the disclosed polypeptide with an appropriate peptidase. For peptide synthesis, for example, solid phase synthesis or liquid phase synthesis may be used.

Furthermore, the present disclosure provides polynucleotides encoding a polypeptide of the present invention. The disclosed polynucleotides can be used for the *in vivo* or *in vitro* production of a discloded polypeptide as described above. Any form of the disclosed polynucleotide can be used, so long as it encodes a disclosed polypeptide, including mRNA, RNA, cDNA, genomic DNA, chemically synthesized polynucleotides. The disclosed polynucleotides include a DNA comprising a given nucleotide sequences as well as its degenerate sequences, so long as the resulting DNA encodes a disclosed polypeptide.

The polynucleotides can be prepared by methods known to a person skilled in the art. For example, the polynucleotide can be from a cDNA library from cells which express a polypeptide of the present invention, by conducting hybridization using a partial sequence of the DNA of the present invention (for example, SEQ ID NO: 1, 3, 5, or 7) as a probe. A cDNA library can be prepared, for example, by the method described in Sambrook et al., Molecular Cloning, Cold Spring Harbor Laboratory Press (1989); alternatively, commercially available cDNA libraries may be used. A cDNA library can be also prepared by extracting RNAs from cells expressing the disclosed polypeptide synthesizing oligo DNAs based on the sequence of a disclosed DNA (for example, SEQ ID NO: 1, 3, 5, or 7), conducting PCR using the oligo DNAs as primers, and amplifying cDNAs encoding the disclosed protein.

In addition, by sequencing the nucleotides of the obtained cDNA, the translation region encoded by the cDNA can be routinely determined, and the amino acid sequence of the disclosed polypeptide can be easily obtained. Moreover, by screening the genomic DNA library using the obtained cDNA or parts thereof as a probe, the genomic DNA can be isolated.

More specifically, mRNAs may first be prepared from a cell, tissue, or organ (*e.g.,* testis or breast cancer cell line for *B1194;* and breast cancer cell line for *A2282V1, A2282V2,* or *A2282V3*) in which an object polypeptide is expressed. Known methods can be used to isolate mRNAs; for instance, total RNA may be prepared by guanidine ultracentrifugation (Chirgwin et al., Biochemistry 18:5294-9 (1979)) or AGPC method (Chomczynski and Sacchi, Anal Biochem 162:156-9 (1987)). In addition, mRNA may be purified from total RNA using mRNA Purification Kit (Pharmacia) and such or, alternatively, mRNA may be directly purified by QuickPrep mRNA Purification Kit (Pharmacia).

The obtained mRNA is used to synthesize cDNA using reverse transcriptase. cDNA may be synthesized using a commercially available kit, such as the AMV Reverse Transcriptase First-strand cDNA Synthesis Kit (Seikagaku Kogyo). Alternatively, cDNA may be synthesized and amplified following the 5'-RACE method (Frohman et al., Proc Natl Acad Sci USA 85: 8998-9002 (1988); Belyavsky et al., Nucleic Acids Res 17: 2919-32 (1989)), which uses a primer and such, described herein, the 5'-Ampli FINDER RACE Kit (Clontech), and polymerase chain reaction (PCR).

A desired DNA fragment is prepared from the PCR products and ligated with a vector DNA. The recombinant vectors are used to transform *E. coli* and such, and a desired recombinant vector is prepared from a selected colony. The nucleotide sequence of the desired DNA can be verified by conventional methods, such as dideoxynucleotide chain termination.

The nucleotide sequence of a disclosed polynucleotide may be designed to be expressed more efficiently by taking into account the frequency of codon usage in the host to be used for expression (Grantham et al., Nucleic Acids Res 9: 43-74 (1981)). In addition, the sequence of the disclosed polynucleotide may be altered by a commercially available kit or a conventional method. For instance, the sequence may be altered by digestion with restriction enzymes, insertion of a synthetic oligonucleotide or an appropriate polynucleotide fragment, addition of a linker, or insertion of the initiation codon (ATG) and/or the stop codon (TAA, TGA, or TAG).

In a particularly preferred embodiment, the polynucleotide of the present disclosure encompasses DNA comprising the nucleotide sequence of SEQ ID NO: 1, 3, 5, or 7.

Furthermore, the present disclosure provides a polynucleotide that hybridizes under stringent conditions with a polynucleotide having a nucleotide sequence of SEQ ID NO: 1, 3, 5, or 7, and encodes a polypeptide functionally equivalent to the B 1194, A2282V1, A2282V2, or A2282V3 protein described above. As discussed above, one skilled in the art may appropriately choose stringent conditions. For example, low stringency conditions can be used. More preferably, high stringency conditions are used. These conditions are as described above. The hybridizing DNA above is preferably a cDNA or a chromosomal DNA.

The present disclosure also provides a vector into which a disclosed polynucleotide is inserted. A vector of the present disclosure is useful to keep a polynucleotide, especially a DNA, of the present disclosure in host cell; to express the disclosed polypeptide.

When *E. coli* is selected as the host cell and the vector is amplified and produced in a large amount in *E*. *coli* (*e.g.,* JM109, DH5α, HB101, or XL1Blue), the vector should have "ori" to be amplified in *E*. *coli* and a marker gene for selecting transformed *E. coli* (*e.g*., a drug-resistance gene selected by a drug such as ampicillin, tetracycline, kanamycin, chloramphenicol or the like). For example, M13-series vectors, pUC-series vectors, pBR322, pBluescript, pCR-Script, etc. can be used. In addition, pGEM-T, pDIRECT, and pT7 can also be used for subcloning and extracting cDNA as well as the vectors described above. When a vector is used to produce a protein of the present invention, an expression vector is especially useful. For example, an expression vector to be expressed in *E*. *coli* should have the above characteristics to be amplified in *E*. *coli. When E. coli,* such as JM109, DH5α, HB 101, or XL1Blue, are used as a host cell, the vector should have a promoter, for example, lacZ promoter (Ward et al., Nature 341: 544-6 (1989); FASEB J 6: 2422-7 (1992)), araB promoter (Better et al., Science 240: 1041-3 z, or T7 promoter or the like, that can efficiently express the desired gene in E. *colic* In that respect, pGEX-SX-1 (Pharmacia), "QIAexpress system" (Qiagen), pEGFP and pET (in this case, the host is preferably BL21 which expresses T7 RNA polymerase), for example, can be used instead of the above vectors. Additionally, the vector may also contain a signal sequence for polypeptide secretion. An exemplary signal sequence that directs the polypeptide to be secreted to the periplasm of the *E*. *coli is* the pelB signal sequence (Lei et al., J Bacteriol 169: 4319-83 (1987)). Means for introducing of the vectors into the target host cells include, for example, the calcium chloride method, and the electroporation method.

In addition to *E*. *coli,* for example, expression vectors derived from mammalian cells (for example, pcDNA3 (Invitrogen) and pEGF-BOS (Mizushima S., Nucleic Acids Res 18(17): 5322 (1990)), pEF, pCDM8), expression vectors derived from insect cells (for example, "Bac-to-BAC baculovirus expression system" (GIBCO BRL), pBacPAK8), expression vectors derived from plants (*e.g.,* pMH1, pMH2), expression vectors derived from animal viruses (*e.g*., pHSV, pMV, pAdexLcw), expression vectors derived from retroviruses (*e.g*., pZIpneo), expression vector derived from yeast (*e.g.,* "Pichia Expression Kit" (Invitrogen), pNV11, SP-Q01), and expression vectors derived from *Bacillus subtilis* (*e.g.*, pPL608, pKTH50) can be used for producing the polypeptide of the present invention.

In order to express a vector in animal cells, such as CHO, COS, or NIH3T3 cells, the vector should have a promoter necessary for expression in such cells, for example, the SV40 promoter (Mulligan et al., Nature 277: 108-14 (1979)), the MMLV-LTR promoter, the EF1α promoter (Mizushima et al., Nucleic Acids Res 18: 5322 (1990)), the CMV promoter, and the like, and preferably a marker gene for selecting transformants (for example, a drug resistance gene selected by a drug (*e.g*., neomycin, G418)). Examples of known vectors with these characteristics include, for example, pMAM, pDR2, pBK-RSV, pBK-CMV, pOPRSV, and pOP13.

In addition, methods may be used to express a gene stably and, at the same time, to amplify the copy number of the gene in cells. For example, a vector comprising the complementary DHFR gene (*e.g*., pCHO I) may be introduced into CHO cells in which the nucleic acid synthesizing pathway is deleted, and then amplified by methotrexate (MTX). Furthermore, in case of transient expression of a gene, the method wherein a vector comprising a replication origin of SV40 (pcD, *etc.*) is transformed into COS cells comprising the SV40 T antigen expressing gene on the chromosome can be used.

A polypeptide of the present disclosure obtained as above may be isolated from inside or outside (such as medium) of host cells, and purified as a substantially pure homogeneous polypeptide. The term "substantially pure" as used herein in reference to a given polypeptide means that the polypeptide is substantially free from other biological macromolecules. The substantially pure polypeptide is at least 75% (*e.g*., at least 80, 85, 95, or 99%) pure by dry weight. Purity can be measured by any appropriate standard method, for example by column chromatography, polyacrylamide gel electrophoresis, or HPLC analysis. The method for polypeptide isolation and purification is not limited to any specific method; in fact, any standard method may be used.

For instance, column chromatography, filter, ultrafiltration, salt precipitation, solvent precipitation, solvent extraction, distillation, immunoprecipitation, SDS-polyacrylamide gel electrophoresis, isoelectric point electrophoresis, dialysis, and recrystallization may be appropriately selected and combined to isolate and purify the polypeptide.

Examples of chromatography include, for example, affinity chromatography, ion-exchange chromatography, hydrophobic chromatography, gel filtration, reverse phase chromatography, adsorption chromatography, and such (Strategies for Protein Purification and Characterization: A Laboratory Course Manual. Ed. Daniel R. Marshak et al., Cold Spring Harbor Laboratory Press (1996)). These chromatographies may, be performed by liquid chromatography, such as HPLC and FPLC. Thus, the present disclosure provides for highly purified polypeptides prepared by the above methods.

A polypeptide of the present disclosure may be optionally modified or partially deleted by treating it with an appropriate protein modification enzyme before or after purification. Useful protein modification enzymes include, but are not limited to, trypsin, chymotrypsin, lysylendopeptidase, protein kinase, glucosidase, and the like.

### IV. Antibodies

The present disclosure also provides antibodies that bind to a disclosed polypeptide. Such An antibody can be used in any form, such as monoclonal or polyclonal antibodies, and includes antiserum obtained by immunizing an animal such as a rabbit with the disclosed polypeptide, all classes of polyclonal and monoclonal antibodies, human antibodies, and humanized antibodies produced by genetic recombination.

A polypeptide of the present disclosure used as an antigen to obtain an antibody may be derived from any animal species, but preferably is derived from a mammal such as a human, mouse, or rat, more preferably from a human. A human-derived polypeptide may be obtained from the nucleotide or amino acid sequences disclosed herein. According to the present disclosure the polypeptide to be used as an immunization antigen may be a complete protein or a partial peptide of the protein. A partial peptide may comprise, for example, the amino (N)-terminal or carboxy (C)-terminal fragment of a polypeptide of the present invention.

A gene encoding a polypeptide of the disclosure or its fragment may be inserted into a known expression vector, which is then used to transform a host cell as described herein. The desired polypeptide or its fragment may be recovered from the outside or inside of host cells by any standard method, and may subsequently be used as an antigen. Alternatively, whole cells expressing the polypeptide or their lysates, or a chemically synthesized polypeptide may be used as the antigen.

Any mammalian animal may be immunized with the antigen, but preferably the compatibility with parental cells used for cell fusion is taken into account. In general, animals of the orders Rodentia, Lagomorpha, or Primates are used. Animals of the order Rodentia include, for example, mouse, rat, and hamster. Animals of the order Lagomorpha include, for example, rabbit. Animals of the Primate order include, for example, a monkey of Catarrhini (old world monkey) such as *Macaca fascicularis,* rhesus monkey, sacred baboon, and chimpanzees.

Methods for immunizing animals with antigens are known in the art. For example, intraperitoneal injection or subcutaneous injection of antigens is a standard method for immunization of mammals. More specifically, antigens may be diluted and suspended in an appropriate amount of phosphate buffered saline (PBS), physiological saline, etc. If desired, the antigen suspension may be mixed with an appropriate amount of a standard adjuvant, such as Freund's complete adjuvant, made into emulsion, and then administered to mammalian animals. Preferably, it is followed by several administrations of antigen mixed with and appropriately amount of Freund's incomplete adjuvant every 4 to 21 days. An appropriate carrier may also be used for immunization. After immunization as above, serum is examined by a standard method for an increase in the amount of desired antibodies.

Polyclonal antibodies against the polypeptides of the present disclosure may be prepared by collecting blood from the immunized mammal examined for the increase of desired antibodies in the serum, and by separating serum from the blood by any conventional method. Polyclonal antibodies include serum containing the polyclonal antibodies, as well as fractions containing the polyclonal antibodies isolated from the serum. Immunoglobulin G or M can be prepared from a fraction which recognizes only the polypeptide of the present invention using, for example, an affinity column coupled with the polypeptide of the present disclosure, and further purifying this fraction using protein A or protein G column.

To prepare monoclonal antibodies, immune cells are collected from the mammal immunized with the antigen and checked for the increased level of desired antibodies in the serum as described above, and are subjected to cell fusion. The immune cells used for cell fusion are preferably obtained from spleen. Other preferred parental cells to be fused with the above immunocyte include, for example, myeloma cells of mammalians, and more preferably myeloma cells having an acquired property for the selection of fused cells by drugs.

The above immunocyte and myeloma cells can be fused according to known methods, for example, the method of Milstein *et al.* (Galfre and Milstein, Methods Enzymol 73: 3-46 (1981)).

Resulting hybridomas obtained by the cell fusion may be selected by cultivating them in a standard selection medium, such as HAT medium (hypoxanthine, aminopterin, and thymidine containing medium). The cell culture is typically continued in the HAT medium for several days to several weeks, the time being sufficient to allow all the other cells, with the exception of the desired hybridoma (non-fused cells), to die. Then, the standard limiting dilution is performed to screen and clone a hybridoma cell producing the desired antibody.

In addition to the above method, in which a non-human animal is immunized with an antigen for preparing hybridoma, human lymphocytes such as those infected by EB virus may be immunized with a polypeptide, polypeptide expressing cells, or their lysates *in vitro.* Then, the immunized lymphocytes are fused with human-derived myeloma cells that are capable of indefinitely dividing, such as U266, to yield a hybridoma producing a desired human antibody that is able to bind to the polypeptide can be obtained (Unexamined Published Japanese Patent Application No. (JP-A) Sho 63-17688).

The obtained hybridomas are subsequently transplanted into the abdominal cavity of a mouse and the ascites are extracted. The obtained monoclonal antibodies can be purified by, for example, ammonium sulfate precipitation, a protein A or protein G column, DEAE ion exchange chromatography, or an affinity column to which the polypeptide of the present disclosure is coupled. The antibody of the present disclosure can be used not only for purification and detection of the polypeptide of the present disclosure, but also as a candidate for agonists and antagonists of the polypeptide of the present disclosure. In addition, this antibody can be applied to the antibody treatment for diseases related to the polypeptide of the present disclosure. When the obtained antibody is to be administered to the human body (antibody treatment), a human antibody or a humanized antibody is preferable for reducing immunogenicity.

For example, transgenic animals having a repertory of human antibody genes may be immunized with an antigen selected from a polypeptide, polypeptide expressing cells, or their lysates. Antibody producing cells are then collected from the animals and fused with myeloma cells to obtain hybridoma, from which human antibodies against the polypeptide can be prepared (*see* WO92-03918, WO94-02602, WO94-25585, WO96-33735, and WO96-34096).

Alternatively, an immune cell, such as an immunized lymphocyte, producing antibodies may be immortalized by an oncogene and used for preparing monoclonal antibodies.

Monoclonal antibodies thus obtained can be also recombinantly prepared using genetic engineering techniques (*see,* for example; Borrebaeck and Larrick, Therapeutic Monoclonal Antibodies, published in the United Kingdom by MacMillan Publishers LTD (1990)). For example, a DNA encoding an antibody may be cloned from an immune cell, such as a hybridoma or an immunized lymphocyte producing the antibody, inserted into an appropriate vector, and introduced into host cells to prepare a recombinant antibody. The present invention also provides recombinant antibodies prepared as described above.

Furthermore, an antibody of the present disclosure may be a fragment of an antibody or modified antibody, so long as it binds to one or more of the disclosed polypeptides. For instance, the antibody fragment may be Fab, F(ab')₂, Fv, or single chain Fv (scFv), in which Fv fragments from H and L chains are ligated by an appropriate linker (Huston et al., Proc Natl Acad Sci USA 85: 5879-83 (1988)). More specifically, an antibody fragment may be generated by treating an antibody with an enzyme, such as papain or pepsin. Alternatively, a gene encoding the antibody fragment may be constructed, inserted into an expression vector, and expressed in an appropriate host cell (*see*, for example, Co et al., J Immunol 152: 2968-76 (1994); Better and & Horwitz, Methods Enzymol 178: 476-96 (1989); Pluckthun and Skerra, Methods Enzymol 178: 497-515 (1989); Lamoyi, Methods Enzymol 121: 652-63 (1986); Rousseaux et al., Methods Enzymol 121: 663-9 (1986); Bird and Walker, Trends Biotechnol 9: 132-7 (1991)).

An antibody may be modified by conjugation with a variety of molecules, such as polyethylene glycol (PEG). The present disclosure provides for such modified antibodies. The modified antibody can be obtained by chemically modifying an antibody. These modification methods are conventional in the field.

Alternatively, an antibody of the present disclosure may be obtained as a chimeric antibody, between a variable region derived from nonhuman antibody and the constant region derived from human antibody, or as a humanized antibody, comprising the complementarity determining region (CDR) derived from nonhuman antibody, the frame work region (FR) and the constant region derived from human antibody. Such antibodies can be prepared by using known technology.

Antibodies obtained as above may be purified to homogeneity. For example; the separation and purification of the antibody can be performed according to separation and purification methods used for general proteins. For example, the antibody may be separated and isolated by the appropriately selected and combined use of column chromatographies, such as affinity chromatography, filter, ultrafiltration, salting-out, dialyses, SDS polyacrylamide gel electrophoresis, isoelectric focusing, and others (Antibodies: A Laboratory Manual. Ed Harlow and David Lane, Cold Spring Harbor Laboratory (1988)), but are not limited thereto. A protein A column and protein G column can be used as the affinity column. Exemplary protein A columns to be used include, for example, Hyper D, POROS, and Sepharose F.F. (Pharmacia).

Examples of chromatography, with the exception of affinity include ion-exchange chromatography, hydrophobic chromatography, gel filtration, reverse-phase chromatography, adsorption chromatography, and the like (Strategies for Protein Purification and Characterization: A Laboratory Course Manual. Ed Daniel R. Marshak et al., Cold Spring Harbor Laboratory Press(1996)). The chromatographic procedures can be carried out by liquid-phase chromatography, such as HPLC, and FPLC.

For example, absorbance assays, enzyme-linked immunosorbent assays (ELISA), enzyme immunoassays (EIA), radioimmunoassays (RIA), and/or immunofluorescence assays may be used to measure the antigen binding activity of the antibody of the disclosure. In ELlSA, an antibody of the present disclosure is immobilized on a plate, a polypeptide of the disclosure is applied to the plate, and then a sample containing a desired antibody, such as culture supernatant of antibody producing cells or purified antibodies, is applied. Then, a secondary antibody that recognizes the primary antibody and is labeled with an enzyme, such as alkaline phosphatase, is applied, and the plate is incubated. Next, after washing, an enzyme substrate, such as p-nitrophenyl phosphate, is added to the plate, and the absorbance is measured to evaluate the antigen binding activity of the sample. A fragment of the polypeptide, such as a C-terminal or N-terminal fragment, may be used as the antigen to evaluate the binding activity of the antibody. BIAcore (Pharmacia) may be used to evaluate the activity of the antibody according to the present disclosure.

The above methods allow for the detection or measurement of the disclosed polypeptide , by exposing the disclosed antibody to a sample assumed to contain the disclosed polypeptide, and detecting or measuring the immune complex formed by the antibody and the polypeptide.

Because the method of detection or measurement of the polypeptide according to the disclosure can specifically detect or measure a polypeptide, the method may be useful in a variety of experiments in which the polypeptide is used.

### V. Antisense oligonucleotides

As noted above, the present disclosure also provides a polynucleotide which hybridizes with a polynucleotide encoding human B1194, A2282V1, A2282V2, or A2282V3 protein (SEQ ID NO: 1, 3, 5, or 7) or the complementary strand thereof, and which comprises at least 15 nucleotides. The polynucleotide of the present disclosure preferably a polynucleotide which specifically hybridizes with the DNA encoding the polypeptide of the present invention. The term "specifically hybridize" as used herein, means that cross-hybridization does not occur significantly with DNA encoding other proteins, under the usual hybridizing conditions, preferably under stringent hybridizing conditions. Such polynucleotides include, probes, primers, nucleotides and nucleotide derivatives (for example, antisense oligonucleotides and ribozymes), which specifically hybridize with DNA encoding the polypeptide of the invention or its complementary strand. Moreover, such polynucleotide can be utilized for the preparation of DNA chip.

Accordingly, the present disclosure includes an antisense oligonucleotide that hybridizes with any site within the nucleotide sequence of SEQ ID NO: 1, 3, 5, or 7. Such an antisense oligonucleotide is preferably directed against at least 15 continuous nucleotides of the nucleotide sequence of SEQ ID NO: 1, 3, 5, or 7. The above-mentioned antisense oligonucleotide, which contains an initiation codon in the above-mentioned at least 15 continuous nucleotides, is even more preferred.

Derivatives or modified products of antisense oligonucleotides can be used as antisense oligonucleotides of the present disclosure. Examples of such modified products include lower alkyl phosphonate modifications, such as methyl-phosphonate-type or ethyl-phosphonate-type, phosphorothioate modifications and phosphoroamidate modifications.

The antisense oligonucleotide derivatives of the present disclosure act upon cells producing the disclosed polypeptide by binding to the DNA or mRNA encoding the polypeptide, inhibiting its transcription or translation, promoting the degradation of the mRNA, and inhibiting the expression of the disclosed polypeptide, thereby resulting in the inhibition of the polypeptide's function.

An antisense oligonucleotide derivative of the present disclosure can be made into an external preparation, such as a liniment or a poultice, by mixing with a suitable base material which is inactive against the derivatives.

Also, as needed, the derivatives can be formulated into tablets, powders, granules, capsules, liposome capsules, injections, solutions nose-drops and freeze-drying agents by adding excipients, isotonic agents, solubilizers, stabilizers, preservatives, pain-killers, and such. These can be prepared by following usual methods.

The antisense oligonucleotide derivative may be given to the patient by directly applying onto the ailing site or by injecting into a blood vessel so that it will reach the site of ailment. An antisense-mounting medium can also be used to increase durability and membrane-permeability. Examples include, but are not limited to, liposome, poly-L-lysine, lipid, cholesterol, lipofectin or derivatives of these.

The dosage of the antisense oligonucleotide derivative of the present disclosure can be adjusted suitably according to the patient's condition and used in desired amounts. For example, a dose range of 0.1 to 100 mg/kg, preferably 0.1 to 50 mg/kg can be administered.

The term "siRNA" refers to a double stranded RNA molecule which prevents translation of a target mRNA. Standard techniques are used for introducing siRNA into cells, including those wherein DNA is used as the template to transcribe RNA. An siRNA of the present disclosure comprises a sense nucleic acid sequence and an anti-sense nucleic acid sequence of a polynucleotide encoding human B 1194, A2282V1, A2282V2, or A2282V3 protein (SEQ ID NO: 1, 3, 5, or 7). The siRNA is constructed such that a single transcript (double stranded RNA) has both the sense and complementary antisense sequences from the target gene, *e.g.*, a hairpin.

Binding of the siRNA to a transcript corresponding to B1194, A2282V1, A2282V2, or A2282V3 in the target cell results in a reduction in the protein production by the cell. The length of the oligonucleotide is at least 10 nucleotides and may be as long as the naturally-occurring the transcript. Preferably, the oligonucleotide is less than 75, 50, 25 nucleotides in length. Most preferably, the oligonucleotide is 19-25 nucleotides in length. Examples of B1194, A2282V1, A2282V2, and A2282V3 siRNAs oligonucleotides which inhibit the growth of the cancer cell include the target sequence containing SEQ ID NO:38-41.

Furthermore, in order to enhance the inhibition activity of the siRNA, nucleotide "u" can be added to 3'end of the antisense strand of the target sequence. The number of "u"s to be added is at least 2, generally 2 to 10, preferably 2 to 5. The added "u"s form single strand at the 3'end of the antisense strand of the siRNA.

B 1194, A2282V1, A2282V2, and A2282V3 siRNA may be directly introduced into the cells in a form that is capable of binding to the mRNA transcripts. Alternatively, the DNA encoding the B1194, A2282V1, A2282V2, or A2282V3 siRNA may be contained in vector.

Vectors are produced, for example, by cloning a B 1194, A2282V1, A2282V2, or A2282V3 target sequence into an expression vector operatively-linked regulatory sequences flanking the B1194, A2282V1, A2282V2, or A2282V3 sequence in a manner that allows for expression (by transcription of the DNA molecule) of both strands (Lee, N.S. et al., (2002) Nature Biotechnology 20:500-505.). An RNA molecule that is antisense to a B 1194, A2282V1, A2282V2, or A2282V3 mRNA is transcribed by a first promoter (*e.g.*, a promoter sequence 3' of the cloned DNA) and an RNA molecule that is the sense strand for a B1194, A2282V1, A2282V2, or A2282V3 mRNA is transcribed by a second promoter (*e.g*., a promoter sequence 5' of the cloned DNA). The sense and antisense strands hybridize *in vivo* to generate siRNA constructs for silencing of the B 1194, A2282V1, A2282V2, or A2282V3 gene. Alternatively, two constructs may be utilized to create the sense and anti-sense strands of the siRNA construct. Cloned B 1194, A2282V1, A2282V2, or A2282V3 can encode a construct having secondary structure, *e.g*., hairpins, wherein a single transcript has both the sense and complementary antisense sequences from the target gene.

Furthermore, a loop sequence consisting of an arbitrary nucleotide sequence can be located between the sense and antisense sequence in order to form the hairpin loop structure. Thus, the present disclosure also provides siRNA having the general formula 5'-[A]-[B]-[A']-3', wherein [A] is a ribonucleotide sequence corresponding to a sequence of nucleotides SEQ ID NO:38-41., [B] is a ribonucleotide sequence consisting of 3 to 23 nucleotides, and [A'] is a ribonucleotide sequence consisting of the complementary sequence of [A]. The loop sequence may consist of an arbitrary sequence preferably 3 to 23 nucleotide in length. The loop sequence, for example, can be selected from group consisting of following sequences (http://www.ambion.com/techlib/tb/tb_506.html). In the siRNA of the present disclosure the nucleotide "u" can be added to the 3'end of [A'], in order to enhance the inhibiting activity of the siRNA. The number of "u"s to be added is at least 2, generally 2 to 10, preferably 2 to 5. Furthermore, loop sequence consisting of 23 nucleotides also provides active siRNA (Jacque, J.-M. et al., Nature 418: 435-438 (2002).):
- CCC, CCACC or CCACACC: Jacque, J. M. et al., Nature, Vol. 418: 435-438 (2002);
- UUCG: Lee, N.S. et al., Nature Biotechnology 20 : 500-505.; Fruscoloni, P. et al., Proc. Natl. Cad. Sci. USA 100(4): 1639-1644 (2003); and
- UUCAAGAGA: Dykxhoorn, D. M. et al., Nature Reviews Molecular Cell Biology 4: 457-467 (2003).

Examples of preferred siRNAs having hairpin structure of the present invention are shown below. In the following structure, the loop sequence can be selected from group consisting of CCC, UUCG, CCACC, CCACACC, and UUCAAGAGA. A preferred loop sequence is UUCAAGAGA ("ttcaagaga" in DNA).
guauaucuugcccucugaa-[B]-uucagagggcaagauauac (for target sequence of SEQ ID NO: 38)
guccgaacacaucuuuguu-[B]-aacaaagauguguucggac (for target sequence of SEQ ID NO: 39)
gacauccuaucuagcugca-[B]-ugcgcuagauaggauguc (for target sequence of SEQ ID NO: 40)
aguucauuggaacuaccaa-[B]-uugguaguuccaaugaacu (for target sequence of SEQ ID NO: 41)

The regulatory sequences flanking the B1194, A2282V 1, A2282V2, or A2282V3 sequence are identical or different, such that their expression can be modulated independently, or in a temporal or spatial manner. siRNAs are transcribed intracellularly by cloning the B1194, A2282V1, A2282V2, or A2282V3 gene templates into a vector containing, *e.g*., a RNA polymerase III transcription unit from the small nuclear RNA (snRNA) U6 or the human H1 RNA promoter. For introducing the vector into the cell, transfection-enhancing agent can be used. FuGENE (Roche Diagnostics), Lipofectamine 2000 (Invitrogen), Oligofectamine (Invitrogen), and Nucleofector (Wako pure Chemical) are useful as the transfection-enhancing agent.

The nucleotide sequence of siRNAs may be designed using an siRNA design computer program available from the Ambion website (http://www.ambion.com/techlib/misc/siRNA_finder.html). Nucleotide sequences for the siRNA are selected by the computer program based on the following protocol:

Selection of siRNA Target Sites:
1. Beginning with the AUG start codon of the object transcript, scan downstream for AA dinucleotide sequences. Record the occurrence of each AA and the 3' adjacent 19 nucleotides as potential siRNA target sites. Tuschl, et al., Genes Dev 13(24):3191-7(1999), not to recommend against designing siRNA to the 5' and 3' untranslated regions (UTRs) and regions near the start codon (within 75 bases) as these may be richer in regulatory protein binding sites. UTR-binding proteins and/or translation initiation complexes may interfere with the binding of the siRNA endonuclease complex.
2. Compare the potential target sites to the human genome database and eliminate from consideration any target sequences with significant homology to other coding sequences. The homology search can be performed using BLAST(Altschul SF, et al., J Mol Biol. 1990;215:403-10.; Altschul SF, et al. ; Nucleic Acids Res. 1997;25:3389-402.), which can be found on the NCBI server at: www.ncbi.nlm.nih.gov/BLAST/.
3. Select qualifying target sequences for synthesis. At Ambion, preferably several target sequences can be selected along the length of the gene for evaluation.

Oligonucleotides and oligonucleotides complementary to various portions of B1194, A2282V1, A2282V2, and A2282V3 mRNA were tested *in vitro* for their ability to decrease production of B1194, A2282V1, A2282V2, or A2282V3 in tumor cells (*e.g.,* using the T47D or MCF7 breast cancer cell line) according to standard, methods. A reduction in B1194, A2282V1, A2282V2, or A2282V3 gene product in cells contacted with the candidate siRNA composition as compared to cells cultured in the absence of the candidate composition can be detected using B 1194; A2282V1, A2282V2, or A2282V3-specific antibodies or other detection strategies. Sequences which decrease the production of B1194, A2282V1, A2282V2, or A2282V3 in *in vitro* cell-based or cell-free assays may then be tested for there inhibitory effects on cell growth. Sequences which inhibit cell growth in *in vitro* cell-based assay are test in *in vivo* in rats or mice to confirm decreased B1194, A2282V1, A2282V2, or A2282V3 production and decreased tumor cell growth in animals with malignant neoplasms.

Also included in the disclosure are double-stranded molecules that include the nucleic acid sequence of target sequences, for example, nucleotides SEQ ID NO: 38-41. In the present invention, the double-stranded molecule comprising a sense strand and an antisense strand, wherein the sense strand comprises a ribonucleotide sequence corresponding to SEQ ID NO: 38-41, and wherein the antisense strand comprises a ribonucleotide sequence which is complementary to said sense strand, wherein said sense strand and said antisense strand hybridize to each other to form said double-stranded molecule, and wherein said double-stranded molecule, when introduced into a cell expressing the B1194, A2282V1, A2282V2, or A2282V3 gene, inhibits expression of said gene. In the present invention, when the isolated nucleic acid is RNA or derivatives thereof, base "t" should be replaced with "u" in the nucleotide sequences. As used herein, the term "complementary" refers to Watson-Crick or Hoogsteen base pairing between nucleotides units of a nucleic acid molecule, and the term "binding" means the physical or chemical interaction between two polypeptides or compounds or associated polypeptides or compounds or combinations thereof.

Complementary nucleic acid sequences hybridize under appropriate conditions to form stable duplexes containing few or no mismatches. Furthermore, the sense strand and antisense strand of the isolated nucleotide of the present invention, can form double stranded nucleotide or hairpin loop structure by the hybridization. In a preferred embodiment, such duplexes contain no more than 1 mismatch for every 10 matches. In an especially preferred embodiment, where the strands of the duplex are fully complementary, such duplexes contain no mismatches. For example, the nucleic acid molecule is less than 500, 200, or 75 nucleotides in length. Also included in the invention is a vector containing one or more of the nucleic acids described herein, and a cell containing the vectors. The isolated nucleic acids of the present disclosure are useful for siRNA against B1194, A2282V1, A2282V2, or A2282V3 or DNA encoding the siRNA. When the nucleic acids are used for siRNA or coding DNA thereof, the sense strand is preferably longer than 19 nucleotides, and more preferably longer than 21 nucleotides. The present invention specifically relates to the si RNAs characterized in the claims.

### VI. Diagnosing Breast Cancer

An antisense oligonucleotide or siRNA of the present disclosure inhibit the expression of a polypeptide of the disclosure and is thereby useful for suppressing the biological activity of the polypeptide of the disclosure. Also expression-inhibitors, comprising the antisense oligonucleotide or siRNA of the disclosure, are useful in the point that they can inhibit the biological activity of the polypeptide of the disclosure. Therefore, a composition comprising the antisense oligonucleotide or siRNA of the present invention is useful in the treatment of breast cancer. Moreover, the present disclosure provides a method for diagnosing breast cancer using the expression level of the polypeptides of the present disclosure as a diagnostic marker.

The diagnostic method of the present disclosure preferably comprises the steps of: (a) detecting the expression level of the *B1194, A2282V1, A2282Y2, or A2282V3* gene of the present disclosure, and (b) relating an elevation in the expression level to the breast cancer.

The expression level of the *B1194, A2282V1, A2282V2,* or *A2282V3* gene in a particular specimen can be estimated by quantifying mRNA corresponding to or protein encoded by the *B1194, A2282V1, A2282V2, or A2282V3* gene. Quantification methods for mRNA are known to those skilled in the art. For example, the levels of mRNAs corresponding to the *B1194, A2282V1, A2282V2, or A2282V3* gene can be estimated by Northern blotting or RT-PCR. Since the full-length nucleotide sequences of the *B1194, A2282V1, A2282Y2, and A2282V3* genes are shown in SEQ ID NO: 1, 3, 5, or 7, anyone skilled in the art can design the nucleotide sequences for probes or primers to quantify the *B1194, A2282V1, A2282V2,* or *A2282V3* gene.

Also the expression level of the *B1194, A2282V1, A2282V2,* or *A2282V3* gene can be analyzed based on the activity or quantity of protein encoded by the gene. A method for determining the quantity of the B 1194, A2282V1, A2282V2, or A2282V3 protein is shown in below. For example, an immunoassay method is useful for determining proteins in biological materials. Any biological materials can be used for the determination of the protein or its activity. For example, a blood sample may be analyzed for estimation of the protein encoded by a serum marker. On the other hand, a suitable method can be selected for the determination of the activity of a protein encoded by the *B1194, A2282V1, A2282V2, or A2282V3* gene according to the activity of each protein to be analyzed.

In accordance with the methods of the present disclosure, expression levels of the *B1194, A2282V1, A2282V2,* or *A2282V3* gene in a specimen (test sample) are estimated and compared with those in a normal sample. When such a comparison shows that the expression level of the target gene is higher than that of the normal sample, the subject is judged to be affected with breast cancer. The expression level of the *B1194, A2282V1, A2282V2,* or *A2282V3* gene in the specimens from the normal sample and subject may be determined at the same time. Alternatively, normal ranges of the expression levels can be determined by a statistical method based on the results obtained from analyzing specimens previously collected from a control group. A result obtained from a subject sample is compared with the normal range; when the result does not fall within the normal range, the subject is judged to be affected with the breast cancer.

In the present disclosure a diagnostic agent for diagnosing breast cancer, is also provided. The diagnostic agent comprises a compound that binds to a polynucleotide or a polypeptide of the present disclosure. Preferably, an oligonucleotide that hybridizes to the disclosed polynucleotide, or an antibody that binds to the disclosed polypeptide may be used as such a compound.

### VII. Monitoring Breast Cancer Treatment

The expression levels of the *B1194, A2282V1, A2282Y2,* and *A2282V3* genes also allow for the course of treatment of breast cancer to be monitored. In this method, a test cell population is provided from a subject undergoing treatment for breast cancer. If desired, test cell populations are obtained from the subject at various time points, before, during, and/or after treatment. Expression of one or more of the *B1194, A2282V1, A2282V2, and A2282V3* genes in the cell population is then determined and compared to a reference cell population, which includes cells whose breast cancer state is known. In the context of the present disclosure the reference cells should have not been exposed to the treatment of interest.

If the reference cell population contains no breast cancer cells, a similarity in the expression one or more of the *B1194, A2282V1, A2282V2,* and *A2282V3* genes in the test cell population and the reference cell population indicates that the treatment of interest is efficacious. However, a difference in the expression of these genes in the test population and a normal control reference cell population indicates a less favorable clinical outcome or prognosis. Similarly, if the reference cell population contains breast cancer cells, a difference between the expression of one or more of the genes of the present disclosure in the test cell population and the reference cell population indicates that the treatment of interest is efficacious, while a similarity in the expression of such genes in the test population and a reference cell population indicates a less favorable clinical outcome or prognosis.

Additionally, the expression level of the genes of the present disclosure determined in a subject-derived biological sample obtained after treatment (*i.e*., post-treatment levels) can be compared to the expression level of the one or more of the *B1194, A2282V1, A2282V2,* and *A2282V3* genes determined in a subject-derived biological sample obtained prior to treatment onset (*i.e*., pre-treatment levels). A decrease in the expression level in a post-treatment sample indicates that the treatment of interest is efficacious while an increase or maintenance in the expression level in the post-treatment sample indicates a less favorable clinical outcome or prognosis.

As used herein, the term "efficacious" indicates that the treatment leads to a reduction in the expression of a pathologically up-regulated gene, an increase in the expression of a pathologically down-regulated gene or a decrease in size, prevalence, or metastatic potential of breast ductal carcinoma in a subject. When a treatment of interest is applied prophylactically, the term "efficacious" means that the treatment retards or prevents a breast tumor from forming or retards, prevents, or alleviates a symptom of clinical breast cancer. Assessment of breast tumors can be made using standard clinical protocols.

In addition, efficaciousness can be determined in association with any known method for diagnosing or treating breast cancer. Breast cancer can be diagnosed, for example, by identifying symptomatic anomalies, *e.g.*, weight loss, abdominal pain, back pain, anorexia, nausea, vomiting and generalized malaise, weakness, and jaundice.

### VIII. Treating Breast Cancer

The present invention further provides a method of screening for a compound useful in the treatment of breast cancer using a polypeptide of the present invention. An embodiment of such a screening method comprises the steps of: (a) contacting a test compound with a polypeptide of the present invention, (b) detecting the binding activity between the polypeptide of the present invention and the test compound, and (c) selecting the test compound that binds to the polypeptide of the present invention and inhibits its biological activity of enhancement of cell poliferation and kinase activity.

A polypeptide of the present invention to be used for screening may be a recombinant polypeptide or a protein derived from the nature, or a partial peptide thereof. Any test compound, for example, cell extracts, cell culture supernatant, products of fermenting microorganism, extracts from marine organism, plant extracts, purified or crude proteins, peptides, non-peptide compounds, synthetic micromolecular compounds and natural compounds, can be used. The polypeptide of the present invention to be contacted with a test compound can be, for example, a purified polypeptide, a soluble protein, a form bound to a carrier, or a fusion protein fused with other polypeptides.

As a method of screening for proteins, for example, that bind to a polypeptide of the present invention using a polypeptide of the present invention, many methods well known by a person skilled in the art can be used. Such a screening can be conducted using, for example, an immunoprecipitation method, specifically, in the following manner. A gene encoding a polypeptide of the present invention is expressed in animal cells by inserting the gene to an expression vector for foreign genes, such as pSV2neo, pcDNA I, and pCD8. The promoter to be used for the expression may be any promoter that is commonly used and includes, for example, the SV40 early promoter (Rigby in Williamson (ed.), Genetic Engineering, vol. 3. Academic Press, London, 83-141 (1982)), the EF-1α promoter (Kim et al., Gene 91: 217-23 (1990)), the CAG promoter (Niwa et al., Gene 108: 193-9 (1991)), the RSV LTR promoter (Cullen, Methods in Enzymology 152: 684-704 (1987)) the SRα promoter (Takebe et al., Mol Cell Biol 8: 466-72 (1988)), the CMV immediate early promoter (Seed and Aruffo, Proc Natl Acad Sci USA 84: 3365-9 (1987)), the SV40 late promoter (Gheysen and Fiers, J Mol Appl Genet 1: 385-94 (1982)), the Adenovirus late promoter (Kaufman et al., Mol Cell Biol 9: 946-58 (1989)), the HSV TK promoter, and so on. The introduction of the gene into animal cells to express a foreign gene can be performed according to any methods, for example, the electroporation method (Chu et al., Nucleic Acids Res 15: 1311-26 (1987)), the calcium phosphate method (Chen and Okayama, Mol Cell Biol 7: 2745-52 (1987), the DEAE dextran method (Lopata et al., Nucleic Acids Res 12: 5707-17 (1984); Sussman and Milman, Mol Cell Biol 4: 1641-3 (1984)), the Lipofectin method (Derijard B, Cell 76: 1025-37 (1994); Lamb et al., Nature Genetics 5: 22-30 (1993): Rabindran et al., Science 259: 230-4 (1993)), and so on. The polypeptide of the present invention can be expressed as a fusion protein comprising a recognition site (epitope) of a monoclonal antibody by introducing the epitope of the monoclonal antibody, whose specificity has been revealed, to the N- or C- terminus of the polypeptide of the present invention. A commercially available epitope-antibody system can be used (Experimental Medicine 13: 85-90 (1995)). Vectors which can express a fusion protein with, for example, β-galactosidase, maltose binding protein, glutathione S-transferase, green florescence protein (GFP) and so on by the use of its multiple cloning sites are commercially available.

A fusion protein prepared by introducing only small epitopes consisting of several to a dozen amino acids so as not to change the property of the polypeptide of the present invention by the fusion is also reported. Epitopes, such as polyhistidine (His-tag), influenza aggregate HA, human c-myc, FLAG, Vesicular stomatitis virus glycoprotein (VSV-GP), T7 gene 10 protein (T7-tag), human simple herpes virus glycoprotein (HSV-tag), E-tag (an epitope on monoclonal phage), and such, and monoclonal antibodies recognizing them can be used as the epitope-antibody system for screening proteins binding to the polypeptide of the present invention (Experimental Medicine 13: 85-90 (1995)).

In immunoprecipitation, an immune complex is formed by adding these antibodies to cell lysate prepared using an appropriate detergent. The immune complex consists of a polypeptide of the present invention, a polypeptide having a binding affinity for the polypeptide, and an antibody. Immunoprecipitation can be also conducted using antibodies against a polypeptide of the present invention, in addition to using antibodies against the above epitopes, which antibodies can be prepared as described above.

An immune complex can be precipitated, for example, by Protein A sepharose or Protein G sepharose when the antibody is a mouse IgG antibody. If the polypeptide of the present invention is prepared as a fusion protein with an epitope, such as GST, an immune complex can be formed in the same manner as in the use of the antibody against the polypeptide of the present invention, using a substance specifically binding to these epitopes, such as glutathione-Sepharose 4B.

Immunoprecipitation can be performed by following or according to, for example, the methods in the literature (Harlow and Lane, Antibodies, 511-52, Cold Spring Harbor Laboratory publications, New York (1988)).

SDS-PAGE is commonly used for analysis of immunoprecipitated proteins and the bound protein can be analyzed by the molecular weight of the protein using gels with an appropriate concentration. Since the protein bound to the polypeptide of the present invention is difficult to detect by a common staining method, such as Coomassie staining or silver staining, the detection sensitivity for the protein can be improved by culturing cells in culture medium containing radioactive isotope, ³⁵S-methionine or ³⁵S-cystein, labeling proteins in the cells, and detecting the proteins. The target protein can be purified directly from the SDS-polyacrylamide gel and its sequence can be determined, when the molecular weight of a protein has been revealed

As a method for screening proteins binding to the polypeptide using the polypeptide, for example, West-Western blotting analysis (Skolnik et al., Cell 65: 83-90 (1991)) can be used. Specifically, a protein binding to the polypeptide can be obtained by preparing a cDNA library from cells, tissues, organs (for example, breast cancer cell lines for screening proteins binding to A2282V1, A2282V2, A2282V3), or cultured cells, expected to express a protein binding to the polypeptide of the present invention using a phage vector (*e.g.*, ZAP), expressing the protein on LB-agarose, fixing the protein expressed on a filter, reacting the purified and labeled polypeptide of the present invention with the above filter, and detecting the plaques expressing proteins bound to the polypeptide of the present invention according to the label. The polypeptide may be labeled by utilizing the binding between biotin and avidin, or by utilizing an antibody that specifically binds to the polypeptide, or a peptide or polypeptide (for example, GST) that is fused to the polypeptide. Methods using radioisotope or fluorescence and such may be also used.

Alternatively, in another embodiment of the screening method of the present invention, a two-hybrid system utilizing cells may be used ("MATCHMAKER Two-Hybrid system", "Mammalian MATCHMAKER Two-Hybrid Assay Kit", "MATCHMAKER one-Hybrid system" (Clontech); "HybriZAP Two-Hybrid Vector System" (Stratagene); the references "Dalton and Treisman, Cell 68: 597-612 (1992)", "Fields and Sternglanz, Trends Genet 10: 286-92 (1994)").

In the two-hybrid system, the polypeptide is fused to the SRF-binding region or GAL4-binding region and expressed in yeast cells. A cDNA library is prepared from cells expected to express a protein binding to the polypeptide , such that the library, when expressed, is fused to the VP16 or GAL4 transcriptional activation region. The cDNA library is then introduced into the above yeast cells and the cDNA derived from the library is isolated from the positive clones detected (when a protein binding to the polypeptide is expressed in yeast cells, the binding of the two activates a reporter gene, making positive clones detectable). A protein encoded by the cDNA can be prepared by introducing the cDNA isolated above to *E. coli* and expressing the protein.

As a reporter gene, for example, Ade2 gene, lacZ gene, CAT gene, luciferase gene and such can be used in addition to the HIS3 gene.

A compound binding to a polypeptide can also be screened using affinity chromatography. For example, the polypeptide may be immobilized on a carrier of an affinity column; and a test compound, containing a protein capable of binding to the polypeptide, is applied to the column. A test compound herein may be, for example, cell extracts, cell lysates, etc. After loading the test compound, the column is washed, and compounds bound to the polypeptide can be prepared.

When the test compound is a protein, the amino acid sequence of the obtained protein is analyzed, an oligo DNA is synthesized based on the sequence, and DNA libraries are screened using the oligo DNA as a probe to obtain a DNA encoding the protein.

A biosensor using the surface plasmon resonance phenomenon may be used as a mean for detecting or quantifying the bound compound in the present invention. When such a biosensor is used, the interaction between the polypeptide and a test compound can be observed real-time as a surface plasmon resonance signal, using only a minute amount of polypeptide and without labeling (for example, BIAcore, Pharmacia). Therefore, it is possible to evaluate the binding between the polypeptide and a test compound using a biosensor such as BIAcore.

The methods of screening for molecules that bind when the immobilized polypeptide is exposed to synthetic chemical compounds, or natural substance banks, or a random phage peptide display library, and the methods of screening using high-throughput based on combinatorial chemistry techniques (Wrighton et al., Science 273: 458-63 (1996); Verdine, Nature 384: 11-13 (1996)) to isolate not only proteins but chemical compounds that bind to the protein (including agonist and antagonist) are well known to one skilled in the art.

Alternatively, the screening method of the present invention may comprise the following steps:
(a) contacting a candidate compound with a cell into which a vector comprising the transcriptional regulatory region of one or more marker genes and a reporter gene that is expressed under the control of the transcriptional regulatory region has been introduced, wherein the one or more marker genes are selected from the group consisting of A2282V1, A2282V2, and A2282V3
(b) measuring the expression or activity of said reporter gene; and
(c) selecting a compound that reduces the expression or activity level of said reporter gene as compared to the expression or activity level of said reporter gene detected in the absence of the test compound.

Suitable reporter genes and host cells are well known in the art. The reporter construct required for the screening can be prepared by using the transcriptional regulatory region of a marker gene. When the transcriptional regulatory region of a marker gene has been known to those skilled in the art, a reporter construct can be prepared by using the previous sequence information. When the transcriptional regulatory region of a marker gene remains unidentified, a nucleotide segment containing the transcriptional regulatory region can be isolated from a genome library based on the nucleotide sequence information of the marker gene.

A compound isolated by the screening is a candidate for drugs which inhibit the activity of a polypeptide, which, in turn, may be used to treat or prevent breast cancer. A compound in which a part of the structure of the compound obtained by the present screening method having the activity of binding to a polypeptide is converted by addition, deletion and/or replacement, is included in the compounds obtained by the screening method of the present invention.

In a further embodiment, the present disclosure provides methods for screening candidate agents which are potential targets in the treatment of breast cancer. As discussed in detail above, by controlling the expression level of the B1194, A2282V1, A2282V2, or A2282V3 protein, one can control the onset and progression of breast cancer. Thus, candidate agents, which are potential targets in the treatment of breast cancer, can be identified through screenings that use the expression levels and activities of A2282V1, A2282V2, A2282V3 as indices. In the context of the present invention, such screening may comprise, for example, the following steps:
(a) contacting a candidate compound with a cell expressing the A2282V1, A2282V2, or A2282V3 protein and
(b) selecting a compound that reduces the expression level of A2282V1, A2282V2, or A2282V3 in comparison with the expression level detected in the absence of the test compound.

Cells expressing at least one of the A2282V1, A2282V2, or A2282V3 protein include, for example, cell lines established from breast cancer; such cells can be used for the above screening of the present invention. Expression level can be estimated by methods well known to one skilled in the art. In the method of screening, a compound that reduces the expression level of at least one of A2282V1, A2282V2, or A2282V3 can be selected as candidate agents.

In another embodiment of the method for screening a compound useful in the treatment of breast cancer of the present invention, the method utilizes the biological activity of a polypeptide as an index; i.e. the enhancement of cell proliferation and kinase activity. Since the A2282V1, A2282V2, and A2282V3 proteins have the activity of promoting cell proliferation, a compound which inhibits the activity of one of these proteins can be screened using this activity as an index. Furthermore, in the present invention, it was confirmed that the A2282V1, A2282V2, and A2282V3 proteins have protein kinase activity. Thus, a compound that inhibits the protein kinase activity of one of A2282V1, A2282V2, or A2282V3 proteins can be screened using such activity as an index. This screening method includes the steps of: (a) contacting a test compound with the polypeptide (b) detecting the biological activity of the polypeptide of step (a), i.e. the enhancement of cell proliferation and kinase activity; and (c) selecting a compound that suppresses said biological activity of the polypeptide in comparison with the biological activity detected in the absence of the test compound.

Any polypeptides can be used for screening so long as they comprise the biological activity of the A2282V1, A2282V2, or A2282V3 protein. Such biological activity include cell-proliferating activity of the human A2282V1, A2282V2, A2282V3 protein, or protein kinase activity of the A2282V1, A2282V2, or A2282V3 protein. For example, a Human A2282V1, A2282V2, A2282V3 protein can be used and polypeptides functionally equivalent to these proteins can also be used. Such polypeptides may be expressed endogenously or exogenously by cells.

In the present invention, the biological activity of the A2282V1, A2282V2, or A2282V3 protein is preferably protein kinase activity. The skilled artisan can estimate protein kinase activity. For example, a cell expressing at least one of A2282V1, A2282V2, or A2282V3 proteins can be contacted with a test compound in the presence of [γ-³²P]-ATP. Next, proteins phosphorylated through the protein kinase activity of the A2282V1, A2282V2, or A2282V3 protein can be determined. For detection of phosphorylated protein, SDS-PAGE or immunoprecipitation can be used. Furthermore, an antibody recognizes phosphorylated tyrosine residue can be used for phosphorylated protein level.

Any test compounds, for example, cell extracts, cell culture supernatant, products of fermenting microorganism, extracts of marine organism, plant extracts, purified or crude proteins, peptides, non-peptide compounds, synthetic microinolecular compounds, natural compounds, can be used.

The compound isolated by this screening is a candidate for antagonists of the polypeptide. Likewise, the term "antagonist" refers to molecules that inhibit the function of the polypeptide by binding thereto. Moreover, a compound isolated by this screening is a candidate for compounds which inhibit the *in vivo* interaction of the polypeptide with molecules (including DNAs and proteins).

When the biological activity to be detected in the present method is cell proliferation, it can be detected, for example, by preparing cells which express the polypeptide, culturing the cells in the presence of a test compound, and determining the speed of cell proliferation, measuring the cell cycle and such, as well as by measuring the colony forming activity as described in the Examples.

### IX. Isolated Compounds and Pharmaceutical Compositions

A compound isolated by the above screenings is a candidate for drugs which inhibit the activity of the polypeptide and can be applied to the treatment of breast cancer. More particularly, when the biological activity of the B1194, A2282V1, A2282V2, or A2282V3 protein is used as the index, compounds screened by the present method serve as a candidate for drugs for the treatment of breast cancer.

Moreover, compounds in which a part of the structure of the compound inhibiting the activity of the B1194, A2282V1, A2282V2, or A2282V3 protein is converted by addition, deletion and/or replacement are also included in the compounds obtainable by the screening method of the present invention.

When administrating a compound isolated by the methods of the invention as a pharmaceutical for humans and other mammals, such as mice, rats, guinea-pigs, rabbits, cats, dogs, sheep, pigs, cattle, monkeys, baboons, chimpanzees, for treating breast cancer, the isolated compound can be directly administered or can be formulated into a dosage form using known pharmaceutical preparation methods. For example, according to the need, the drugs can be taken orally, as sugarcoated tablets, capsules, elixirs and microcapsules, or non-orally, in the form of injections of sterile solutions or suspensions with water or any other pharmaceutically acceptable liquid. For example, the compounds can be mixed with pharmacologically acceptable carriers or medium, specifically, sterilized water, physiological saline, plant-oil, emulsifiers, suspending agents, surfactants, stabilizers, flavoring agents, excipients, vehicles, preservatives, binders and such, in a unit dose form required for generally accepted drug implementation. The amount of active ingredients in these preparations makes a suitable dosage within the indicated range acquirable.

Examples of additives that can be mixed to tablets and capsules are, binders such as gelatin, corn starch, tragacanth gum and arabic gum; excipients such as crystalline cellulose; swelling agents such as corn starch, gelatin and alginic acid; lubricants such as magnesium stearate; sweeteners such as sucrose, lactose or saccharin; flavoring agents such as peppermint, Gaultheria adenothrix oil and cherry. When the unit dosage form is a capsule, a liquid carrier, such as oil, can also be further included in the above ingredients. Sterile composites for injections can be formulated following normal drug implementations using vehicles such as distilled water used for injections.

Physiological saline, glucose, and other isotonic liquids including adjuvants, such as D-sorbitol, D-mannose, D-mannitol, and sodium chloride, can be used as aqueous solutions for injections. These can be used in conjunction with suitable solubilizers, such as alcohol, specifically ethanol, polyalcohols such as propylene glycol and polyethylene glycol, non-ionic surfactants, such as Polysorbate 80 (TM) and HCO-50.

Sesame oil or Soy-bean oil can be used as a oleaginous liquid and may be used in conjunction with benzyl benzoate or benzyl alcohol as a solubilizers and may be formulated with a buffer, such as phosphate buffer and sodium acetate buffer; a pain-killer, such as procaine hydrochloride; a stabilizer, such as benzyl alcohol, phenol; and an anti-oxidant. The prepared injection may be filled into a suitable ampule.

Methods well known to one skilled in the art may be used to administer the inventive pharmaceutical compound to patients, for example as intra-arterial, intravenous, percutaneous injections and also as intranasal, transbronchial, intramuscular or oral administrations. The dosage and method of administration vary according to the body-weight and age of a patient and the administration method; however, one skilled in the art can routinely select them. If said compound is encodable by a DNA, the DNA can be inserted into a vector for gene therapy and the vector administered to perform the therapy. The dosage and method of administration vary according to the body-weight, age, and symptoms of a patient but one skilled in the art can select them suitably.

For example, although there are some differences according to the symptoms, the dose of a compound that binds with the polypeptide of the present invention and regulates its activity is about 0.1 mg to about 100 mg per day, preferably about 1.0 mg to about 50 mg per day and more preferably about 1.0 mg to about 20 mg per day, when administered orally to a normal adult (weight 60 kg).

When administering parenterally, in the form of an injection to a normal adult (weight 60 kg), although there are some differences according to the patient, target organ, symptoms and method of administration, it is convenient to intravenously inject a dose of about 0.01 mg to about 30 mg per day, preferably about 0.1 to about 20 mg per day and more preferably about 0.1 to about 10 mg per day. Also, in the case of other animals too, it is possible to administer an amount converted to 60kgs of body-weight.

### X. Methods of Inducing Anti-Tumor Immunity and Tumor Vaccines

The present disclosure also relates to a method of inducing anti-tumor immunity comprising the step of administering a B 1194, A2282V1, A2282V2, or A2282V3 protein or an immunologically active fragment thereof, or a polynucleotide encoding the protein or fragments thereof. The B 1194, A2282V1, A2282V2, and A2282V3 proteins or the immunologically active fragments thereof are useful as vaccines against breast cancer. In some cases the proteins or fragments thereof may be administered in a form bound to the T cell receptor (TCR) or presented by an antigen presenting cell (APC), such as macrophage, dendritic cell (DC), or B-cells. Due to the strong antigen presenting ability of DC, the use of DC is most preferable among the APCs.

In the present disclosure a vaccine against breast cancer refers to a substance that has the function to induce anti-tumor immunity upon inoculation into animals. In general, anti-tumor immunity includes immune responses such as follows:
- induction of cytotoxic lymphocytes against breast cancer,
- induction of antibodies that recognize breast cancer, and
- induction of anti-tumor cytokine production.

Therefore, when a certain protein induces any one of these immune responses upon inoculation into an animal, the protein is deemed to have anti-tumor immunity inducing effect. The induction of the anti-tumor immunity by a protein can be detected by observing *in vivo* or *in vitro* the response of the immune system in the host against the protein.

For example, a method for detecting the induction of cytotoxic T lymphocytes is well known. A foreign substance that enters the living body is presented to T cells and B cells by the action of antigen presenting cells (APCs). T cells that respond to the antigen presented by APC in antigen specific manner differentiate into cytotoxic T cells (or cytotoxic T lymphocytes; CTLs) due to stimulation by the antigen, and then proliferate (this is referred to as activation of T cells). Therefore, CTL induction by a certain peptide can be evaluated by presenting the peptide to T cell by APC, and detecting the induction of CTL. Furthermore, APC has the effect of activating CD4+ T cells, CD8+ T cells, macrophages, eosinophils, and NK cells. Since CD4+ T cells and CD8+ T cells are also important in anti-tumor immunity, the anti-tumor immunity inducing action of the peptide can be evaluated using the activation effect of these cells as indicators.

A method for evaluating the inducing action of CTL using dendritic cells (DCs) as APC is well known in the art. DC is a representative APC having the strongest CTL inducing action among APCs. In this method, the test polypeptide is initially contacted with DC, and then this DC is contacted with T cells. Detection of T cells having cytotoxic effects against the cells of interest after the contact with DC shows that the test polypeptide has an activity of inducing the cytotoxic T cells. Activity of CTL against tumors can be detected, for example, using the lysis of ⁵¹Cr-labeled tumor cells as the indicator. Alternatively, the method of evaluating the degree of tumor cell damage using ³H-thymidine uptake activity or LDH (lactose dehydrogenase)-release as the indicator is also well known.

Apart from DC, peripheral blood mononuclear cells (PBMCs) may also be used as the APC. The induction of CTL is reported that it can be enhanced by culturing PBMC in the presence of GM-CSF and IL-4. Similarly, CTL has been shown to be induced by culturing PBMC in the presence of keyhole limpet hemocyanin (KLH) and IL-7.

The test polypeptides confirmed to possess CTL inducing activity by these methods are polypeptides having DC activation effect and subsequent CTL inducing activity. Therefore, polypeptides that induce CTL against tumor cells are useful as vaccines against tumors. Furthermore, APC that acquired the ability to induce CTL against tumors by contacting with the polypeptides are useful as vaccines against tumors. Furthermore, CTL that acquired cytotoxicity due to presentation of the polypeptide antigens by APC can be also used as vaccines against tumors. Such therapeutic methods for tumors using anti-tumor immunity due to APC and CTL are referred to as cellular immunotherapy.

Generally, when using a polypeptide for cellular immunotherapy, efficiency of the CTL-induction is known to increase by combining a plurality of polypeptides having different structures and contacting them with DC. Therefore, when stimulating DC with protein fragments, it is advantageous to use a mixture of multiple types of fragments.

Alternatively, the induction of anti-tumor immunity by a polypeptide can be confirmed by observing the induction of antibody production against tumors. For example, when antibodies against a polypeptide are induced in a laboratory animal immunized with the polypeptide, and when growth of tumor cells is suppressed by those antibodies, the polypeptide can be determined to have an ability to induce anti-tumor immunity.

Anti-tumor immunity is induced by administering the disclosed vaccine and the induction of anti-tumor immunity enables treatment and prevention of breast cancer. Therapy against cancer or prevention of the onset of cancer includes any of the steps, such as inhibition of the growth of cancerous cells, involution of cancer, and suppression of occurrence of cancer. Decrease in mortality of individuals having cancer, decrease of tumor markers in the blood, alleviation of detectable symptoms accompanying cancer, and such are also included in the therapy or prevention of cancer. Such therapeutic and preventive effects are preferably statistically significant. For example, in observation, at a significance level of 5% or less, wherein the therapeutic or preventive effect of a vaccine against breast cancer is compared to a control without vaccine administration. For example, Student's t-test, the Mann-Whitney U-test, or ANOVA may be used for statistical analyses.

The above-mentioned protein having immunological activity or a vector encoding the protein may be combined with an adjuvant. An adjuvant refers to a compound that enhances the immune response against the protein when administered together (or successively) with the protein having immunological activity. Examples of adjuvants include cholera toxin, salmonella toxin, alum, and such, but are not limited thereto. Furthermore, the disclosed vaccine may be combined appropriately with a pharmaceutically acceptable carrier. Examples of such carriers are sterilized water, physiological saline, phosphate buffer, culture fluid, and such. Furthermore, the vaccine may contain as necessary, stabilizers, suspensions, preservatives, surfactants, and such. The vaccine is administered systemically or locally. Vaccine administration may be performed by single administration, or boosted by multiple administrations.

When using APC or CTL as the vaccine of this disclosure tumors can be treated or prevented, for example, by the *ex vivo* method. More specifically, PBMCs of the subject receiving treatment or prevention are collected, the cells are contacted with the polypeptide *ex vivo,* and following the induction of APC or CTL, the cells may be administered to the subject. APC can be also induced by introducing a vector encoding the polypeptide into PBMCs *ex vivo.* APC or CTL induced in vitro can be cloned prior to administration. By cloning and growing cells having high activity of damaging target cells, cellular immunotherapy can be performed more effectively. Furthermore, APC and CTL isolated in this manner may be used for cellular immunotherapy not only against individuals from whom the cells are derived, but also against similar types of tumors from other individuals.

Furthermore, a pharmaceutical composition for treating or preventing breast cancer, comprising a pharmaceutically effective amount of the polypeptide of the present disclosure is provided. The pharmaceutical composition may be used for raising anti-tumor immunity. The normal expression of B 1194, restricted to testis; expression of A2282V1, A2282V2, and A2282V3 in normal organ is not observed. Therefore, suppression of these genes may not adversely affect other organs. Thus, the B1194, A2282V1, A2282V2, and A2282V3 polypeptides are preferable for treating breast cancer. In the present disclosure, the polypeptide or fragment thereof is administered at a dosage sufficient to induce anti-tumor immunity, which is in the range of 0.1 mg to 10 mg, preferably 0.3mg to 5mg, more preferably 0.8mg to 1.5 mg. The administrations are repeater. For example, 1mg of the peptide or fragment thereof may be administered 4 times in every two weeks for inducing the anti-tumor immunity.

Hereinafter, the present invention is described in more detail by reference to the Examples.

### EXAMPLES

As can be appreciated from the disclosure provided above, the present invention has a wide variety of applications.

The present invention is illustrated in detail by the following Examples.

### Example 1 - Materials and Methods

### (1) Cell lines and clinical materials

Human-breast cancer cell lines HBL100, HCC1937, MCF7, MDA-MB-435S, YMB1, SKBR3, T47D, cervical adenocarcinoma HeLa and COS-7 cell lines were purchased from American Type Culture Collection (ATCC) and were cultured under their respective depositor's recommendation. HBC4, HBC5 and MDA-MB-231 cells lines are kind gifts from Dr. Yamori of Molecular Pharmacology, Cancer Chemotherapy Center of the Japanese Foundation for Cancer Research.

All cells were cultured in appropriate media; i.e. RPMI-1640 (Sigma, St. Louis, MO) for HBC4, HBC5, SKBR3, T47D, YMB1, and HCC1937 (with 2mM L-glutamine); Dulbecco's modified Eagle's medium (Invitrogen, Carlsbad, CA) for HBL100, COS7; EMEM (Sigma) with 0.1mM essential amino acid (Roche), 1mM sodium pyruvate (Roche), 0.01mg/ml Insulin (Sigma) for MCF-7; L-15 (Roche) for MDA-MB-231 and MDA-MB-435S. Each medium was supplemented with 10% fetal bovine serum (Cansera) and 1% antibiotic/antimycotic solution (Sigma). MDA-MB-231 and MDA-MB-435S cells were maintained at 37°C an atmosphere of humidified air without CO₂. Other cell lines were maintained at 37°C an atmosphere of humidified air with 5% CO₂. Clinical samples (breast cancer and normal breast duct) were obtained from surgical specimens, concerning which all patients had given informed consent.

### (2) Isolation of novel human genes on the cDNA microarray

Fabrication of the cDNA microarray slides has been described elsewhere (Ono K, et al., (2000). Cancer Res, 60, 5007-5011). For each analysis of expression profiles, duplicate sets of slides containing 27,648 cDNA spots were prepared to reduce experimental fluctuation. Briefly, total RNAs were purified from each sample of laser-microdissected cells, and T7-based RNA amplification was carried out to obtain adequate quantities of RNA for microarray experiments. Aliquots of amplified RNA from breast cancer cells and the normal breast ductal cells were labeled by reverse transcription with Cy5-dCTP and Cy3-dCTP, respectively (Amersham Biosciences, Buckinghamshire, UK). Hybridization, washing, and detection were carried out as described previously (Ono K, el al., (2000). Cancer Res, 60, 5007-5011). To detect genes that were commonly up-regulated in breast cancer, the overall expression patterns of the 27,648 genes on the microarray were screened to select those with expression ratios >2.0 that were present in >50% of all of 77 premenopausal breast cancer cases. Eventually, a total of 468 genes that appeared to up-regulated in tumor cells were identified.

To detect genes that were commonly up-regulated in breast cancer, the overall expression patterns of the 27,648 genes on the microarray were screened to select those with expression ratios >3.0 that were present in >50% of i) all of 77 premenopausal breast cancer cases, ii) 69 invasive ductal carcinomas, iii) 31 well-, iv) 14 moderately, or v) 24 poorly-differentiated lesions, respectively. Eventually, the total of 493 genes that appeared to up-regulated in tumor cells were selected.

### (3) Semi-quantitative RT-PCR analysis

Total RNA was extracted from each population of laser-captured cells and then performed T7-based amplification and reverse transcription as described previously (Kitahara O, et al., Cancer Res 61, 3544-3549 (2001)). Appropriate dilutions ofeach single-stranded cDNA were prepared for subsequent PCR amplification by monitoring the glyceraldehyde-3-phosphate dehydrogenase (GAPDH) as a quantitative internal control. The PCR primer sequences were 5'- CGACCACTTTGTCAAGCTCA-3' (SEQ ID NO.9) and 5'-GGTTGAGCACAGGGTACTTTATT-3'(SEQ ID NO.10) for GAPDH; and 5'-TGGGTAACAAGAGAATGGTTCA-3'(SEQ ID NO.11) and 5'- ATCCAAGTCCTAATCCCTTTGG-3'(SEQ ID NO.12) for B1194; and 5'- GCTGCAAGGTATAATTGATGGA-3'(SEQ ID NO.13) and 5'-CAGTAACATAATGACAGATGGGC-3'(SEQ ID NO. 14) for A2282.

### (4) Northern-blot analysis

Total RNAs were extracted from all breast cancer cell lines using RNeasy kit (QIAGEN) according to the manufacturer's instructions. After treatment with DNase I (Nippon Gene, Osaka, Japan), mRNA was isolated with mRNA purification kit (Amersham Biosciences) following the manufacturer's instructions. A 1-µg aliquot of each mRNA, along with polyA(+) RNAs isolated from normal adult human lung, heart, liver, kidney, bone marrow, brain (BD, Clontech, Palo Alto, CA), were separated on 1% denaturing agarose gels and transferred to nylon membranes (Breast cancer-Northern blots). Human multiple-tissue Northern blots (Clontech, Palo Alto, CA) and Breast cancer-Northern blot were hybridized with an [α³²P]-dCTP-labeled PCR products of B1194 and A2282 prepared by RT-PCR (see below). Pre-hybridization, hybridization and washing were performed according to the supplier's recommendations. The blots were autoradiographed with intensifying screens at -80°C for 14 days. Specific probes for B1194 (411bp) was prepared by PCR using a primer set; 5'-TGG GTAACAAGAGAATGGTTCA-3' (SEQ ID NO.11) and 5'-ATCCAAGTCCTAATCCCTTTGG-3' (SEQ ID NO.12); for variant 1, 2, and 3 of A2282 (554bp) and for variant 1 and 2 of A2282 (170bp) were prepared by PCR using the following primer sets; 554bp: 5'-TTATCACTGTGCTCACCAGGAG-3' (SEQ ID NO.15) and 5'-CAGTAACATAATGACAGATGGGC-3' (SEQ ID NO.14); 170bp 5'-AAACTTGCCTGCCATATCCTTA-3' (SEQ ID NO.16), and 5'-ATTTTGTTGGCTGTCTCTAGCA-3' (SEQ ID NO. 17), and is radioactively labeled with megaprime DNA labeling system (Amersham bioscience).

### (5) cDNA library screening

A cDNA library was constructed using and superscript^{™} plasmid system with gateway^{™} technology for cDNA synthesis and cloning kit (Invitrogen) and poly(A)+ RNA obtained from breast cancer cell line T47D, and screened 3X10⁶ independent clones of this library with cDNA probe corresponding to nucleotide 1-1112 of V1 variant.

### (6) In vitro translation assay

The four variants (V1, V2, V3 and V4) of A2282 were each cloned into pSPORT-1 expression vector used constructing a cDNA library as above and then used as templates for transcription/translation experiments *in vitro.* The plasmids (1 µg) were transcribed and translated using TNT Coupled Reticulocyte Lysate Systems (Promega, Madison, WI) in the presence of ε-labeled biotinylated lysine-tRNA according to the manufacturer's instructions. Protein products were separated by electrophoresis on 5-20 % gradient SDS-polyacrylamide gels. After electroblotting, the biotinylated proteins are visualized by binding streptavidin-horseradish peroxidase, follows by chemiluminescent detection (Amersham Biosciences).

### (7) Construction of expression vectors

For constructing of mammalian expression vector, the entire coding sequence of B1194 cDNA was amplified by PCR using KOD-Plus DNA polymerase (Toyobo, Osaka, Japan) with primer sets; 5'-AAAGAATTCGGGTGTCGTTAATGTTCGGGG-3' (SEQ ID NO.18); and 5'-AAAGCGGCCGCTTAGGCGGATTTTCCTGCA-3' (SEQ ID NO.19). The PCR products were inserted into the EcoRI and Not I sites of pCMV-N-myc expression vector (Clontech).

For constructing of V1, V2, and V3 variants of A2282 expression vectors, the entire coding sequence of each variant of A2282 cDNA was amplified by the PCR using KOD-Plus DNA polymerase (Toyobo, Osaka, Japan) with the following primer sets; 5'-CGGAATTCACTATGAAAGATTATGATGAAC-3' (SEQ ID NO.20); and 5'-AAACTCGAGTACCTTGCAGCTAGATAGGAT-3' (SEQ ID NO.21) because all variants contain the same 5' sequence of ORF. The PCR products were inserted into the EcoRI and Xho I sites of pCAGGS-HA expression vector. These constructs, pCMV-myc-B 1194 and pCAGGS-A2282-HA were confirmed by DNA sequencing.

### (8) Immunocytochemical staining

To examine the sub-cellular localization of B1194, COS7 cells transfected with B1194 were seeded at 5x10⁴ cells per well on Lab-Tek^{®} II Chamber Slide System (Nalgen Nunc International), and followed by fixation with 4% paraformaldehyde in PBS and permeabilization with 0.1% Triton X-100 in PBS for 3 min at 4°C. After blocking with 3% BSA in PBS for 1 hour at room temperature, the cells were incubated with mouse anti-myc 9E10 monoclonal antibodies (0.2 µg/ml, Santa Cruz Biotechnology) for I hour at room temperature. Cells are subsequently stained with a FITC-conjugated goat anti-mouse secondary antibody before visualization under TCS SP2 AOBS microscope (Leica, Tokyo, Japan).

### (9) Western Blot Analysis

To examine the expression of exogenous B1194 and A2282 protein, B 1194-expressing plasmid, pCAGGS-A2282-HA or pCMV-N-myc (Mock) as negative control were transiently transfected into COS7 cells or HeLa cells, respectively. Cell lysates were separated on 5%-10% SDS-polyacrylamide gels and transferred to a nitrocellulose membrane, followed by blocking with BlockAce^{™} powder (Dainippon Seiyaku) and treated with mouse anti-myc 9E10 monoclonal antibodies or mouse anti-HA antibodies (0.4 µg/ml, Santa Cruz Biotechnology) or monoclonal β-actin antibody served as a loading control for proteins with 1:1000 dilution (clone AC-15, Sigma-Aldrich, MO). After washing, the blots were treated with horseradish peroxidase-conjugated donkey anti-mouse IgG for β-actin antibody (Amersham Biosciences) and proteins are visualized by binding horseradish peroxidase, follows by chemiluminescent detection (Amersham Biosciences).

### (10) Synchronization and flow cytometry analysis

HeLa cells (1x10⁶) were transfected with 8 µg of pCAGGS-A2282-HA expression vector using FuGENE6 (Roche) according to supplier's protocol. Cells were arrested in G1 phase 24 hours after transfection with aphidicolin 1(µg/ml) for further 16 hours. Cell cycle was released by washing three times with fresh medium and cells are collected at indicated time points. To arrest cells at mitotic phase, cells were incubated with Nocodazole (250 ng/ml) 16 hours before harvest.

For FACS analysis, 400 µl aliquot of synchronized adherent and detached cells were combined and fixed with 70% ethanol at 4°C. After washing with PBS (-) twice, cells were incubated for 30 min with 1 µl of PBS containing 1µg of RNase I at 37°C. Cells were then stained in 1 ml of PBS containing 50 µg ofpropidium iodide (PI). The percentages of each fraction of cell cycle phases were determined from at least 10000 cells in a flow cytometer (FACScalibur; Becton Dickinson, San Diego, CA).

### (11) Construction of B1194 or A2282 specific-siRNA expression vectors

A vector-based RNAi system was established using psiH1BX3.0 siRNA expression vector according to the previous report (Shimokawa T, et al., (2003). Cancer Res, 63, 6116-6120). A siRNA expression vector against B1194 (psiH1BX-B1194 Si-1 and Si-5) and A2282 (psiH1BX-A2282 Si-3 and Si-4) was prepared by cloning of double-stranded oligonucleotides in Table 1 into the BbsI site in the psiH1BX vector. A control plasmid, psiH1BX-SC and psiH1BX-LUC, was prepared by cloning double-stranded oligonucleotides of for LUC (luciferase control) into the BbsI site in the psiH1BX3.0 vector, respectively.

**Table 1 Sequences of specific double-stranded oligonucleotides inserted into siRNA expression vector**

| | | | | SEQ ID No. |
|---|---|---|---|---|
| B1194 | Si-1 | F | | 30 |
| | | R | | 31 |
| | Si-5 | F | | 32 |
| | | R | | 33 |
| A2282 | Si-3 | F | | 34 |
| | | R | | 35 |
| | Si-4 | F | | 36 |
| | | R | | 37 |

The target sequences of each siRNAs are shown in table 2.

**Table 2**

| | | target sequence | SEQ ID No. |
|---|---|---|---|
| B1194 | Si-1 | GTATATCTTGCCCTCTGAA | 38 |
| | Si-5 | GTCCGAACACATCTTTGTT | 39 |
| A2282 | Si-3 | GACATCCTATCTAGCTGCA | 40 |
| | Si-4 | AGTTCATTGGAACTACCAA | 41 |

### (12) Gene-silencing effect of B1194 or A2282

Human breast cancer cells lines, T47D or MCF-7, were plated onto 15-cm dishes (4x10⁶ cells/dish) and transfected with 16µg of each psiH1BX-LUC (luciferase control) psiH1BX-SC (scrambled control) as negative controls, psiH1BX-A2282 and psiH1BX-B 1194 using FuGENE6 reagent according to the supplier's recommendations (Roche). 24 hour after transfection, cells are reseeded again for colony formation assay (3x10⁶ cells/10 cm dish), RT-PCR (1x10⁶ cells / 10 cm dish) and MTT assay (5x10⁵ cells / well). The B1194 or A2282-introducing cells were selected with medium containing 0.7 or 0.6 mg/ml of neomycin (Geneticin, Gibco) in T47D or MCF-7 cells, respectively. Afterward, we changed medium every two days for 3 weeks. To evaluate the functioning of siRNA, total RNA was extracted from the cells at 4 days after Neomycin selection, and then the knockdown effect of siRNAs was confirmed by semi-quantitative RT-PCR using specific primers for B1194 or A2282 and for β*2MG*; 5'-TTAGCTGTGCTCGCGCTACT-3' (SEQ ID NO.26) and 5'-TCACATGGTTCACACGGCAG-3' (SEQ ID NO.27) for β*2MG* as an internal control; 5'- TTAAGTGAAGGCTCTGATTCTAGTT-3' (SEQ ID NO.28) and 5'-GTCCTTATTGGCTGGTTCGTT-3' (SEQ ID NO.29) for B1194; and 5'-TTATCACTGTGCTCACCAGGAG -3(SEQ ID NO.15) and 5'- CAGTAACATAATGACAGATGGGC -3' (SEQ ID NO.14) for A2282.

Moreover, transfectants expressing siRNAs using T47D or MCF-7 cells were grown for 28 days in selective media containing 0.7 mg/ml of neomycin. After fixation with 4% paraformaldehyde, transfected cells were stained with Giemsa solution to assess colony formation. MTT assays were performed to quantify cell viability. After 7 days of culture in the neomycin-containing medium, MTT solution (3-(4,5-dimethylthiazol-2-yl)-2,5-diphenyl tetrazolium bromide) (Sigma) was added at a concentration of 0.5 mg/ml. Following incubation at 37°C for 2.5 hours, acid-SDS (0.01N HCl/10%SDS) was added; the suspension was mixed vigorously and then incubated overnight at 37°C to dissolve the dark blue crystals. Absorbance at 570nm was measured with a Microplate Reader 550 (BioRad). Each experiment is triplicated.

### (13) Construction of truncated A2282 protein using pCAGGS-HA vector

All the constructs were prepared by polymerase chain reaction (PCR) according to following primer sets. (Full-length forward: 5'-CGGAATTCACTATGAAAGATTATGATGAAC -3' (SEQ ID NO; 20); Truncated No.1 forward: 5'-ACGGAATTCATCATGCAAGATTACAACTATCC -3' (SEQ ID NO; 42); truncated No.2 forward: 5'-GACGGAATTCAATATGGAGGAGACTCCAAAAAG-3' (SEQ ID NO; 43) and common reverse primer: 5'- CCCTCGAGTACCTTGCAGCTAGATAGGATG-3' (SEQ ID NO; 44)). The ORFs were then ligated into EcoRI and Xho I restriction enzyme sites of pCAGGS-HA vector in frame with Hemaglutinnin (HA) tag.

### (14) Cell culture for identification of potential phosphorylation sites

HBC5 cells were cultured on 10 cm dish until reached 80 % confluent prior to transfection. 8 µg of each construct was transfected into HBC5 cells according to manufacture's recommendation (FuGENE 6). 36 hours after transfection cells were treated with Aphidicolin 1µg/ml (Sigma A-0781) and Nocodazole 0.5 µg/ml (sigma M-1404) for further 18 hours.

### (15) Lambda Phosphatase assay

Phosphatase assay was carried out as described by manufacture (New England). Briefly, cell lysate was incubated with 1 µl of lambda phosphatase at 30 degree for I hour.

### (16) Generate the mutant A2282 expression vectors for in vitro kinase assay

The mutant constructs, D150A, T167A and T478A were generated by QuickChange Site-Directed Mutagenesis Kit (Stratagene Cat 200518-5) using pCAGGS-A2282-HA as a template according to manufacture recommendation.

### (17) Transfection and cell culture for immunoprecipitation

HEK 293 cells were transfected with 16 µg of each construct. 48 hours after transfection, immunoprecipitation was carried out using lysis buffer (50 mM Tris-HCl (pH 7.5), 150 mM NaCl, 1% NP-40, 40 mM NAF, 40 mM β-glycerophosphate, protease inhibitor cocktail) and anti-HA rat antibody (Roche). The protein bound Rec-protein G sepharose 4 B beads (ZYMED) were washed twice in lysis buffer and once in kinase buffer (50 mM Tris-HCl, (pH 7.5), 10mM MgCl, 25 mM NaCl, 1 mM DTT).

### (18) In vitro kinase assay

20 µl of IP product was incubated with 5 µg of Histone H1 (UPSTATE, Lake Placid, NY 12946) in the presence of 30 µl of kinase buffer containing 50 µM ATP and 10 ci of [γ³²P]-ATP at 30 °C for 30 min. The reaction was stopped by adding 10 µl of SDS sample buffer and boiled for 3 min.

### Example 2- B1194

### (1) Identification of B1194 as an up-regulated gene in breast cancer

Through the selection criteria for candidate gene from the gene-expression profiles of cancer cells from pre-menopausal 77 breast cancer patients using a cDNA microarray representing 27,648 human, 468 genes were identified that were commonly at least 2-fold up-regulated in breast cancer cells, as compared with normal breast duct cells genes (see Materials and methods). From among them, B 1194, which designed FLJ-10252, (Genbank Accession NM_018040), was selected. Expression of B1194 gene was elevated in 24 of 41 informative breast cancer cases on the microarray. Subsequent semi-quantitative RT-PCR confirmed that B1194 was significantly up-regulated in 8 of 12 clinical specimens (well-differentiated type) (Figure 1a) and in almost all 9 breast cancer cell lines (Figure 1b) as compared to normal breast ductal cells and other normal tissues although weak expression of B1194 was observed in mammary gland and heart. To further examine the expression pattern of B1194, Northern blot analysis was performed with multiple-human tissues and breast cancer cell lines using the 411 bp of cDNA fragment as a probe (*see* Material and Method). As a result, B1194 was exclusively expressed in testis (Figure 2a), and was specifically over-expressed in all of breast cancer cell lines (Figure 2b), suggesting B1194 might be good candidate targets for development of anti-cancer drugs. B 1194 consists of 10 exons, designed FLJ-10252 hypothetical protein, located on the chromosome 1q41. The full-length cDNA sequence of B1194 contained 2338 nucleotides, and encodes 528 amino acids (58 kDa). The SMART computer program predicted that this gene product has a highly conserved G-patch, glycine rich nucleic binding domain at its carboxyl ends, suggesting that it was predicted to have an RNA binding function. The open reading frame (ORF) start at exon 1, and ends at exon 10.

### (2) Subcellular localization of B1194

PSORTII computer program predicted B1194 gene product mainly localizes to nucleus. To further examine the characterization of B1194, the sub-cellular localization of this gene product was investigated in mammalian cells. When a plasmid expressing B 1194 protein (pCMV(+)-myc-B1194) was transiently transfected into COS7 cells, immunocytochemical staining revealed exogenous B1194 localized to throughout the nucleus in COS7 cells (Figure 3a) with a molecular weight of 58 kDa (Figure 3b).

### (3) Growth-inhibitory effects of siRNA against B1194

To assess the growth-promoting role of B 1194, the expression of endogenous B1194 was knocked down in the breast cancer cell line T47D, a cell line that has shown the over-expression of B1194 (*see* Figure 1b and 2b), by means of the mammalian vector-based RNA interference (RNAi) technique (*see* Materials and Methods). Expression levels of B1194 were examined by semi-quantitative RT-PCR experiments. As shown in Figure 4a, among the two siRNA constructs of the gene examined, B1194-specific siRNAs (si1 and si5) significantly suppressed expression of B1194, compared with a control siRNA construct (psiH1BX-SC). To confirm the cell growth inhibition with a B1194-specific siRNAs, MTT assays were performed. As a result, introduction of B1194-specific siRNAs (si1 and si5) constructs suppressed growth of T47D cells (Figure 4b), consisting with the result of above reduced expression. Each result was verified by three independent experiments. Hence, the present findings suggest that B1194 has a significant function in the cell growth of the breast cancer.

### Example 3 - A2282

### (1) Identification of A2282 as an up-regulated gene in breast cancer

When gene-expression profiles of cancer cells from pre-menopausal 77 breast cancer patients were analyzed using a cDNA microarray representing 27,648 human genes, 493 genes were identified that were commonly up-regulated in breast cancer cells. From among them, A2282, designed to maternal embryonic leucine kinase, was selected. MELK (Genbank Accession NM_014791) is located at chromosome 9p13.1 with a mRNA transcript 2501 bases in length consisting of 18 exons. Expression of A2282 was elevated in 25 of 33 (76%) breast cancer cases which were able to obtain expression data, especially in 10 of 14 (71%) cases with moderately-differentiated typed breast cancer specimens. Intriguingly, A2282 is mainly expressed in patients whose estrogen and progesterone receptor status are negative. To confirm the expression pattern of this gene in breast cancers, semi-quantitative RT-PCR analysis was performed using breast cancer cell lines and normal human tissues including normal breast cells. As a result, it was discovered that A2282, whose expression showed the elevated expression in 11 of 12 clinical breast cancer specimens (moderately-differentiated type) as compared to normal breast ductal cells and other normal vital tissues (Figure 5a), was over-expressed in all of 6 breast cancer cell lines as well (Figure 5b), although it was observed the expression in bone marrow. To further examine the expression pattern of this gene, Northern blot analysis was performed with multiple-human tissues and breast cancer cell lines using a cDNA fragment (554 bp) located within 3' UTR of A2282 as a probe (Figure 6a). Unexpectedly, it was observed that two apparent transcripts (approximately 1.4kb and 0.5kb) were ubiquitously expressed in all of normal human tissues. Particularly, 0.5 kb transcript showed higher expression in almost of normal tissues than 1.4kb transcript (Figure 6b). In contrast, an approximately 2.4kb transcript found with breast cancer-Northern blot analysis was specifically over-expressed in breast cancer cell lines (Figure 6c).

### (2) Isolation of breast cancer specific-expressed transcript of A2282

To isolate breast cancer specific-expressed variants of A2282, a cDNA library constructed using poly(A)+ RNA obtained from breast cancer cell line T47D was screened. Five different variants were isolated (Figure 7a). Among them, three transcripts were in similar size of approximately 2.4 kb, designated as V1, V2 and V3 according to full length, 133 bases deletion and 250 bases deletion in the transcript. The other two transcripts, V4 and V5, are 1.23 kb and 0.5 kb respectively. To investigate which transcript was over-expressed in breast cancer cells compared to normal breast, northern blot analysis was performed using V1 and V2 specific sequence as a probe (nucleotide 214-383). Not unexpectedly, V1 and V2 transcripts of A2282 gene are specifically over-expressed in all of breast cancer cell lines (Figure 7b). Therefore, A2282 V1, V2 and V3 transcripts were selected. A2282V1, designed to MELK, encodes a 75 kDa protein with a serine threonine kinase catalytic domain at N-terminus and a kinase associated domain (KA1) at near the C-terminus. The human MELK protein is evolutionary conserved between species sharing 65% (Heyer BS, et al., Dev Dyn. 1999;215:344-51) and 29.91% (Gilardi-Hebenstreit, P. et al., (1992) Oncogene 7(12),2499-2506) identity with xenopus and mouse MELK protein, respectively, suggesting that human MELK protein may have similar functions or binding partners in vivo. Xenopus MELK kinase has been reported as the new member of KIN1/PAR-1/MARK family which involves in the establishment of cell polarity and both microtubules dynamic and cytoskeleton organization. Most importantly, it is believed to play an important role in cell cycle regulation during xenopus embryogenesis (Blot J, et al., (2002) Dev Biol241, 327-338). Human MELK protein has also been suggested to participate in cell cycle progression (Davezac N, et al., (2002). Oncogene, 21, 7630-7641). However, the exact molecular pathways and machineries are yet to be elucidated.

### (3) In vitro translation

To further examine whether these five different variants could be potentially translated into functional proteins, *in vitro* translation experiments were performed. It was confirmed that V1, V2 and V3 transcripts were able to be translated *in vitro* with the predicted protein molecular weight of 75, 71 and 66 kDa respectively (Figure 8). However, no band was detected for V4 in breast cancer cell lines. These findings suggest that V1, V2 and V3 transcripts of A2282 are good candidates as molecular targets for the development of novel anti-cancer drugs.

### (4) Expression of V1, V2 and V3 of A2282 protein on any cell cycle phases

Since human MELK was report to be involved in cell cycle regulation and its kinase activity and phosphorylation status are observed reaching maximal level during mitotic phase (Davezac N, et al., (2002). Oncogene, 21, 7630-7641), Western blot and flow cytometry analyses were performed to examine whether V2 and V3 also possess characteristic of V1. Accordingly, an extra slower migrating band of V1 protein was observed during mitotic phase in synchronized HeLa cells (Figure 9a, b); however, no extra band was seen in V2 and V3 proteins, which suggests that there might be no phosphorylations of V2 and V3 proteins in any cell cycle phases.

### (5) Growth-inhibitory effects of siRNA against A2282

To assess the growth-promoting role of A2282, the expression of endogenous A2282 was knocked down in the breast cancer cell lines T47D and MCF-7, each of which have been shown to over-express A2282, by means of the mammalian vector-based RNA interference (RNAi) technique (*see* Materials and Methods) (Figure 10). Expression levels of A2282 were examined by semi-quantitative RT-PCR experiments. As shown in Figure 10a, A2282 (si3 and si4)-specific siRNAs significantly suppressed expression, as compared with control siRNA constructs (psiH1BX-LUC or -SC). To confirm the cell growth inhibition with A2282-specific siRNAs, MTT and colony-formation assays, respectively, were performed (Figure.10b, c). As a result, introduction of A2282-specific siRNA constructs suppressed growth of T47D and MCF-7 cells, consisting with the result of above reduced expression of this gene. Each result was verified by three independent experiments. Thus, the present findings suggest that A2282 has a significant function in the cell growth of the breast cancer.

### (6) A2282 protein phosphorylated at kinase domain

To identify the potential phosphorylation sites, wild type (WT) and two kinase domain deleted proteins of A2282 were examined for phosphorylation (Figure 11a). As shown in Figure 11b, WT protein migrated as a fuzzy band in all cell cycle phases, particularly in the mitotic phase; however, no fuzzy bands in truncated constructs (TC1 and TC2). To investigate whether a fuzzy band of WT protein is phosphorylation, a lambda phosphatase assay was performed (Figure 11c). Treatment of lambda phosphatase reduced the fuzzy band to a single band, suggesting that WT protein was extensively phosphorylated at M phase. By contrast, neither double nor fuzzy band was observed in the other two kinase truncated proteins. These data indicated that phosphorylation site(s) were likely to locate in the kinase region of A2282 protein.

### (7) Regulation of A2282_kinase activity by phosphorylation

The activation of MARK and AMPK related kinases has been reported to be regulated by the phosphorylation of their T-loop Threonine residue (Drewes G and Nurse P, FEBS Lett. 2003;554:45-9; Spicer J, et al., Oncogene. 2003;22:4752-6) . Accordingly, several substituted and deleted mutants (*see* Material and Methods) were constructed. Immunoprecipitation was performed in mammalian cells and kinase activities of these constructs were examined using Histone H1, the substrate used in previous reports. The predicted ATP binding packet (D150 residues) and the potential phosphorylated threonine (T167 residues) were replaced with alanine residue as described in Materials and Methods. A reported phosphorylation site (Vulsteke V, et al., J Biol Chem. 2004;279(10):8642-7) Thr 478 was also mutated as a control (Figure 12a). It was also discovered that all the transcripts were phosphorylated in HEK293 cell line as seen in Western blot (Figure 12b). In respect to kinase activity, wild type and T478A proteins possessing intact kinase domain was observed phosphorylated Histone HI *in vitro* (Figure 12c). This activity was severely compromised in T167A mutant and completely abolished in D150A protein. Furthermore a band located at the 75 kDa was observed in WT, T167A and T478A but not in D150A, indicating the possibility of autophosphorylation of A2282 protein.

### Industrial Applicability

The expression of novel human genes *B1194, A2282V1, A2282V2,* and *A2282V3* is markedly elevated in breast cancer as compared to non-cancerous human tissues. Accordingly, these genes may serve as diagnostic markers of cancer and the proteins encoded thereby may be used in diagnostic assays of cancer.

Herein, the expression of novel proteins B1194, A2282V1, A2282V2, and A2282V3 were shown to promote cell growth whereas cell growth was suppressed by antisense oligonucleotides or small interfering RNAs corresponding to the *B1194, A2282V1, A2282V2,* and *A2282V3* genes. These findings suggest that each of B1194, A2282V1, A2282V2, and A2282V3 proteins stimulate oncogenic activity. Thus, each of these novel oncoproteins is a useful target for the development of anti-cancer pharmaceuticals. For example, agents that block the expression of B1194, A2282V1, A2282V2, or A2282V3 or prevent its activity may find therapeutic utility as anti-cancer agents, particularly anti-cancer agents for the treatment of breast cancer. Examples of such agents include antisense oligonucleotides, small interfering RNAs, and antibodies that recognize B1194, A2282V1, A2282V2, or A2282V3.

## Claims

1. A small interfering RNA, wherein the sense strand thereof is selected from the group consisting of the nucleotide sequences of SEQ ID NO: 40 and 41 as the target sequence.

2. A method of screening for a compound useful in the treatment of breast cancer, said method comprising the steps of:
(a) contacting a test compound with a polypeptide selected from the group consisting of:
(1) a polypeptide comprising the amino acid sequence of SEQ ID NO: 4, 6, or 8;
(2) a polypeptide that comprises the amino acid sequence of SEQ ID NO: 4, 6, or 8 in which one or more amino acids are substituted, deleted, inserted, and/or added and that has 95% identity to SEQ ID NO: 4, 6 or 8, and a biological activity of enhancement of cell proliferation and kinase activity; and
(3) a polypeptide encoded by a polynucleotide that hybridizes under stringent conditions to a polynucleotide consisting of the nucleotide sequence of SEQ ID NO: 3, 5, or 7, wherein the polypeptide has a biological activity of enhancement of cell proliferation and kinase activity
(b) detecting the binding activity between the polypeptide and the test compound; and
(c) selecting the test compound that binds to the polypeptide and inhibits its biological activity of enhancement of cell proliferation and kinase activity.

3. A method of screening for a compound useful in the treatment of breast cancer, said method comprising the steps of:
(a) contacting a test compound with a cell expressing one or more polynucleotides comprising the nucleotide sequence of SEQ ID NO: 3, 5, or 7; and
(b) selecting a compound that reduces the expression level of one or more polynucleotides comprising the nucleotide sequence of SEQ ID NO: 3, 5, or 7 in comparison with the expression level detected in the absence of the test compound.

4. A method of screening for a compound useful in the treatment of breast cancer, said method comprising the steps of:
(a) contacting a test compound with a polypeptide selected from the group consisting of:
(1) a polypeptide comprising the amino acid sequence of SEQ ID NO: 4, 6, or 8;
(2) a polypeptide that comprises the amino acid sequence of SEQ ID NO: 4, 6, or 8 in which one or more amino acids are substituted, deleted, inserted, and/or added and that has 95% identity to SEQ ID NO: 4, 6 or 8, and a biological activity of enhancement of cell proliferation and kinase activity; and
(3) a polypeptide encoded by a polynucleotide that hybridizes under stringent conditions to a polynucleotide consisting of the nucleotide sequence of SEQ ID NO: 3, 5, or 7, wherein the polypeptide has a biological activity of enhancement of cell proliferation and kinase activity;
(b) detecting the biological activity of enhancement of cell proliferation and kinase activity of the polypeptide of step (a); and
(c) selecting a compound that suppresses the biological activity of the polypeptide in comparison with the biological activity detected in the absence of the test compound.

5. A method of screening for a compound useful in the treatment of breast cancer, said method comprising the steps of:
(a) contacting a test compound with a cell into which a vector comprising the transcriptional regulatory region of a marker gene and a reporter gene that is expressed under the control of the transcriptional regulatory region has been introduced, wherein the marker gene comprises any one of nucleotide sequences selected from the group consisting of SEQ ID:NO 3, 5, and 7,
(b) measuring the expression or activity of said reporter gene; and
(c) selecting the compound that reduces the expression or activity level of said reporter gene as compared to the expression or activity level of said reporter gene detected in the absence of the test compound.

6. A pharmaceutical composition comprising a pharmaceutically effective amount of an antisense polynucleotide or small interfering RNA against a polynucleotide consisting of the nucleotide sequence SEQ ID NO: 3, 5, or 7 as an active ingredient, and a pharmaceutically acceptable carrier for use in treating breast cancer.

7. Use of a pharmaceutically effective amount of an antisense polynucleotide or small interfering RNA against a polynucleotide consisting of the nucleotide sequence of SEQ ID NO: 3, 5, or 7 for the preparation of a pharmaceutical composition for treating breast cancer.

## Patentansprüche

1. Kleine interferierende RNA, wobei der Sense-Strang davon ausgewählt ist aus der Gruppe bestehend aus den Nucleotidsequenzen von SEQ ID NO:40 und 41 als der Zielsequenz.

2. Verfahren zum Screenen auf eine Verbindung, die in der Behandlung von Brustkrebs nützlich ist, wobei das Verfahren die folgenden Schritte umfasst:
(a) Inkontaktbringen einer Testverbindung mit einem Polypeptid ausgewählt aus der Gruppe bestehend aus:
(1) einem Polypeptid, das die Aminosäuresequenz von SEQ ID NO:4, 6 oder 8 umfasst;
(2) einem Polypeptid, das die Aminosäuresequenz von SEQ ID NO:4, 6 oder 8 umfasst, in dem eine oder mehrere Aminosäuren substituiert, deletiert, inseriert und/oder hinzugefügt sind und das eine Identität von 95% mit SEQ ID NO:4, 6 oder 8 und eine die Zellproliferation fördernde biologische Aktivität und Kinaseaktivität aufweist; und
(3) einem Polypeptid, das von einem Polynucleotid codiert wird, das unter stringenten Bedingungen an ein Polynucleotid hybridisiert, bestehend aus der Nucleotidsequenz von SEQ ID NO:3, 5 oder 7, wobei das Polypeptid eine die Zellproliferation fördernde biologische Aktivität und Kinaseaktivität aufweist
(b) Nachweisen der Bindungsaktivität zwischen dem Polypeptid und der Testverbindung; und
(c) Auswählen der Testverbindung, die an das Polypeptid bindet und seine die Zellproliferation fördernde biologische Aktivität und Kinaseaktivität inhibiert.

3. Verfahren zum Screenen auf eine Verbindung, die in der Behandlung von Brustkrebs nützlich ist, wobei das Verfahren die folgenden Schritte umfasst:
(a) Inkontaktbringen einer Testverbindung mit einer Zelle, die ein oder mehrere Polynucleotide exprimiert, die die Nucleotidsequenz von SEQ ID NO:3, 5 oder 7 umfassen; und
(b) Auswählen einer Verbindung, die den Expressionsspiegel eines oder mehrerer Polynucleotide, die die Nucleotidsequenz von SEQ ID NO:3, 5 oder 7 umfassen, im Verhältnis zum Expressionsspiegel, der in Abwesenheit der Testverbindung nachgewiesen wird, herabsetzt.

4. Verfahren zum Screenen auf eine Verbindung, die in der Behandlung von Brustkrebs nützlich ist, wobei das Verfahren die folgenden Schritte umfasst:
(a) Inkontaktbringen einer Testverbindung mit einem Polypeptid ausgewählt aus der Gruppe bestehend aus:
(1) einem Polypeptid, das die Aminosäuresequenz von SEQ ID NO:4, 6 oder 8 umfasst;
(2) einem Polypeptid, das die Aminosäuresequenz von SEQ ID NO:4, 6 oder 8 umfasst, in dem eine oder mehrere Aminosäuren substituiert, deletiert, inseriert und/oder hinzugefügt sind und eine Identität von 95% mit SEQ ID NO:4, 6 oder 8 und eine die Zellproliferation fördernde biologische Aktivität und Kinaseaktivität aufweist; und
(3) einem Polypeptid, das von einem Polynucleotid codiert wird, das unter stringenten Bedingungen an ein Polynucleotid hybridisiert, das aus der Nucleotidsequenz von SEQ ID NO:3, 5 oder 7 besteht, wobei das Polypeptid eine die Zellproliferation fördernde biologische Aktivität und Kinaseaktivität aufweist;
(b) Nachweisen der die Zellproliferation fördernde biologische Aktivität und Kinaseaktivität des Polypeptids aus Schritt (a); und
(c) Auswählen einer Verbindung, die die biologische Aktivität des Polypeptids im Verhältnis zu der biologischen Aktivität, die in Abwesenheit der Testverbindung nachgewiesen wird, hemmt.

5. Verfahren zum Screenen auf eine Verbindung, die in der Behandlung von Brustkrebs nützlich ist, wobei das Verfahren die folgenden Schritte umfasst:
(a) Inkontaktbringen einer Testverbindung mit einer Zelle, in die ein Vektor eingeführt wurde, der die transkriptionelle regulatorische Region eines Markergens und ein Reportergen umfasst, das unter der Kontrolle der transkriptionellen regulatorischen Region exprimiert wird, wobei das Markergen eine Nucleotidsequenz ausgewählt aus der Gruppe bestehend aus SEQ ID NO:3, 5 und 7 umfasst,
(b) Messen der Expression oder Aktivität des Reportergens; und
(c) Auswählen der Verbindung, die den Expressions- oder Aktivitätsspiegel des Reportergens im Verhältnis zu dem in Abwesenheit der Testverbindung nachgewiesenen Expressions- oder Aktivitätsspiegel des Reportergens herabsetzt.

6. Arzneimittel, das als Wirkstoff eine pharmazeutisch wirksame Menge eines Antisense-Polynucleotids oder einer kleinen interferierenden RNA, das/die gegen ein Polynucleotid bestehend aus der Nucleotidsequenz SEQ ID NO:3, 5 oder 7 gerichtet ist, und einen pharmazeutisch verträglichen Träger umfasst, zur Verwendung bei der Behandlung von Brustkrebs.

7. Verwendung einer pharmazeutisch wirksamen Menge eines Antisense-Polynucleotids oder einer kleinen interferierenden RNA, das/die gegen ein Polynucleotid bestehend aus der Nucleotidsequenz von SEQ ID NO:3, 5 oder 7 gerichtet ist, zur Herstellung eines Arzneimittels zur Behandlung von Brustkrebs.

## Revendications

1. Petit ARN interférant, dans lequel le brin sens est choisi dans le groupe constitué par les séquences de nucléotides SEQ ID NO: 40 et 41 en tant que séquence cible.

2. Procédé de criblage d'un composé utile dans le traitement du cancer du sein, ledit procédé comprenant les étapes consistant à :
(a) mettre en contact un composé de test avec un polypeptide choisi dans le groupe constitué par :
(1) un polypeptide comprenant la séquence d'acides aminés SEQ ID NO: 4, 6 ou 8 ;
(2) un polypeptide qui comprend la séquence d'acides aminés SEQ ID NO: 4, 6 ou 8 dans lequel un ou plusieurs acides aminés sont substitués, délétés, insérés et/ou ajoutés et qui possède une identité à 95 % par rapport à la SEQ ID NO: 4, 6 ou 8, et une activité biologique d'augmentation de la prolifération cellulaire et de l'activité kinase ; et
(3) un polypeptide codé par un polynucléotide qui s'hybride dans des conditions stringentes à un polynucléotide constitué de la séquence de nucléotides SEQ ID NO: 3, 5 ou 7, le polypeptide ayant une activité biologique d'augmentation de la prolifération cellulaire et de l'activité kinase ;
(b) détecter l'activité de liaison entre le polypeptide et le composé de test ; et
(c) sélectionner le composé de test qui se lie au polypeptide et qui inhibe son activité biologique d'augmentation de la prolifération cellulaire et de l'activité kinase.

3. Procédé de criblage d'un composé utile dans le traitement du cancer du sein, ledit procédé comprenant les étapes consistant à :
(a) mettre en contact un composé candidat avec une cellule exprimant un ou plusieurs polypeptides comprenant la séquence de nucléotides SEQ ID NO: 3, 5 ou 7 ; et
(b) sélectionner un composé qui réduit le niveau d'expression d'un ou plusieurs polynucléotides comprenant la séquence de nucléotides SEQ ID NO 3, 5 ou 7 comparativement au taux d'expression détecté en l'absence du composé de test.

4. Procédé de criblage d'un composé utile dans le traitement du cancer du sein, ledit procédé comprenant les étapes consistant à :
(a) mettre en contact un composé de test avec un polypeptide choisi dans le groupe constitué par :
(1) un polypeptide comprenant la séquence d'acides aminés SEQ ID NO: 4, 6 ou 8 ;
(2) un polypeptide qui comprend la séquence d'acides aminés SEQ ID NO: 4, 6 ou 8 dans lequel un ou plusieurs acides aminés sont substitués, délétés, insérés et/ou ajoutés et qui possède une identité à 95 % par rapport à la SEQ ID NO: 4, 6 ou 8, et une activité biologique d'augmentation de la prolifération cellulaire et de l'activité kinase ; et
(3) un polypeptide codé par un polynucléotide qui s'hybride dans des conditions stringentes à un polynucléotide constitué de la séquence de nucléotides SEQ ID NO: 3, 5 ou 7, le polypeptide ayant une activité biologique d'augmentation de la prolifération cellulaire et une activité kinase ;
(b) détecter l'activité biologique d'augmentation de la prolifération cellulaire et de l'activité kinase du polypeptide de l'étape (a) ; et
(c) sélectionner un composé qui supprime l'activité biologique du polypeptide comparativement à l'activité biologique détectée en l'absence du composé de test.

5. Procédé de criblage d'un composé utile dans le traitement du cancer du sein, ledit procédé comprenant les étapes consistant à :
(a) mettre en contact un composé candidat avec une cellule dans laquelle un vecteur comprenant la région de régulation transcriptionnelle d'un gène marqueur et un gène rapporteur qui est exprimé sous le contrôle de la région de régulation transcriptionnelle a été introduit, le gène marqueur comprenant n'importe quelle séquence de nucléotides choisie dans le groupe constitué par les SEQ ID NO: 3, 5 et 7 ;
(b) mesurer l'expression ou l'activité dudit gène rapporteur ; et
(c) sélectionner le composé qui réduit le taux d'expression ou l'activité dudit gène rapporteur comparativement au taux d'expression ou à l'activité dudit gène rapporteur détecté en l'absence du composé de test.

6. Composition pharmaceutique comprenant une quantité pharmaceutiquement efficace d'un polynucléotide antisens ou d'un petit ARN interférant dirigé contre un polynucléotide constitué de la séquence de nucléotides SEQ ID NO: 3, 5 ou 7 en tant qu'ingrédient actif, et un support pharmaceutiquement acceptable pour une utilisation dans le traitement du cancer du sein.

7. Utilisation d'une quantité pharmaceutiquement efficace d'un polynucléotide antisens ou d'un petit ARN interférant dirigé contre un polynucléotide constitué de la séquence de nucléotides SEQ ID NO: 3, 5 ou 7 pour la préparation d'une composition pharmaceutique destinée à traiter le cancer du sein.


### SEQUENCE LISTING

<110> ONCOTHERAPY SCIENCE, INC.
   THE UNIVERSITY OF TOKYO
<120> GENES AND POLYPEPTIDES RELATING TO BREAST CANCERS
<130> ONC-A0409P
<150> US 60/600, 146
   <151> 2004-08-10
<160> 44
<170> PatentIn version 3.3
<210> 1
   <211> 2338
   <212> DNA
   <213> Homo sapiens
<220>
   <221> CDS
   <222> (99) .. (1685)
<400> 1
   aaaaaatgct gaactgctct ttggaagtcg ccggtgctgt tgtagttgga gtctgttcac 60
   gggcctgagc ttcgaggcca ggctcccggg tgtcgtta atg ttc ggg gcc gcc ggg 116
   Met Phe Gly Ala Ala Gly
   1 5
   cgc caa ccg atc gga gct cca gca gcc ggg aac agc tgg cat ttc agt 164
   Arg Gln Pro Ile Gly Ala Pro Ala Ala Gly Asn Ser Trp His Phe Ser
   10 15 20
   aga acc atg gag gag ctg gtt cat gac ctt gtc tca gca ttg gaa gag 212
   Arg Thr Met Glu Glu Leu Val His Asp Leu Val Ser Ala Leu Glu Glu
   25 30 35
   agc tca gag caa gct cga ggt gga ttt gct gaa aca gga gac cat tct 260
   Ser Ser Glu Gln Ala Arg Gly Gly Phe Ala Glu Thr Gly Asp His Ser
   40 45 50
   cga agt ata tct tgc cct ctg aaa cgc cag gca agg aaa agg aga ggg 308
   Arg Ser Ile Ser Cys Pro Leu Lys Arg Gln Ala Arg Lys Arg Arg Gly
   55 60 65 70
   aga aaa cgg agg tcg tat aat gtg cat cac ccg tgg gag act ggt cac 356
   Arg Lys Arg Arg Ser Tyr Asn Val His His Pro Trp Glu Thr Gly His
   75 80 85
   tgc tta agt gaa ggc tct gat tct agt tta gaa gaa cca agc aag gac 404
   Cys Leu Ser Glu Gly Ser Asp Ser Ser Leu Glu Glu Pro Ser Lys Asp
   90 95 100
   tat aga gag aat cac aat aat aat aaa aaa gat cac agt gac tct gat 452
   Tyr Arg Glu Asn His Asn Asn Asn Lys Lys Asp His Ser Asp Ser Asp
   105 110 115
   gac caa atg tta gta gca aag cgc agg ccg tca tca aac tta aat aat 500
   Asp Gln Met Leu Val Ala Lys Arg Arg Pro Ser Ser Asn Leu Asn Asn
   120 125 130
   aat gtt cga ggg aaa aga cct cta tgg cat gag tct gat ttt gct gtg 548
   Asn Val Arg Gly Lys Arg Pro Leu Trp His Glu Ser Asp Phe Ala Val
   135 140 145 150
   gac aat gtt ggg aat aga act ctg cgc agg agg aga aag gta aaa cgc 596
   Asp Asn Val Gly Asn Arg Thr Leu Arg Arg Arg Arg Lys Val Lys Arg
   155 160 165
   atg gca gta gat ctc cca cag gac atc tct aac aaa cgg aca atg acc 644
   Met Ala Val Asp Leu Pro Gln Asp Ile Ser Asn Lys Arg Thr Met Thr
   170 175 180
   cag cca cct gag ggt tgt aga gat cag gac atg gac agt gat aga gcc 692
   Gln Pro Pro Glu Gly Cys Arg Asp Gln Asp Met Asp Ser Asp Arg Ala
   185 190 195
   tac cag tat caa gaa ttt acc aag aac aaa gtc aaa aaa aga aag ttg 740
   Tyr Gln Tyr Gln Glu Phe Thr Lys Asn Lys Val Lys Lys Arg Lys Leu
   200 205 210
   aaa ata atc aga caa gga cca aaa atc caa gat gaa gga gta gtt tta 788
   Lys Ile Ile Arg Gln Gly Pro Lys Ile Gln Asp Glu Gly Val Val Leu
   215 220 225 230
   gaa agt gag gaa acg aac cag acc aat aag gac aaa atg gaa tgt gaa 836
   Glu Ser Glu Glu Thr Asn Gln Thr Asn Lys Asp Lys Met Glu Cys Glu
   235 240 245
   gag caa aaa gtc tca gat gag ctc atg agt gaa agt gat tcc agc agt 884
   Glu Gln Lys Val Ser Asp Glu Leu Met Ser Glu Ser Asp Ser Ser Ser
   250 255 260
   ctc agc agc act gat gct gga ttg ttt acc aat gat gag gga aga caa 932
   Leu Ser Ser Thr Asp Ala Gly Leu Phe Thr Asn Asp Glu Gly Arg Gln
   265 270 275
   ggt gat gat gaa cag agt gac tgg ttc tac gaa aag gaa tca ggt gga 980
   Gly Asp Asp Glu Gln Ser Asp Trp Phe Tyr Glu Lys Glu Ser Gly Gly
   280 285 290
   gca tgt ggt atc act gga gtt gtg ccc tgg tgg gaa aag gaa gat cct 1028
   Ala Cys Gly Ile Thr Gly Val Val Pro Trp Trp Glu Lys Glu Asp Pro
   295 300 305 310
   act gag cta gac aaa aat gta cca gat cct gtc ttt gaa agt atc tta 1076
   Thr Glu Leu Asp Lys Asn Val Pro Asp Pro Val Phe Glu Ser Ile Leu
   315 320 325
   act ggt tct ttt ccc ctt atg tca cac cca agc aga aga ggt ttc caa 1124
   Thr Gly Ser Phe Pro Leu Met Ser His Pro Ser Arg Arg Gly Phe Gln
   330 335 340
   gct aga ctc agt cgc ctt cat gga atg tct tca aag aat att aaa aaa 1172
   Ala Arg Leu Ser Arg Leu His Gly Met Ser Ser Lys Asn Ile Lys Lys
   345 350 355
   tct gga ggg act cca act tca atg gta ccc att cct ggc cca gtg ggt 1220
   Ser Gly Gly Thr Pro Thr Ser Met Val Pro Ile Pro Gly Pro Val Gly
   360 365 370
   aac aag aga atg gtt cat ttt tcc ccg gat tct cat cac cat gac cat 1268
   Asn Lys Arg Met Val His Phe Ser Pro Asp Ser His His His Asp His
   375 380 385 390
   tgg ttt agc cct ggg gct agg aca gag cat gac cag cat cag ctt ctg 1316
   Trp Phe Ser Pro Gly Ala Arg Thr Glu His Asp Gln His Gln Leu Leu
   395 400 405
   aga gat aat cga gct gaa aga gga cac aag aaa aat tgt tct gtg aga 1364
   Arg Asp Asn Arg Ala Glu Arg Gly His Lys Lys Asn Cys Ser Val Arg
   410 415 420
   aca gcc agc agg caa aca agc atg cat tta gga tcc tta tgc acg gga 1412
   Thr Ala Ser Arg Gln Thr Ser Met His Leu Gly Ser Leu Cys Thr Gly
   425 430 435
   gat atc aaa cgg aga aga aaa gct gca cct ttg cct gga cct act act 1460
   Asp Ile Lys Arg Arg Arg Lys Ala Ala Pro Leu Pro Gly Pro Thr Thr
   440 445 450
   gca gga ttt gta ggt gaa aat gcc cag cca atc cta gaa aat aat att 1508
   Ala Gly Phe Val Gly Glu Asn Ala Gln Pro Ile Leu Glu Asn Asn Ile
   455 460 465 470
   gga aac cga atg ctt cag aat atg ggc tgg acg cct ggg tca ggc ctt 1556
   Gly Asn Arg Met Leu Gln Asn Met Gly Trp Thr Pro Gly Ser Gly Leu
   475 480 485
   gga cga gat ggc aag ggg atc tct gag cca att caa gcc atg cag agg 1604
   Gly Arg Asp Gly Lys Gly Ile Ser Glu Pro Ile Gln Ala Met Gln Arg
   490 495 500
   cca aag gga tta gga ctt gga ttt cct cta cca aaa agt act tcc gca 1652
   Pro Lys Gly Leu Gly Leu Gly Phe Pro Leu Pro Lys Ser Thr Ser Ala
   505 510 515
   act act acc ccc aat gca gga aaa tcc gcc taa gaaaagcaaa gaagaaatgt 1705
   Thr Thr Thr Pro Asn Ala Gly Lys Ser Ala
   520 525
   tttacagact ttattcacta tgtcccattg ttctaaaatg ataacatgac ttctgttttt 1765
   gaagcaaaaa tctacattgc ctcaaacaca tcactctagc ttccttactg catacagtcc 1825
   tgccatagtg agagaaatgg gatttcatca caattcatgg tgctaaaatg aaaacctctg 1885
   cactttaatt tttttcagta atttccagct atttctaggt ataaagagca gctcgtttct 1945
   cttatttatt ttagtctcat gtgtcaatac tttccgatgc tttgcttaat tcatgtatgt 2005
   gtgcagtgct gcaatgccca gacaaacgtg agcacaccca ccagtttcta aaatggaata 2065
   gacaggaaaa gattgtgttt tatatcatcc ctatctattg taacccaaaa gacctaccat 2125
   cgcatcagtg aagtccgaac acatctttgt ttgaaaggct tgtcaatttc atattccttg 2185
   aattggcttc ttggtgagga ttttctgaca gagtgatacc catcaatttt ctatccttag 2245
   acaatgtagt gtgaagttca cagttgacaa acaacaatta atgtttccct tggatgtttt 2305
   gacaaaaata aacctcatcg ttgttatcac cag 2338
<210> 2
   <211> 528
   <212> PRT
   <213> Homo sapiens
<400> 2
   Met Phe Gly Ala Ala Gly Arg Gln Pro Ile Gly Ala Pro Ala Ala Gly
   1 5 10 15
   Asn Ser Trp His Phe Ser Arg Thr Met Glu Glu Leu Val His Asp Leu
   20 25 30
   Val Ser Ala Leu Glu Glu Ser Ser Glu Gln Ala Arg Gly Gly Phe Ala
   35 40 45
   Glu Thr Gly Asp His Ser Arg Ser Ile Ser Cys Pro Leu Lys Arg Gln
   50 55 60
   Ala Arg Lys Arg Arg Gly Arg Lys Arg Arg Ser Tyr Asn Val His His
   65 70 75 80
   Pro Trp Glu Thr Gly His Cys Leu Ser Glu Gly Ser Asp Ser Ser Leu
   85 90 95
   Glu Glu Pro Ser Lys Asp Tyr Arg Glu Asn His Asn Asn Asn Lys Lys
   100 105 110
   Asp His Ser Asp Ser Asp Asp Gln Met Leu Val Ala Lys Arg Arg Pro
   115 120 125
   Ser Ser Asn Leu Asn Asn Asn Val Arg Gly Lys Arg Pro Leu Trp His
   130 135 140
   Glu Ser Asp Phe Ala Val Asp Asn Val Gly Asn Arg Thr Leu Arg Arg
   145 150 155 160
   Arg Arg Lys Val Lys Arg Met Ala Val Asp Leu Pro Gln Asp Ile Ser
   165 170 175
   Asn Lys Arg Thr Met Thr Gln Pro Pro Glu Gly Cys Arg Asp Gln Asp
   180 185 190
   Met Asp Ser Asp Arg Ala Tyr Gln Tyr Gln Glu Phe Thr Lys Asn Lys
   195 200 205
   Val Lys Lys Arg Lys Leu Lys Ile Ile Arg Gln Gly Pro Lys Ile Gln
   210 215 220
   Asp Glu Gly Val Val Leu Glu Ser Glu Glu Thr Asn Gln Thr Asn Lys
   225 230 235 240
   Asp Lys Met Glu Cys Glu Glu Gln Lys Val Ser Asp Glu Leu Met Ser
   245 250 255
   Glu Ser Asp Ser Ser Ser Leu Ser Ser Thr Asp Ala Gly Leu Phe Thr
   260 265 270
   Asn Asp Glu Gly Arg Gln Gly Asp Asp Glu Gln Ser Asp Trp Phe Tyr
   275 280 285
   Glu Lys Glu Ser Gly Gly Ala Cys Gly Ile Thr Gly Val Val Pro Trp
   290 295 300
   Trp Glu Lys Glu Asp Pro Thr Glu Leu Asp Lys Asn Val Pro Asp Pro
   305 310 315 320
   Val Phe Glu Ser Ile Leu Thr Gly Ser Phe Pro Leu Met Ser His Pro
   325 330 335
   Ser Arg Arg Gly Phe Gln Ala Arg Leu Ser Arg Leu His Gly Met Ser
   340 345 350
   Ser Lys Asn Ile Lys Lys Ser Gly Gly Thr Pro Thr Ser Met Val Pro
   355 360 365
   Ile Pro Gly Pro Val Gly Asn Lys Arg Met Val His Phe Ser Pro Asp
   370 375 380
   Ser His His His Asp His Trp Phe Ser Pro Gly Ala Arg Thr Glu His
   385 390 395 400
   Asp Gln His Gln Leu Leu Arg Asp Asn Arg Ala Glu Arg Gly His Lys
   405 410 415
   Lys Asn Cys Ser Val Arg Thr Ala Ser Arg Gln Thr Ser Met His Leu
   420 425 430
   Gly Ser Leu Cys Thr Gly Asp Ile Lys Arg Arg Arg Lys Ala Ala Pro
   435 440 445
   Leu Pro Gly Pro Thr Thr Ala Gly Phe Val Gly Glu Asn Ala Gln Pro
   450 455 460
   Ile Leu Glu Asn Asn Ile Gly Asn Arg Met Leu Gln Asn Met Gly Trp
   465 470 475 480
   Thr Pro Gly Ser Gly Leu Gly Arg Asp Gly Lys Gly Ile Ser Glu Pro
   485 490 495
   Ile Gln Ala Met Gln Arg Pro Lys Gly Leu Gly Leu Gly Phe Pro Leu
   500 505 510
   Pro Lys Ser Thr Ser Ala Thr Thr Thr Pro Asn Ala Gly Lys Ser Ala
   515 520 525
<210> 3
   <211> 2501
   <212> DNA
   <213> Homo sapiens
<220>
   <221> CDS
   <222> (139) .. (2094)
<400> 3
   cgaaaagatt cttaggaacg ccgtaccagc cgcgtctctc aggacagcag gcccctgtcc      60
   ttctgtcggg cgccgctcag ccgtgccctc cgcccctcag gttctttttc taattccaaa      120
   taaacttgca agaggact atg aaa gat tat gat gaa ctt ctc aaa tat tat       171
                       Met Lys Asp Tyr Asp Glu Leu Leu Lys Tyr Tyr
                       1               5                   10
   gaa tta cat gaa act att ggg aca ggt ggc ttt gca aag gtc aaa ctt       219
   Glu Leu His Glu Thr Ile Gly Thr Gly Gly Phe Ala Lys Val Lys Leu
           15                  20                  25
   gcc tgc cat atc ctt act gga gag atg gta gct ata aaa atc atg gat       267
   Ala Cys His Ile Leu Thr Gly Glu Met Val Ala Ile Lys Ile Met Asp
       30                  35                  40
   aaa aac aca cta ggg agt gat ttg ccc cgg atc aaa acg gag att gag       315
   Lys Asn Thr Leu Gly Ser Asp Leu Pro Arg Ile Lys Thr Glu Ile Glu
   45                  50                  55
   gcc ttg aag aac ctg aga cat cag cat ata tgt caa ctc tac cat gtg       363
   Ala Leu Lys Asn Leu Arg His Gln His Ile Cys Gln Leu Tyr His Val
   60                  65                  70                  75
   cta gag aca gcc aac aaa ata ttc atg gtt ctt gag tac tgc cct gga       411
   Leu Glu Thr Ala Asn Lys Ile Phe Met Val Leu Glu Tyr Cys Pro Gly
   80 85 90
   gga gag ctg ttt gac tat ata att tcc cag gat cgc ctg tca gaa gag 459
   Gly Glu Leu Phe Asp Tyr Ile Ile Ser Gln Asp Arg Leu Ser Glu Glu
   95 100 105
   gag acc cgg gtt gtc ttc cgt cag ata gta tct gct gtt gct tat gtg 507
   Glu Thr Arg Val Val Phe Arg Gln Ile Val Ser Ala Val Ala Tyr Val
   110 115 120
   cac agc cag ggc tat gct cac agg gac ctc aag cca gaa aat ttg ctg 555
   His Ser Gln Gly Tyr Ala His Arg Asp Leu Lys Pro Glu Asn Leu Leu
   125 130 135
   ttt gat gaa tat cat aaa tta aag ctg att gac ttt ggt ctc tgt gca 603
   Phe Asp Glu Tyr His Lys Leu Lys Leu Ile Asp Phe Gly Leu Cys Ala
   140 145 150 155
   aaa ccc aag ggt aac aag gat tac cat cta cag aca tgc tgt ggg agt 651
   Lys Pro Lys Gly Asn Lys Asp Tyr His Leu Gln Thr Cys Cys Gly Ser
   160 165 170
   ctg gct tat gca gca cct gag tta ata caa ggc aaa tca tat ctt gga 699
   Leu Ala Tyr Ala Ala Pro Glu Leu Ile Gln Gly Lys Ser Tyr Leu Gly
   175 180 185
   tca gag gca gat gtt tgg agc atg ggc ata ctg tta tat gtt ctt atg 747
   Ser Glu Ala Asp Val Trp Ser Met Gly Ile Leu Leu Tyr Val Leu Met
   190 195 200
   tgt gga ttt cta cca ttt gat gat gat aat gta atg gct tta tac aag 795
   Cys Gly Phe Leu Pro Phe Asp Asp Asp Asn Val Met Ala Leu Tyr Lys
   205 210 215
   aag att atg aga gga aaa tat gat gtt ccc aag tgg ctc tct ccc agt 843
   Lys Ile Met Arg Gly Lys Tyr Asp Val Pro Lys Trp Leu Ser Pro Ser
   220 225 230 235
   agc att ctg ctt ctt caa caa atg ctg cag gtg gac cca aag aaa cgg 891
   Ser Ile Leu Leu Leu Gln Gln Met Leu Gln Val Asp Pro Lys Lys Arg
   240 245 250
   att tct atg aaa aat cta ttg aac cat ccc tgg atc atg caa gat tac 939
   Ile Ser Met Lys Asn Leu Leu Asn His Pro Trp Ile Met Gln Asp Tyr
   255 260 265
   aac tat cct gtt gag tgg caa agc aag aat cct ttt att cac ctc gat 987
   Asn Tyr Pro Val Glu Trp Gln Ser Lys Asn Pro Phe Ile His Leu Asp
   270 275 280
   gat gat tgc gta aca gaa ctt tct gta cat cac aga aac aac agg caa 1035
   Asp Asp Cys Val Thr Glu Leu Ser Val His His Arg Asn Asn Arg Gln
   285 290 295
   aca atg gag gat tta att tca ctg tgg cag tat gat cac ctc acg gct 1083
   Thr Met Glu Asp Leu Ile Ser Leu Trp Gln Tyr Asp His Leu Thr Ala
   300 305 310 315
   acc tat ctt ctg ctt cta gcc aag aag gct cgg gga aaa cca gtt cgt 1131
   Thr Tyr Leu Leu Leu Leu Ala Lys Lys Ala Arg Gly Lys Pro Val Arg
   320 325 330
   tta agg ctt tct tct ttc tcc tgt gga caa gcc agt gct acc cca ttc 1179
   Leu Arg Leu Ser Ser Phe Ser Cys Gly Gln Ala Ser Ala Thr Pro Phe
   335 340 345
   aca gac atc aag tca aat aat tgg agt ctg gaa gat gtg acc gca agt 1227
   Thr Asp Ile Lys Ser Asn Asn Trp Ser Leu Glu Asp Val Thr Ala Ser
   350 355 360
   gat aaa aat tat gtg gcg gga tta ata gac tat gat tgg tgt gaa gat 1275
   Asp Lys Asn Tyr Val Ala Gly Leu Ile Asp Tyr Asp Trp Cys Glu Asp
   365 370 375
   gat tta tca aca ggt gct gct act ccc cga aca tca cag ttt acc aag 1323
   Asp Leu Ser Thr Gly Ala Ala Thr Pro Arg Thr Ser Gln Phe Thr Lys
   380 385 390 395
   tac tgg aca gaa tca aat ggg gtg gaa tct aaa tca tta act cca gcc 1371
   Tyr Trp Thr Glu Ser Asn Gly Val Glu Ser Lys Ser Leu Thr Pro Ala
   400 405 410
   tta tgc aga aca cct gca aat aaa tta aag aac aaa gaa aat gta tat 1419
   Leu Cys Arg Thr Pro Ala Asn Lys Leu Lys Asn Lys Glu Asn Val Tyr
   415 420 425
   act cct aag tct gct gta aag aat gaa gag tac ttt atg ttt cct gag 1467
   Thr Pro Lys Ser Ala Val Lys Asn Glu Glu Tyr Phe Met Phe Pro Glu
   430 435 440
   cca aag act cca gtt aat aag aac cag cat aag aga gaa ata ctc act 1515
   Pro Lys Thr Pro Val Asn Lys Asn Gln His Lys Arg Glu Ile Leu Thr
   445 450 455
   acg cca aat cgt tac act aca ccc tca aaa gct aga aac cag tgc ctg 1563
   Thr Pro Asn Arg Tyr Thr Thr Pro Ser Lys Ala Arg Asn Gln Cys Leu
   460 465 470 475
   aaa gaa act cca att aaa ata cca gta aat tca aca gga aca gac aag 1611
   Lys Glu Thr Pro Ile Lys Ile Pro Val Asn Ser Thr Gly Thr Asp Lys
   480 485 490
   tta atg aca ggt gtc att agc cct gag agg cgg tgc cgc tca gtg gaa 1659
   Leu Met Thr Gly Val Ile Ser Pro Glu Arg Arg Cys Arg Ser Val Glu
   495 500 505
   ttg gat ctc aac caa gca cat atg gag gag act cca aaa aga aag gga 1707
   Leu Asp Leu Asn Gln Ala His Met Glu Glu Thr Pro Lys Arg Lys Gly
   510 515 520
   gcc aaa gtg ttt ggg agc ctt gaa agg ggg ttg gat aag gtt atc act 1755
   Ala Lys Val Phe Gly Ser Leu Glu Arg Gly Leu Asp Lys Val Ile Thr
   525 530 535
   gtg ctc acc agg agc aaa agg aag ggt tct gcc aga gac ggg ccc aga 1803
   Val Leu Thr Arg Ser Lys Arg Lys Gly Ser Ala Arg Asp Gly Pro Arg
   540 545 550 555
   aga cta aag ctt cac tat aat gtg act aca act aga tta gtg aat cca 1851
   Arg Leu Lys Leu His Tyr Asn Val Thr Thr Thr Arg Leu Val Asn Pro
   560 565 570
   gat caa ctg ttg aat gaa ata atg tct att ctt cca aag aag cat gtt 1899
   Asp Gln Leu Leu Asn Glu Ile Met Ser Ile Leu Pro Lys Lys His Val
   575 580 585
   gac ttt gta caa aag ggt tat aca ctg aag tgt caa aca cag tca gat 1947
   Asp Phe Val Gln Lys Gly Tyr Thr Leu Lys Cys Gln Thr Gln Ser Asp
   590 595 600
   ttt ggg aaa gtg aca atg caa ttt gaa tta gaa gtg tgc cag ctt caa 1995
   Phe Gly Lys Val Thr Met Gln Phe Glu Leu Glu Val Cys Gln Leu Gln
   605 610 615
   aaa ccc gat gtg gtg ggt atc agg agg cag cgg ctt aag ggc gat gcc 2043
   Lys Pro Asp Val Val Gly Ile Arg Arg Gln Arg Leu Lys Gly Asp Ala
   620 625 630 635
   tgg gtt tac aaa aga tta gtg gaa gac atc cta tct agc tgc aag gta 2091
   Trp Val Tyr Lys Arg Leu Val Glu Asp Ile Leu Ser Ser Cys Lys Val
   640 645 650
   taa ttgatggatt cttccatcct gccggatgag tgtgggtgtg atacagccta 2144
   cataaagact gttatgatcg ctttgatttt aaagttcatt ggaactacca acttgtttct 2204
   aaagagctat cttaagacca atatctcttt gtttttaaac aaaagatatt attttgtgta 2264
   tgaatctaaa tcaagcccat ctgtcattat gttactgtct tttttaatca tgtggttttg 2324
   tatattaata attgttgact ttcttagatt cacttccata tgtgaatgta agctcttaac 2384
   tatgtctctt tgtaatgtgt aatttctttc tgaaataaaa ccatttgtga atataaaaaa 2444
   aaaaaaaaaa aaaaaaaaaa aaaaaaaaaa aaaaaaaaaa aaaaaaaaaa aaaaaaa 2501
<210> 4
   <211> 651
   <212> PRT
   <213> Homo sapiens
<400> 4
   Met Lys Asp Tyr Asp Glu Leu Leu Lys Tyr Tyr Glu Leu His Glu Thr
   1 5 10 15
   Ile Gly Thr Gly Gly Phe Ala Lys Val Lys Leu Ala Cys His Ile Leu
   20 25 30
   Thr Gly Glu Met Val Ala Ile Lys Ile Met Asp Lys Asn Thr Leu Gly
   35 40 45
   Ser Asp Leu Pro Arg Ile Lys Thr Glu Ile Glu Ala Leu Lys Asn Leu
   50 55 60
   Arg His Gln His Ile Cys Gln Leu Tyr His Val Leu Glu Thr Ala Asn
   65 70 75 80
   Lys Ile Phe Met Val Leu Glu Tyr Cys Pro Gly Gly Glu Leu Phe Asp
   85 90 95
   Tyr Ile Ile Ser Gln Asp Arg Leu Ser Glu Glu Glu Thr Arg Val Val
   100 105 110
   Phe Arg Gln Ile Val Ser Ala Val Ala Tyr Val His Ser Gln Gly Tyr
   115 120 125
   Ala His Arg Asp Leu Lys Pro Glu Asn Leu Leu Phe Asp Glu Tyr His
   130 135 140
   Lys Leu Lys Leu Ile Asp Phe Gly Leu Cys Ala Lys Pro Lys Gly Asn
   145 150 155 160
   Lys Asp Tyr His Leu Gln Thr Cys Cys Gly Ser Leu Ala Tyr Ala Ala
   165 170 175
   Pro Glu Leu Ile Gln Gly Lys Ser Tyr Leu Gly Ser Glu Ala Asp Val
   180 185 190
   Trp Ser Met Gly Ile Leu Leu Tyr Val Leu Met Cys Gly Phe Leu Pro
   195 200 205
   Phe Asp Asp Asp Asn Val Met Ala Leu Tyr Lys Lys Ile Met Arg Gly
   210 215 220
   Lys Tyr Asp Val Pro Lys Trp Leu Ser Pro Ser Ser Ile Leu Leu Leu
   225 230 235 240
   Gln Gln Met Leu Gln Val Asp Pro Lys Lys Arg Ile Ser Met Lys Asn
   245 250 255
   Leu Leu Asn His Pro Trp Ile Met Gln Asp Tyr Asn Tyr Pro Val Glu
   260 265 270
   Trp Gln Ser Lys Asn Pro Phe Ile His Leu Asp Asp Asp Cys Val Thr
   275 280 285
   Glu Leu Ser Val His His Arg Asn Asn Arg Gln Thr Met Glu Asp Leu
   290 295 300
   Ile Ser Leu Trp Gln Tyr Asp His Leu Thr Ala Thr Tyr Leu Leu Leu
   305 310 315 320
   Leu Ala Lys Lys Ala Arg Gly Lys Pro Val Arg Leu Arg Leu Ser Ser
   325 330 335
   Phe Ser Cys Gly Gln Ala Ser Ala Thr Pro Phe Thr Asp Ile Lys Ser
   340 345 350
   Asn Asn Trp Ser Leu Glu Asp Val Thr Ala Ser Asp Lys Asn Tyr Val
   355 360 365
   Ala Gly Leu Ile Asp Tyr Asp Trp Cys Glu Asp Asp Leu Ser Thr Gly
   370 375 380
   Ala Ala Thr Pro Arg Thr Ser Gln Phe Thr Lys Tyr Trp Thr Glu Ser
   385 390 395 400
   Asn Gly Val Glu Ser Lys Ser Leu Thr Pro Ala Leu Cys Arg Thr Pro
   405 410 415
   Ala Asn Lys Leu Lys Asn Lys Glu Asn Val Tyr Thr Pro Lys Ser Ala
   420 425 430
   Val Lys Asn Glu Glu Tyr Phe Met Phe Pro Glu Pro Lys Thr Pro Val
   435 440 445
   Asn Lys Asn Gln His Lys Arg Glu Ile Leu Thr Thr Pro Asn Arg Tyr
   450 455 460
   Thr Thr Pro Ser Lys Ala Arg Asn Gln Cys Leu Lys Glu Thr Pro Ile
   465 470 475 480
   Lys Ile Pro Val Asn Ser Thr Gly Thr Asp Lys Leu Met Thr Gly Val
   485 490 495
   Ile Ser Pro Glu Arg Arg Cys Arg Ser Val Glu Leu Asp Leu Asn Gln
   500 505 510
   Ala His Met Glu Glu Thr Pro Lys Arg Lys Gly Ala Lys Val Phe Gly
   515 520 525
   Ser Leu Glu Arg Gly Leu Asp Lys Val Ile Thr Val Leu Thr Arg Ser
   530 535 540
   Lys Arg Lys Gly Ser Ala Arg Asp Gly Pro Arg Arg Leu Lys Leu His
   545 550 555 560
   Tyr Asn Val Thr Thr Thr Arg Leu Val Asn Pro Asp Gln Leu Leu Asn
   565 570 575
   Glu Ile Met Ser Ile Leu Pro Lys Lys His Val Asp Phe Val Gln Lys
   580 585 590
   Gly Tyr Thr Leu Lys Cys Gln Thr Gln Ser Asp Phe Gly Lys Val Thr
   595 600 605
   Met Gln Phe Glu Leu Glu Val Cys Gln Leu Gln Lys Pro Asp Val Val
   610 615 620
   Gly Ile Arg Arg Gln Arg Leu Lys Gly Asp Ala Trp Val Tyr Lys Arg
   625 630 635 640
   Leu Val Glu Asp Ile Leu Ser Ser Cys Lys Val
   645 650
<210> 5
   <211> 2368
   <212> DNA
   <213> Homo sapiens
<220>
   <221> CDS
   <222> (146).. (2002)
<400> 5
   aaagattctt aggaacgccg taccagccgc gtctctcagg acagcaggcc cctgtccttc 60
   tgtcgggcgc cgctcagccg tgccctccgc ccctcaggtt ctttttctaa ttccaaataa 120
   acttgcaaga ggactatgaa agatt atg atg aac ttc tca aat att atg aat 172
   Met Met Asn Phe Ser Asn Ile Met Asn
   1 5
   tac atg aaa cta ttg gga cag agt gat ttg ccc cgg atc aaa acg gag 220
   Tyr Met Lys Leu Leu Gly Gln Ser Asp Leu Pro Arg Ile Lys Thr Glu
   10 15 20 25
   att gag gcc ttg aag aac ctg aga cat cag cat ata tgt caa ctc tac 268
   Ile Glu Ala Leu Lys Asn Leu Arg His Gln His Ile Cys Gln Leu Tyr
   30 35 40
   cat gtg cta gag aca gcc aac aaa ata ttc atg gtt ctt gag tac tgc 316
   His Val Leu Glu Thr Ala Asn Lys Ile Phe Met Val Leu Glu Tyr Cys
   45 50 55
   cct gga gga gag ctg ttt gac tat ata att tcc cag gat cgc ctg tca 364
   Pro Gly Gly Glu Leu Phe Asp Tyr Ile Ile Ser Gln Asp Arg Leu Ser
   60 65 70
   gaa gag gag acc cgg gtt gtc ttc cgt cag ata gta tct gct gtt gct 412
   Glu Glu Glu Thr Arg Val Val Phe Arg Gln Ile Val Ser Ala Val Ala
   75 80 85
   tat gtg cac agc cag ggc tat gct cac agg gac ctc aag cca gaa aat 460
   Tyr Val His Ser Gln Gly Tyr Ala His Arg Asp Leu Lys Pro Glu Asn
   90 95 100 105
   ttg ctg ttt gat gaa tat cat aaa tta aag ctg att gac ttt ggt ctc 508
   Leu Leu Phe Asp Glu Tyr His Lys Leu Lys Leu Ile Asp Phe Gly Leu
   110 115 120
   tgt gca aaa ccc aag ggt aac aag gat tac cat cta cag aca tgc tgt 556
   Cys Ala Lys Pro Lys Gly Asn Lys Asp Tyr His Leu Gln Thr Cys Cys
   125 130 135
   ggg agt ctg gct tat gca gca cct gag tta ata caa ggc aaa tca tat 604
   Gly Ser Leu Ala Tyr Ala Ala Pro Glu Leu Ile Gln Gly Lys Ser Tyr
   140 145 150
   ctt gga tca gag gca gat gtt tgg agc atg ggc ata ctg tta tat gtt 652
   Leu Gly Ser Glu Ala Asp Val Trp Ser Met Gly Ile Leu Leu Tyr Val
   155 160 165
   ctt atg tgt gga ttt cta cca ttt gat gat gat aat gta atg gct tta 700
   Leu Met Cys Gly Phe Leu Pro Phe Asp Asp Asp Asn Val Met Ala Leu
   170 175 180 185
   tac aag aag att atg aga gga aaa tat gat gtt ccc aag tgg ctc tct 748
   Tyr Lys Lys Ile Met Arg Gly Lys Tyr Asp Val Pro Lys Trp Leu Ser
   190 195 200
   ccc agt agc att ctg ctt ctt caa caa atg ctg cag gtg gac cca aag 796
   Pro Ser Ser Ile Leu Leu Leu Gln Gln Met Leu Gln Val Asp Pro Lys
   205 210 215
   aaa cgg att tct atg aaa aat cta ttg aac cat ccc tgg atc atg caa 844
   Lys Arg Ile Ser Met Lys Asn Leu Leu Asn His Pro Trp Ile Met Gln
   220 225 230
   gat tac aac tat cct gtt gag tgg caa agc aag aat cct ttt att cac 892
   Asp Tyr Asn Tyr Pro Val Glu Trp Gln Ser Lys Asn Pro Phe Ile His
   235 240 245
   ctc gat gat gat tgc gta aca gaa ctt tct gta cat cac aga aac aac 940
   Leu Asp Asp Asp Cys Val Thr Glu Leu Ser Val His His Arg Asn Asn
   250 255 260 265
   agg caa aca atg gag gat tta att tca ctg tgg cag tat gat cac ctc 988
   Arg Gln Thr Met Glu Asp Leu Ile Ser Leu Trp Gln Tyr Asp His Leu
   270 275 280
   acg gct acc tat ctt ctg ctt cta gcc aag aag gct cgg gga aaa cca 1036
   Thr Ala Thr Tyr Leu Leu Leu Leu Ala Lys Lys Ala Arg Gly Lys Pro
   285 290 295
   gtt cgt tta agg ctt tct tct ttc tcc tgt gga caa gcc agt gct acc 1084
   Val Arg Leu Arg Leu Ser Ser Phe Ser Cys Gly Gln Ala Ser Ala Thr
   300 305 310
   cca ttc aca gac atc aag tca aat aat tgg agt ctg gaa gat gtg acc 1132
   Pro Phe Thr Asp Ile Lys Ser Asn Asn Trp Ser Leu Glu Asp Val Thr
   315 320 325
   gca agt gat aaa aat tat gtg gcg gga tta ata gac tat gat tgg tgt 1180
   Ala Ser Asp Lys Asn Tyr Val Ala Gly Leu Ile Asp Tyr Asp Trp Cys
   330 335 340 345
   gaa gat gat tta tca aca ggt gct gct act ccc cga aca tca cag ttt 1228
   Glu Asp Asp Leu Ser Thr Gly Ala Ala Thr Pro Arg Thr Ser Gln Phe
   350 355 360
   acc aag tac tgg aca gaa tca aat ggg gtg gaa tct aaa tca tta act 1276
   Thr Lys Tyr Trp Thr Glu Ser Asn Gly Val Glu Ser Lys Ser Leu Thr
   365 370 375
   cca gcc tta tgc aga aca cct gca aat aaa tta aag aac aaa gaa aat 1324
   Pro Ala Leu Cys Arg Thr Pro Ala Asn Lys Leu Lys Asn Lys Glu Asn
   380 385 390
   gta tat act cct aag tct gct gta aag aat gaa gag tac ttt atg ttt 1372
   Val Tyr Thr Pro Lys Ser Ala Val Lys Asn Glu Glu Tyr Phe Met Phe
   395 400 405
   cct gag cca aag act cca gtt aat aag aac cag cat aag aga gaa ata 1420
   Pro Glu Pro Lys Thr Pro Val Asn Lys Asn Gln His Lys Arg Glu Ile
   410 415 420 425
   ctc act acg cca aat cgt tac act aca ccc tca aaa gct aga aac cag 1468
   Leu Thr Thr Pro Asn Arg Tyr Thr Thr Pro Ser Lys Ala Arg Asn Gln
   430 435 440
   tgc ctg aaa gaa act cca att aaa ata cca gta aat tca aca gga aca 1516
   Cys Leu Lys Glu Thr Pro Ile Lys Ile Pro Val Asn Ser Thr Gly Thr
   445 450 455
   gac aag tta atg aca ggt gtc att agc cct gag agg cgg tgc cgc tca 1564
   Asp Lys Leu Met Thr Gly Val Ile Ser Pro Glu Arg Arg Cys Arg Ser
   460 465 470
   gtg gaa ttg gat ctc aac caa gca cat atg gag gag act cca aaa aga 1612
   Val Glu Leu Asp Leu Asn Gln Ala His Met Glu Glu Thr Pro Lys Arg
   475 480 485
   aag gga gcc aaa gtg ttt ggg agc ctt gaa agg ggg ttg gat aag gtt 1660
   Lys Gly Ala Lys Val Phe Gly Ser Leu Glu Arg Gly Leu Asp Lys Val
   490 495 500 505
   atc act gtg ctc acc agg agc aaa agg aag ggt tct gcc aga gac ggg 1708
   Ile Thr Val Leu Thr Arg Ser Lys Arg Lys Gly Ser Ala Arg Asp Gly
   510 515 520
   ccc aga aga cta aag ctt cac tat aat gtg act aca act aga tta gtg 1756
   Pro Arg Arg Leu Lys Leu His Tyr Asn Val Thr Thr Thr Arg Leu Val
   525 530 535
   aat cca gat caa ctg ttg aat gaa ata atg tct att ctt cca aag aag 1804
   Asn Pro Asp Gln Leu Leu Asn Glu Ile Met Ser Ile Leu Pro Lys Lys
   540 545 550
   cat gtt gac ttt gta caa aag ggt tat aca ctg aag tgt caa aca cag 1852
   His Val Asp Phe Val Gln Lys Gly Tyr Thr Leu Lys Cys Gln Thr Gln
   555 560 565
   tca gat ttt ggg aaa gtg aca atg caa ttt gaa tta gaa gtg tgc cag 1900
   Ser Asp Phe Gly Lys Val Thr Met Gln Phe Glu Leu Glu Val Cys Gln
   570 575 580 585
   ctt caa aaa ccc gat gtg gtg ggt atc agg agg cag cgg ctt aag ggc 1948
   Leu Gln Lys Pro Asp Val Val Gly Ile Arg Arg Gln Arg Leu Lys Gly
   590 595 600
   gat gcc tgg gtt tac aaa aga tta gtg gaa gac atc cta tct agc tgc 1996
   Asp Ala Trp Val Tyr Lys Arg Leu Val Glu Asp Ile Leu Ser Ser Cys
   605 610 615
   aag gta taattgatgg attcttccat cctgccggat gagtgtgggt gtgatacagc 2052
   Lys Val
   ctacataaag actgttatga tcgctttgat tttaaagttc attggaacta ccaacttgtt 2112
   tctaaagagc tatcttaaga ccaatatctc tttgttttta aacaaaagat attattttgt 2172
   gtatgaatct aaatcaagcc catctgtcat tatgttactg tcttttttaa tcatgtggtt 2232
   ttgtatatta ataattgttg actttcttag attcacttcc atatgtgaat gtaagctctt 2292
   aactatgtct ctttgtaatg tgtaatttct ttctgaaata aaaccatttg tgaatataaa 2352
   aaaaaaaaaa aaaaaa 2368
<210> 6
   <211> 619
   <212> PRT
   <213> Homo sapiens
<400> 6
   Met Met Asn Phe Ser Asn Ile Met Asn Tyr Met Lys Leu Leu Gly Gln
   1 5 10 15
   Ser Asp Leu Pro Arg Ile Lys Thr Glu Ile Glu Ala Leu Lys Asn Leu
   20 25 30
   Arg His Gln His Ile Cys Gln Leu Tyr His Val Leu Glu Thr Ala Asn
   35 40 45
   Lys Ile Phe Met Val Leu Glu Tyr Cys Pro Gly Gly Glu Leu Phe Asp
   50 55 60
   Tyr Ile Ile Ser Gln Asp Arg Leu Ser Glu Glu Glu Thr Arg Val Val
   65 70 75 80
   Phe Arg Gln Ile Val Ser Ala Val Ala Tyr Val His Ser Gln Gly Tyr
   85 90 95
   Ala His Arg Asp Leu Lys Pro Glu Asn Leu Leu Phe Asp Glu Tyr His
   100 105 110
   Lys Leu Lys Leu Ile Asp Phe Gly Leu Cys Ala Lys Pro Lys Gly Asn
   115 120 125
   Lys Asp Tyr His Leu Gln Thr Cys Cys Gly Ser Leu Ala Tyr Ala Ala
   130 135 140
   Pro Glu Leu Ile Gln Gly Lys Ser Tyr Leu Gly Ser Glu Ala Asp Val
   145 150 155 160
   Trp Ser Met Gly Ile Leu Leu Tyr Val Leu Met Cys Gly Phe Leu Pro
   165 170 175
   Phe Asp Asp Asp Asn Val Met Ala Leu Tyr Lys Lys Ile Met Arg Gly
   180 185 190
   Lys Tyr Asp Val Pro Lys Trp Leu Ser Pro Ser Ser Ile Leu Leu Leu
   195 200 205
   Gln Gln Met Leu Gln Val Asp Pro Lys Lys Arg Ile Ser Met Lys Asn
   210 215 220
   Leu Leu Asn His Pro Trp Ile Met Gln Asp Tyr Asn Tyr Pro Val Glu
   225 230 235 240
   Trp Gln Ser Lys Asn Pro Phe Ile His Leu Asp Asp Asp Cys Val Thr
   245 250 255
   Glu Leu Ser Val His His Arg Asn Asn Arg Gln Thr Met Glu Asp Leu
   260 265 270
   Ile Ser Leu Trp Gln Tyr Asp His Leu Thr Ala Thr Tyr Leu Leu Leu
   275 280 285
   Leu Ala Lys Lys Ala Arg Gly Lys Pro Val Arg Leu Arg Leu Ser Ser
   290 295 300
   Phe Ser Cys Gly Gln Ala Ser Ala Thr Pro Phe Thr Asp Ile Lys Ser
   305 310 315 320
   Asn Asn Trp Ser Leu Glu Asp Val Thr Ala Ser Asp Lys Asn Tyr Val
   325 330 335
   Ala Gly Leu Ile Asp Tyr Asp Trp Cys Glu Asp Asp Leu Ser Thr Gly
   340 345 350
   Ala Ala Thr Pro Arg Thr Ser Gln Phe Thr Lys Tyr Trp Thr Glu Ser
   355 360 365
   Asn Gly Val Glu Ser Lys Ser Leu Thr Pro Ala Leu Cys Arg Thr Pro
   370 375 380
   Ala Asn Lys Leu Lys Asn Lys Glu Asn Val Tyr Thr Pro Lys Ser Ala
   385 390 395 400
   Val Lys Asn Glu Glu Tyr Phe Met Phe Pro Glu Pro Lys Thr Pro Val
   405 410 415
   Asn Lys Asn Gln His Lys Arg Glu Ile Leu Thr Thr Pro Asn Arg Tyr
   420 425 430
   Thr Thr Pro Ser Lys Ala Arg Asn Gln Cys Leu Lys Glu Thr Pro Ile
   435 440 445
   Lys Ile Pro Val Asn Ser Thr Gly Thr Asp Lys Leu Met Thr Gly Val
   450 455 460
   Ile Ser Pro Glu Arg Arg Cys Arg Ser Val Glu Leu Asp Leu Asn Gln
   465 470 475 480
   Ala His Met Glu Glu Thr Pro Lys Arg Lys Gly Ala Lys Val Phe Gly
   485 490 495
   Ser Leu Glu Arg Gly Leu Asp Lys Val Ile Thr Val Leu Thr Arg Ser
   500 505 510
   Lys Arg Lys Gly Ser Ala Arg Asp Gly Pro Arg Arg Leu Lys Leu His
   515 520 525
   Tyr Asn Val Thr Thr Thr Arg Leu Val Asn Pro Asp Gln Leu Leu Asn
   530 535 540
   Glu Ile Met Ser Ile Leu Pro Lys Lys His Val Asp Phe Val Gln Lys
   545 550 555 560
   Gly Tyr Thr Leu Lys Cys Gln Thr Gln Ser Asp Phe Gly Lys Val Thr
   565 570 575
   Met Gln Phe Glu Leu Glu Val Cys Gln Leu Gln Lys Pro Asp Val Val
   580 585 590
   Gly Ile Arg Arg Gln Arg Leu Lys Gly Asp Ala Trp Val Tyr Lys Arg
   595 600 605
   Leu Val Glu Asp Ile Leu Ser Ser Cys Lys Val
   610 615
<210> 7
   <211> 2251
   <212> DNA
   <213> Homo sapiens
<220>
   <221> CDS
   <222> (148) .. (1887)
(400) 7
<210> 8
   <211> 580
   <212> PRT
   <213> Homo sapiens
<400> 8
   Met Met Asn Phe Ser Asn Ile Met Asn Tyr Met Lys Leu Leu Gly Gln
   1 5 10 15
   Tyr Cys Pro Gly Gly Glu Leu Phe Asp Tyr Ile Ile Ser Gln Asp Arg
   20 25 30
   Leu Ser Glu Glu Glu Thr Arg Val Val Phe Arg Gln Ile Val Ser Ala
   35 40 45
   Val Ala Tyr Val His Ser Gln Gly Tyr Ala His Arg Asp Leu Lys Pro
   50 55 60
   Glu Asn Leu Leu Phe Asp Glu Tyr His Lys Leu Lys Leu Ile Asp Phe
   65 70 75 80
   Gly Leu Cys Ala Lys Pro Lys Gly Asn Lys Asp Tyr His Leu Gln Thr
   85 90 95
   Cys Cys Gly Ser Leu Ala Tyr Ala Ala Pro Glu Leu Ile Gln Gly Lys
   100 105 110
   Ser Tyr Leu Gly Ser Glu Ala Asp Val Trp Ser Met Gly Ile Leu Leu
   115 120 125
   Tyr Val Leu Met Cys Gly Phe Leu Pro Phe Asp Asp Asp Asn Val Met
   130 135 140
   Ala Leu Tyr Lys Lys Ile Met Arg Gly Lys Tyr Asp Val Pro Lys Trp
   145 150 155 160
   Leu Ser Pro Ser Ser Ile Leu Leu Leu Gln Gln Met Leu Gln Val Asp
   165 170 175
   Pro Lys Lys Arg Ile Ser Met Lys Asn Leu Leu Asn His Pro Trp Ile
   180 185 190
   Met Gln Asp Tyr Asn Tyr Pro Val Glu Trp Gln Ser Lys Asn Pro Phe
   195 200 205
   Ile His Leu Asp Asp Asp Cys Val Thr Glu Leu Ser Val His His Arg
   210 215 220
   Asn Asn Arg Gln Thr Met Glu Asp Leu Ile Ser Leu Trp Gln Tyr Asp
   225 230 235 240
   His Leu Thr Ala Thr Tyr Leu Leu Leu Leu Ala Lys Lys Ala Arg Gly
   245 250 255
   Lys Pro Val Arg Leu Arg Leu Ser Ser Phe Ser Cys Gly Gln Ala Ser
   260 265 270
   Ala Thr Pro Phe Thr Asp Ile Lys Ser Asn Asn Trp Ser Leu Glu Asp
   275 280 285
   Val Thr Ala Ser Asp Lys Asn Tyr Val Ala Gly Leu Ile Asp Tyr Asp
   290 295 300
   Trp Cys Glu Asp Asp Leu Ser Thr Gly Ala Ala Thr Pro Arg Thr Ser
   305 310 315 320
   Gln Phe Thr Lys Tyr Trp Thr Glu Ser Asn Gly Val Glu Ser Lys Ser
   325 330 335
   Leu Thr Pro Ala Leu Cys Arg Thr Pro Ala Asn Lys Leu Lys Asn Lys
   340 345 350
   Glu Asn Val Tyr Thr Pro Lys Ser Ala Val Lys Asn Glu Glu Tyr Phe
   355 360 365
   Met Phe Pro Glu Pro Lys Thr Pro Val Asn Lys Asn Gln His Lys Arg
   370 375 380
   Glu Ile Leu Thr Thr Pro Asn Arg Tyr Thr Thr Pro Ser Lys Ala Arg
   385 390 395 400
   Asn Gln Cys Leu Lys Glu Thr Pro Ile Lys Ile Pro Val Asn Ser Thr
   405 410 415
   Gly Thr Asp Lys Leu Met Thr Gly Val Ile Ser Pro Glu Arg Arg Cys
   420 425 430
   Arg Ser Val Glu Leu Asp Leu Asn Gln Ala His Met Glu Glu Thr Pro
   435 440 445
   Lys Arg Lys Gly Ala Lys Val Phe Gly Ser Leu Glu Arg Gly Leu Asp
   450 455 460
   Lys Val Ile Thr Val Leu Thr Arg Ser Lys Arg Lys Gly Ser Ala Arg
   465 470 475 480
   Asp Gly Pro Arg Arg Leu Lys Leu His Tyr Asn Val Thr Thr Thr Arg
   485 490 495
   Leu Val Asn Pro Asp Gln Leu Leu Asn Glu Ile Met Ser Ile Leu Pro
   500 505 510
   Lys Lys His Val Asp Phe Val Gln Lys Gly Tyr Thr Leu Lys Cys Gln
   515 520 525
   Thr Gln Ser Asp Phe Gly Lys Val Thr Met Gln Phe Glu Leu Glu Val
   530 535 540
   Cys Gln Leu Gln Lys Pro Asp Val Val Gly Ile Arg Arg Gln Arg Leu
   545 550 555 560
   Lys Gly Asp Ala Trp Val Tyr Lys Arg Leu Val Glu Asp Ile Leu Ser
   565 570 575
   Ser Cys Lys Val
   580
<210> 9
   <211> 20
   <212> DNA
   <213> Artificial
<220>
   <223> Artifically synthesized primer for RT-PCR
<400> 9
   cgaccacttt gtcaagctca 20
<210> 10
   <211> 23
   <212> DNA
   <213> Artificial
<220>
   <223> Artificially synthesized primer for RT-PCR
<400> 10
   ggttgagcac agggtacttt att 23
<210> 11
   <211> 22
   <212> DNA
   <213> Artificial
<220>
   <223> Artificially synthesized primer for RT-PCR
<400> 11
   tgggtaacaa gagaatggtt ca 22
<210> 12
   <211> 22
   <212> DNA
   <213> Artificial
<220>
   <223> Airtificially synthesized primer for RT-PCR
<400> 12
   atccaagtcc taatcccttt gg 22
<210> 13
   <211> 22
   <212> DNA
   <213> Artificial
<220>
   <223> Artificially synthesized primer for RT-PCR
<400> 13
   gctgcaaggt ataattgatg ga 22
<210> 14
   <211> 23
   <212> DNA
   <213> Artificial
<220>
   <223> Airtificially synthesized primer for RT-PCR
<400> 14
   cagtaacata atgacagatg ggc 23
<210> 15
   <211> 22
   <212> DNA
   <213> Artificial
<220>
   <223> Artificially synthesized primer for PCR
<400> 15
   ttatcactgt gctcaccagg ag 22
<210> 16
   <211> 22
   <212> DNA
   <213> Artificial
<220>
   <223> Artificially synthesized primer for PCR
<400> 16
   aaacttgcct gccatatcct ta 22
<210> 17
   <211> 22
   <212> DNA
   <213> Artificial
<220>
   <223> Artificially synthesized primer for PCR
<400> 17
   attttgttgg ctgtctctag ca 22
<210> 18
   <211> 30
   <212> DNA
   <213> Artificial
<220>
   <223> Artificially synthesized primer for PCR
<400> 18
   aaagaattcg ggtgtcgtta atgttcgggg 30
<210> 19
   <211> 30
   <212> DNA
   <213> Artificial
<220>
   <223> Artificially synthesized primer for PCR
<400> 19
   aaagcggccg cttaggcgga ttttcctgca 30
<210> 20
   <211> 30
   <212> DNA
   <213> Artificial
<220>
   <223> Artificially synthesized primer for PCR
<400> 20
   cggaattcac tatgaaagat tatgatgaac 30
<210> 21
   <211> 30
   <212> DNA
   <213> Artificial
<220>
   <223> Artificially synthesized primer for PCR
<400> 21
   aaactcgagt accttgcagc tagataggat 30
<210> 22
   <211> 51
   <212> DNA
   <213> Artificial
<220>
   <223> Artificially synthesized oligonucleotide for siRNA
<400> 22
   tcccgcgcgc tttgtaggat tcgttcaaga gacgaatcct acaaagcgcg c 51
<210> 23
   <211> 51
   <212> DNA
   <213> Artificial
<220>
   <223> Artificially synthesized primer for siRNA
<400> 23
   aaaagcgcgc tttgtaggat tcgtctcttg aacgaatcct acaaagcgcg c 51
<210> 24
   <211> 51
   <212> DNA
   <213> Artificial
<220>
   <223> Artificially synthesized oligonucleotide for siRNA
<400> 24
   tccccgtacg cggaatactt cgattcaaga gatcgaagta ttccgcgtac g 51
<210> 25
   <211> 51
   <212> DNA
   <213> Artificial
<220>
   <223> Artificially synthesized oligonucleotide for siRNA
<400> 25
   aaaacgtacg cggaatactt cgatctcttg aatcgaagta ttccgcgtac g 51
<210> 26
   <211> 20
   <212> DNA
   <213> Artificial
<220>
   <223> Artificially synthesized primer for RT-PCR
<400> 26
   ttagctgtgc tcgcgctact 20
<210> 27
   <211> 20
   <212> DNA
   <213> Artificial
<220>
   <223> Artificially synthesized primer for RT-PCR
<400> 27
   tcacatggtt cacacggcag 20
<210> 28
   <211> 25
   <212> DNA
   <213> Artificial
<220>
   <223> Artificially synthesized primer for RT-PCR
<400> 28
   ttaagtgaag gctctgattc tagtt 25
<210> 29
   <211> 21
   <212> DNA
   <213> Artificial
<220>
   <223> Artificially synthesized primer for RT-PCR
<400> 29
   gtccttattg gctggttcgt t 21
<210> 30
   <211> 51
   <212> DNA
   <213> Artificial
<220>
   <223> Artificially synthesized oligonucleotide for siRNA
<400> 30
   tcccgtatat cttgccctct gaattcaaga gattcagagg gcaagatata c 51
<210> 31
   <211> 51
   <212> DNA
   <213> Artificial
<220>
   <223> Artificially synthesized oligonucleotide for siRNA
<400> 31
   aaaagtatat cttgccctct gaatctcttg aattcagagg gcaagatata c 51
<210> 32
   <211> 51
   <212> DNA
   <213> Artificial
<220>
   <223> Artificially synthesized oligonucleotide for siRNA
<400> 32
   tcccgtccga acacatcttt gttttcaaga gaaacaaaga tgtgttcgga c 51
<210> 33
   <211> 51
   <212> DNA
   <213> Artificial
<220>
   <223> Artificially synthesized oligonucleotide for siRNA
<400> 33
   aaaagtccga acacatcttt gtttctcttg aaaacaaaga tgtgttcgga c 51
<210> 34
   <211> 51
   <212> DNA
   <213> Artificial
<220>
   <223> Artificially synthesized oligonucleotides for siRNA
<400> 34
   tcccgacatc ctatctagct gcattcaaga gatgcagcta gataggatgt c 51
<210> 35
   <211> 51
   <212> DNA
   <213> Artificial
<220>
   <223> Artificially synthesized oligonucleotide for siRNA
<400> 35
   aaaagacatc ctatctagct gcatctcttg aatgcagcta gataggatgt c 51
<210> 36
   <211> 51
   <212> DNA
   <213> Artificial
<220>
   <223> Artificially synthesized oligonucleotide for siRNA
<400> 36
   tcccagttca ttggaactac caattcaaga gattggtagt tccaatgaac t 51
<210> 37
   <211> 51
   <212> DNA
   <213> Artificial
<220>
   <223> Artificially synthesized oligonucleotide for siRNA
<400> 37
   aaaaagttca ttggaactac caatctcttg aattggtagt tccaatgaac t 51
<210> 38
   <211> 19
   <212> DNA
   <213> Artificial
<220>
   <223> Target sequence for siRNA
<400> 38
   gtatatcttg ccctctgaa 19
<210> 39
   <211> 19
   <212> DNA
   <213> Artificial
<220>
   <223> Target sequence for siRNA
<400> 39
   gtccgaacac atctttgtt 19
<210> 40
   <211> 19
   <212> DNA
   <213> Artificial
<220>
   <223> Target sequence for siRNA
<400> 40
   gacatcctat ctagctgca 19
<210> 41
   <211> 19
   <212> DNA
   <213> Artificial
<220>
   <223> Target sequence for siRNA
<400> 41
   agttcattgg aactaccaa 19
<210> 42
   <211> 32
   <212> DNA
   <213> Artificial
<220>
   <223> Artificially synthesized primer for PCR
<400> 42
   acggaattca tcatgcaaga ttacaactat cc 32
<210> 43
   <211> 33
   <212> DNA
   <213> Artificial
<220>
   <223> Artificially synthesized primer for PCR
<400> 43
   gacggaattc aatatggagg agactccaaa aag 33
<210> 44
   <211> 30
   <212> DNA
   <213> Artificial
<220>
   <223> Artificially synthesized primer for PCR
<400> 44
   ccctcgagta ccttgcagct agataggatg 30